# EUROPÄISCHE PATENTSCHRIFT

(11) **EP 1 599 478 B1**
(45) Veröffentlichungstag und Bekanntmachung des Hinweises auf die Patenterteilung: **09.05.2007**
(21) Anmeldenummer: 03816028.9
(22) Anmeldetag: 26.02.2003
(51) Int. Cl.: C07D 475/04, A61K 31/4375, A61K 31/4985

(54) **DIHYDROPTERIDINONE, VERFAHREN ZU DEREN HERSTELLUNG UND DEREN VERWENDUNG ALS ARZNEIMITTEL**
DIHYDROPTERIDINONES, METHOD FOR THE PRODUCTION AND USE THEREOF IN THE FORM OF DRUGS
DIHYDROPTERIDINONES, PRODEDE DE PRODUCTION ET UTILISATION DE CES DERNIERES COMME MEDICAMENTS

(43) Veröffentlichungstag der Anmeldung: 30.11.2005
(73) Patentinhaber: Boehringer Ingelheim Pharma GmbH & Co.KG, 55216 Ingelheim am Rhein (DE)
(72) Erfinder: HOFFMANN, Matthias, 88441 MITTELBIBERACH (DE); GRAUERT, Matthias, 88400 BIBERACH (DE); BRANDL, Trixi, 4056 Basel (CH); BREITFELDER, Steffen, 88433 ASSMANNSHARDT (DE); EICKMEIER, Christian, 88441 MITTELBIBERACH (DE); STEEGMAIER, Martin, A-1120 WIEN (AT); SCHNAPP, Gisela, 88400 BIBERACH-RINDENMOOS (DE); BAUM, Anke, A-2534 ALLAND (AT); QUANT, Jens, Jürgen, A-2353 GUNTRAMSDORF (AT); SOLCA, Flavio, A-1230 WIEN (AT); COLBATZKY, Florian, 88441 STAFFLANGEN (DE)
(86) Internationale Anmeldenummer: PCT/EP2003/001935
(87) Internationale Veröffentlichungsnummer: WO 2004/076454

(56) Entgegenhaltungen:
- WO-A-01/19825
- WO-A-02/076985
- WO-A-03/020722
- FERRAND G ET AL: "Synthesis and potential antiallergic activity of new pteridinones and related compounds." EUROPEAN JOURNAL OF MEDICINAL CHEMISTRY, Bd. 31, Nr. 4, 1996, Seiten 273-280, XP002246920 ISSN: 0223-5234

## Beschreibung

Die vorliegende Erfindung betrifft neue Dihydropteridinone der allgemeinen Formel (1) 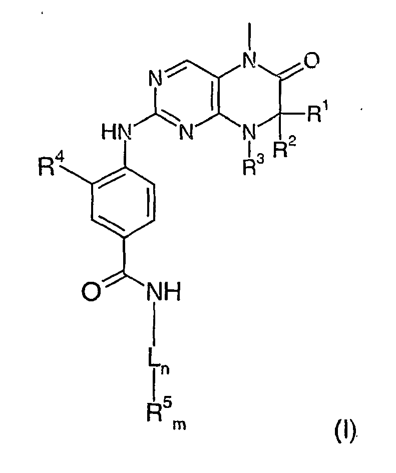 wobei die Reste L, R¹, R², R³, R⁴ und R⁵ die in den Ansprüchen und der Beschreibung genannten Bedeutungen haben, deren Isomere,
Verfahren zur Herstellung dieser Dihydropteridinone sowie deren Verwendung als Arzneimittel.

### Hintergrund der Erfindung

Pteridinon-Derivate sind als Wirkstoffe mit antiproliferativer Wirkung aus dem Stand der Technik bekannt. WO 01/019825 beschreibt die Verwendung von Pteridinonderivaten zur Behandlung von Tumor- und Viruserkrankungen.
Die Resistenz vieler Turnorarten erfordert die Entwicklung neuer Arzneimittel zur Tumorbekämpfung.
Es ist die Aufgabe der vorliegenden Erfindung neue Verbindungen mit antiinflammatorischer und antiprolifei-ativer Wirkung bereitzustellen.

### Detaillierte Beschreibung der Erfindung

Überraschenderweise wurde gefunden, daß Verbindungen der allgemeinen Formel (I), worin die Reste L und R¹ bis R⁵ die nachstehend genannten Bedeutungen haben, als Inhibitoren spezifischer Zellzyktuskinasen wirken. Somit können die erfindungsgemäßen Verbindungen beispielsweise zur Behandlung von Erkrankungen, die mit der Aktivität spezifischer Zellzykluskinasen in Zusammenhang stehen und durch exzessive oder anomale Zellproliferation charakterisiert sind, verwendet werden.

Die vorliegende Erfindung betrifft daher Verbindungen der allgemeinen Formel (I) 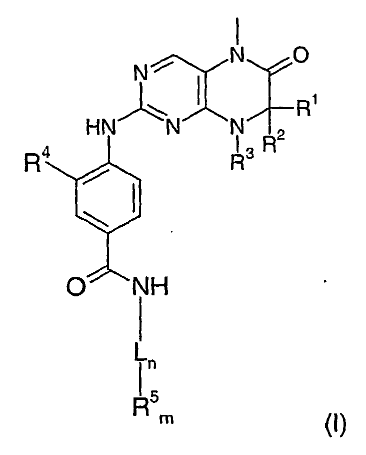 worin
R¹, R² gleich oder verschieden, Wasserstoff oder gegebenenfalls substituiertes C₁-C₆-Alkyl,
   oder
R¹ und R² gemeinsam eine 2- bis 5-gliedrige Alkylbrücke, die 1 bis 2 Heteroatome enthalten kann,
R³ Wasserstoff oder ein Rest ausgewählt aus der Gruppe bestehend aus gegebenenfalls substituiertem C₁-C₁₂-Alkyl, C₂-C₁₂-Alkenyl, C₂-C₁₂-Alkinyl und C₆-C₁₄-Aryl, oder
ein Rest ausgewählt aus der Gruppe bestehend aus gegebenenfalls substituiertem und/oder verbrücktern C₃-C₁₂-Cycloalkyl, C₃-C₁₂-Cycloalkenyl, C₇-C₁₂-Polycycloalkyl, C₇-C₁₂-Polycycloalkenyl, C₅-C₁₂-Spirocycloalkyl, C₃-C₁₂-Heterocycloalkyl, das 1 bis 2 Heteroatome enthält, und C₃-C₁₂-Heterocycloalkenyl, das 1 bis 2 Heteroatome enthält, oder
R¹ und R³ oder R² und R³ gemeinsam eine gesättigte oder ungesättigte C₃-C₄-Alkylbrücke, die 1 Heteroatom enthalten kann,
R⁴ ein Rest ausgewählt aus der Gruppe bestehend aus Wasserstoff, -CN, Hydroxy, -NR₆R₇ und Halogen, oder
   ein Rest ausgewählt aus der Gruppe bestehend aus gegebenenfalls substituiertem C₁-C₆-Alkyl, C₂-C₆-Alkenyl, C₂-C₆-Alkinyl, C₁-C₅-Alkyloxy, C₂-C₅-Alkenyloxy, C₂-C₅-Alkinyloxy, C₁-C₆-Alkylthio, C₁-C₆-Alkylsulfoxo und C₁-C₆-Alkylsulfonyl,
L ein Linker ausgewählt aus der Gruppe bestehend aus gegebenenfalls substituiertem C₂-C₁₀-Alkyl, C₂-C₁₀-Alkenyl, C₆-C₁₄-Aryl, -C₂-C₄-Alkyl-C₆-C₁₄-Aryl, - C₆-C₁₄-Aryl-C₁-C₄-Alkyl, gegebenenfalls verbrücktem C₃-C₁₂-Cycloalkyl und Heteroaryl, das 1 oder 2 Stickstoffatome enthält,
   - n: 0 oder 1
   - m: 1 oder 2
R⁵ ein Rest, ausgewählt aus der Gruppe bestehend aus gegebenenfalls substituiertem Morpholinyl, Piperidinyl, Piperazinyl, Piperazinylcarbonyl, Pyrrolidinyl, Tropenyl, R⁸-Diketomethylpiperazinyl, Sulfoxomorpholinyl, Sulfonylmorpholinyl, Thiornorpholinyl, -NR⁸R⁹ und Azacycloheptyl,
R⁶, R⁷ gleich oder verschieden, Wasserstoff oder C₁-C₄-Alkyl, und
R⁸, R⁹ unsubstituierte Stickstoffsubstituenten an R⁵, gleich oder verschieden, entweder Wasserstoff oder ein Rest ausgewählt aus der Gruppe bestehend aus C₁-C₆-Alkyl, -C₁-C₄-Alkyl-C₃-C₁₀-Cycloalkyl, C₃-C₁₀-Cycloalkyl, C₆-C₁₄-Aryl, -C₁-C₄-Alkyl-C₆-C₁₄-aryl, Pyranyl, Pyridinyl, Pyrimidinyl, C₁-C₄-Alkyloxycarbonyl, C₆-C₁₄-Arylcarbonyl-, C₁-C₄-Alkylcarbonyl-, C₆-C₁₄-Arylmethyloxycarbonyl-, C₆-C₁₄-Arylsulfonyl-, C₁-C₄-Alkylsulfonyl- und C₆-C₁₄-Aryl-C₁-C₄-Alkylsulfonyl-, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze, bedeuten.

Bevorzugt sind Verbindungen der Formel (1), worin
R¹ bis R⁴, R⁶ und R⁷ die angegebene Bedeutung aufweisen, und
L ein Linker ausgewählt aus der Gruppe bestehend aus gegebenenfalls substituiertem C₂-C₁₀-Alkyl, C₂-C₁₀-Alkenyl, C₆-C₁₄-Aryl, -C₂-C₄-Alkyl-C₆-C₁₄-Aryl, - C₆-C₁₄-Aryl-C₁-C₄-Alkyl, gegebenenfalls verbrücktem C₃-C₁₂-Cycloalkyl und Heteroaryl, das 1 oder 2 Stickstoffatome enthält
   - n: 1
   - m: 1 oder 2
R⁵ ein Rest, der über ein Stickstoffatom an L gebunden ist, ausgewählt aus der Gruppe bestehend aus gegebenenfalls substituiertem Morpholinyl, Piperidinyl, R⁸-Piperazinyl, Pyrrolidinyl, Tropenyl, R⁸-Diketomethylpiperazinyl, Sulfoxomorpholinyl, Sulfonylmorpholinyl, Thiomorpholinyl, -NR⁸R⁹ und Azacycloheptyl,
R⁸, R⁹ unsubstituierte Stickstoffsubstituenten an R⁵, gleich oder verschieden, die Wasserstoff oder ein Rest ausgewählt aus der Gruppe bestehend aus C₁-C₆-Alkyl, -C₁-C₄-Alkyl-C₃-C₁₀-Cycloalkyl, C₃-C₁₀-Cycloalkyl, C₆-C₁₄-Aryl, -C₁-C₄-Alkyl-C₆-C₁₄-aryl, Pyranyl, Pyridinyl, Pyrimidinyl, C₁-C₄-Alkyloxycarbonyl, C₆-C₁₄-Arylcarbonyl-, C₁-C₄-Alkylcarbonyl-, C₆-C₁₄-Arylmethyloxycarbonyl-, C₆-C₁₄-Arylsulfonyl-, C₁-C₄-Alkylsulfonyl- und C₆-C₁₄-Aryl-C₁-C₄-Alkylsulfonyl-,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.

Weiterhin bevorzugt sind Verbindungen der Formel (I), worin
R¹ bis R⁴, R⁶ und R⁷ die angegebene Bedeutung aufweisen,
L ein Linker ausgewählt aus der Gruppe bestehend aus gegebenenfalls substituiertem C₂-C₁₀-Alkyl, C₂-C₁₀-Alkenyl, C₆-C₁₄-Aryl, -C₂-C₄-Alkyl-C₆-C₁₄-Aryl, - C₆-C₁₄-Aryl-C₁-C₄-Alkyl, gegebenenfalls verbrücktem C₃-C₁₂-Cycloalkyl und
Heteroaryl, das 1 oder 2 Stickstoffatome enthält
   - n: 0 oder 1
   - m: 1 oder 2
R⁵ ein Rest, der über ein Kohlenstoffatom an L gebunden ist, ausgewählt aus der Gruppe bestehend aus R⁸ - Piperidinyl-, R⁸R⁹-Piperazinyl-, R⁸-Pyrrolidinyl, R⁸-Piperazinylcarbonyl-, R⁸-Tropenyl, R⁸-Morpholinyl und R⁸-Azacycloheptyl, und
R⁸, R⁹ unsubstituierte Stickstoffsubstituenten an R⁵, gleich oder verschieden, die Wasserstoff oder ein Rest ausgewählt aus der Gruppe bestehend aus C₁-C₆-Alkyl, -C₁-C₄-Alkyl-C₃-C₁₀-Cycloalkyl, C₃-C₁₀-Cycloalkyl, C₆-C₁₄-Aryl, -C₁-C₄-Alkyl-C₆-C₁₄-aryl, Pyranyl, Pyridinyl, Pyrimidinyl, C₁-C₄-Alkyloxycarbonyl, C₆-C₁₄-Arylcarbonyl-, C₁-C₄-Alkylcarbonyl-, C₆-C₁₄-Arylmethyloxycarbonyl-, C₆-C₁₄-Arylsulfonyl-, C₁-C₄-Alkylsulfonyl- und C₆-C₁₄-Aryl-C₁-C₄-Alkylsulfonyl-, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.

Besonders bevorzugt sind Verbindungen der Formel I, worin
L, m, n und R³ bis R⁹ die angegebene Bedeutung aufweisen, und
R¹, R² gleich oder verschieden, ein Rest ausgewählt aus der Gruppe bestehend aus Wasserstoff, Me, Et, Pr, oder
R¹ und R² gemeinsam eine C₂-C₄-Alkylbrücke bilden,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.

Insbesondere bevorzugt sind Verbindungen der Formel 1, worin
R¹, R², m, n und R⁵ bis R⁸ die angegebene Bedeutung aufweisen, und
R³ ein Rest ausgewählt aus der Gruppe bestehend aus gegebenenfalls substituiertem C₁-C₁₀-Alkyl, C₃-C₇-Cycloalkyl, C₃-C₆-Heterocycloalkyl und C₆-C₁₄-Aryl oder
R¹ und R³ oder R² und R³ gemeinsam eine gesättigte oder ungesättigte C₃-C₄-Alkylbrücke, die 1 bis 2 Heteroatome enthalten kann,
R⁴ ein Rest ausgewählt aus der Gruppe bestehend aus Wasserstoff, OMe, OH, Me, Et, Pr, OEt, NHMe, NH₂, F, CL, Br, O-Propargyl, O-Butinyl, CN, SMe, NMe₂, CONH₂, Ethinyl, Propinyl, Butinyl und Allyl, und
L ein Linker ausgewählt aus der Gruppe bestehend aus gegebenenfalls substituiertem Phenyl, Phenylmethyl, Cyclohexyl und verzweigtem C₁-C₆-Alkyl, gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.

Ein weiterer Gegenstand der Erfindung sind Verbindungen der Formel I zur Verwendung als Arzneimittel.

Erfindungsgemäß von besonderer Bedeutung sind Verbindungen der Formel I zur Verwendung als Arzneimittel mit antiproliferativer Wirkung.

Ein weiterer Gegenstand der Erfindung ist die Verwendung einer Verbindung der Formel 1 zur Herstellung eines Arzneimittels zur Behandlung und/oder Prävention von Krebs, Infektionen, Entzündungs- und Autoimmunerkrankungen.

Ein weiterer Gegenstand der Erfindung sind pharmazeutische Zubereitungen, enthaltend als Wirkstoff eine oder mehrere Verbindungen der allgemeinen Formel (I) oder deren physiologisch verträgliche Salze gegebenenfalls in Kombination mit üblichen Hilfs- und/oder Trägerstoffen.

Ein weiterer Gegenstand der Erfindung ist ein Verfahren zur Herstellung einer Verbindung der allgemeinen Formel (I), 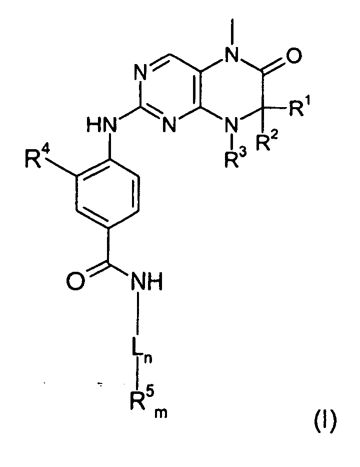 worin
R¹-R⁵,m ,n und L die angegebene Bedeutung aufweisen,
dadurch gekennzeichnet, daß eine Verbindung der allgemeinen Formel (II) 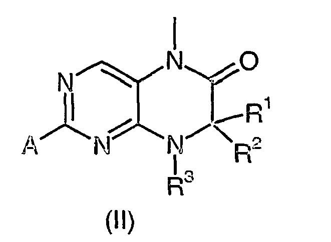 worin
R¹-R³ die angegebene Bedeutung aufweisen und A eine Abgangsgruppe ist, mit einer gegebenenfalls substituierten Verbindung der allgemeinen Formel (III), 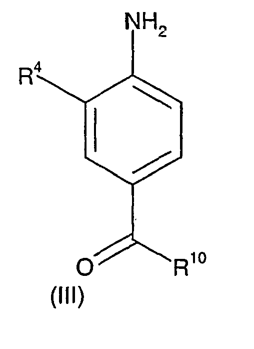 worin
R⁴ die angegebene Bedeutung aufweist und
R¹⁰ OH, NH-L-R⁵, -O-Methyl, -O-Ethyl bedeutet,
umgesetzt wird, und gegebenenfalls anschließend das Produkt der allgemeinen Formel (IV) 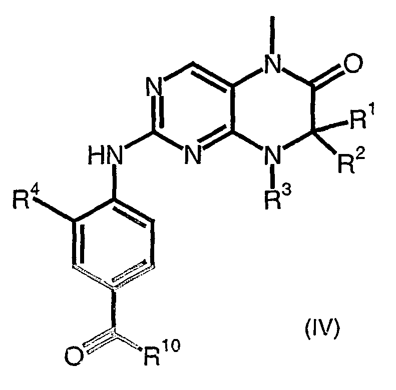 worin
R¹ bis R⁴ die angegebene Bedeutung aufweist und
R¹⁰ OH, -NH-L-R⁵, -O-Methyl oder -O-Ethyl bedeutet,
gegebenenfalls nach vorhergehender Hydrolyse der Estergruppe -COR¹⁰, mit einem Amin der allgemeinen Formel (V)

   NH₂-L-R⁵ₘ (V)

   worin
R⁵ die angegebene Bedeutung aufweist,
umgesetzt wird.

Ein weiterer Gegenstand der Erfindung ist eine Verbindung der Formel (II), 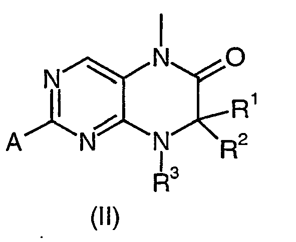 worin
R¹-R³ die angegebene Bedeutung aufweisen und A eine Abgangsgruppe ist.

Als Alkylgruppen sowie Alkylgruppen, welche Bestandteil anderer Reste sind, werden verzweigte und unverzweigte Alkylgruppen mit 1 bis 12 Kohlenstoffatomen bevorzugt 1 - 6, besonders bevorzugt 1-4 Kohlenstoffatomen bezeichnet, beispielsweise werden genannt: Methyl, Ethyl, Propyl, Butyl, Pentyl, Hexyl, Heptyl, Octyl, Nonyl, Decyl und Dodecyl. Sofern nicht anders genannt, sind von den vorstehend genannten Bezeichnungen Propyl, Butyl, Pentyl, Hexyl, Heptyl, Octyl, Nonyl, Decyl und Dodecyl sämtliche der möglichen isomeren Formen umfaßt. Beispielsweise umfaßt die Bezeichnung Propyl die beiden isomeren Reste n-Propyl und iso-Propyl, die Bezeichnung Butyl n-Butyl, iso-Butyl, sec. Butyl und tert.-Butyl, die Bezeichnung Pentyl, iso-Pentyl, Neopentyl etc.
In den vorstehend genannten Alkylgruppen können gegebenenfalls ein oder mehrere Wasserstoffatome durch andere Reste ersetzt sein. Beispielsweise können diese Alkylgruppen durch Fluor substituiert sein. Es können gegebenenfalls auch alle Wasserstoffatome der Alkylgruppe ersetzt sein.

Als Alkylbrücke werden, soweit nicht anders angegeben, verzweigte und unverzweigte Alkylgruppen mit 1 bis 5 Kohlenstoffatomen, beispielsweise Methylen, Etyhlen, Propylen-, Isopropylen-, n-Butylen, iso-Butyl, sec. Butyl und tert.-Butyl etc. Brücken bezeichnet. Besonders bevorzugt sind Methylen, Etyhlen, Propylen- und Butylen-Brückeh. In den genannten Alkylbrücken können gegebenenfalls 1 bis 2 C-Atome durch ein oder mehrere Heteroatome ausgewählt aus der Gruppe Sauerstoff, Stickstoff oder Schwefel ersetzt sein

Als Alkenylgruppen (auch soweit sie Bestandteil anderer Reste sind) werden verzweigte und unverzweigte Alkylengruppen mit 2 bis 10 Kohlenstoffatomen, bevorzugt 2 - 6 Kohlenstoffatomen, besonders bevorzugt 2 - 3 Kohlenstoffatomen betrachtet, soweit sie mindestens eine Doppelbindung aufweisen. Beispielsweise werden genannt: Ethenyl, Propenyl, Butenyl, Pentenyl etc. Sofern nicht anders genannt, sind von den vorstehend genannten Bezeichnungen Propenyl, Butenyl etc. sämtliche der möglichen isomeren Formen umfaßt. Beispielsweise umfaßt die Bezeichnung Butenyl 1-Butenyl, 2-Butenyl, 3-Butenyl, 1-Methyl-1-Propenyl, 1-Methyl-2-Propenyl, 2-Methyl-1-Propenyl, 2-Methyl-2-Propenyl und 1-Ethyl-1-Ethenyl.
In den vorstehend genannten Alkenylgruppen können, soweit nicht anders beschrieben, gegebenenfalls ein oder mehrere Wasserstoffatome durch andere Reste ersetzt sein. Beispielsweise können diese Alkylgruppen durch die Halogenatome Fluor substituiert sein. Es können gegebenenfalls auch alle Wasserstoffatome der Alkenylgruppe ersetzt sein.

Als Alkinylgruppen (auch soweit sie Bestandteil anderer Reste sind) werden verzweigte und unverzweigte Alkinylgruppen mit 2 bis 10 Kohlenstoffatomen bezeichnet, soweit sie mindestens eine Dreifachbindung aufweisen, beispielsweise Ethinyl, Propargyl, Butinyl, Pentinyl, Hexinyl etc., vorzugsweise Ethinyl oder Propinyl.

In den vorstehend genannten Alkinylgruppen können, soweit nicht anders beschrieben, gegebenenfalls ein oder mehrere Wasserstoffatome durch andere Reste ersetzt sein. Beispielsweise können diese Alkylgruppen Fluor substituiert sein. Es können gegebenenfalls auch alle Wasserstoffatome der Alkinylgruppe ersetzt sein.

Der Begriff Aryl steht für ein aromatisches Ringsystem mit 6 bis 14 Kohlenstoffatomen, vorzugsweise 6 oder 10 Kohlenstoffatomen, bevorzugt Phenyl, das, soweit nicht anders beschrieben, beispielsweise einen oder mehrere der nachfolgend genannten Substituenten tragen kann: OH, NO₂, CN, OMe, - OCHF₂, -OCF₃, -NH₂, Halogen, vorzugsweise Fluor oder Chlor, C₁-C₁₀-Alkyl, vorzugsweise C₁-C₅-Alkyl, bevorzugt C₁-C₃-Alkyl, besonders bevorzugt Methyl oder Ethyl, -O-C₁-C₃-Alkyl, vorzugsweise -O-Methyl oder -O-Ethyl, -COOH, -COO-C₁-C₄-Alkyl, vorzugsweise -O-Methyl oder -O-Ethyl, -CONH₂, .
Als Heteroarylreste, in denen bis zu zwei C-Atome durch ein oder zwei Stickstoffatome ersetzt sind werden beispielsweise, Pyrrol, Pyrazol, Imidazol, Triazol, Pyridin, Pyrimidin, , genannt, wobei jeder der vorstehend genannten Heteroarylringe gegebenenfalls ferner an einen Benzolring anneliert sein kann, vorzugsweise Benzimidazol, und wobei diese Heterocyclen ,soweit nicht anders beschrieben, beispielsweise einen oder mehrere der nachfolgend genannten Substituenten tragen können: F, Cl, Br, OH, OMe, Methyl, Ethyl, CN, CONH₂, NH₂, gegebenenfalls substituiertes Phenyl, gegebenenfalls substituiertes Heteroaryl, vorzugsweise gegebenenfalls substituiertes Pyridyl.

Als Cycloalkylreste werden Cycloalkylreste mit 3 - 12 Kohlenstoffatomen beispielsweise Cyclopropyl, Cyclobutyl, Cyclopentyl, Cyclohexyl, Cycloheptyl oder Cyclooctyl, vorzugsweise Cyclopropyl, Cyclopentyl oder Cyclohexyl bezeichnet, wobei jeder der vorstehend genannten Cycloalkylreste gegebenenfalls ferner einen oder mehrere Substituenten tragen kann, beispielsweise: OH, NO₂, CN, OMe, -OCHF₂, -OCF₃, -NH₂ oder Halogen, vorzugsweise Fluor oder Chlor, C₁-C₁₀-Alkyl, vorzugsweise C₁-C₅-Alkyl, bevorzugt C₁-C₃-Alkyl, besonders bevorzugt Methyl oder Ethyl, -O-C₁-C₃-Alkyl, vorzugsweise -O-Methyl oder -O-Ethyl, - COOH, -COO-C₁-C₄-Alkyl, vorzugsweise -COO-Methyl oder -COO-Ethyl oder -CONH₂. Besonders bevorzugte Substituenten der Cycloalkylreste sind =O, OH, NH₂, Methyl oder F.

Als Cycloalkenylreste werden Cycloalkylreste mit 3 - 12 Kohlenstoffatomen, die mindestens eine Doppelbindung aufweisen, beispielsweise Cyclopropenyl, Cyclobutenyl, Cyclopentenyl, Cyclohexenyl oder Cycloheptenyl, vorzugsweise Cyclopropenyl, Cyclopententyl oder Cyclohexenyl bezeichnet, wobei jeder der vorstehend genannten Cycloalkenylreste gegebenenfalls ferner einen oder mehrere Substituenten tragen kann.

"=O" bedeutet ein über eine Doppelbindung verknüpftes Sauerstoffatom.

Als Heterocycloalkylreste werden, soweit in den Definitionen nicht anders beschrieben, 3 bis 12 gliedrige, vorzugsweise 5- ,6- oder 7-gliedrige, gesättigte oder ungesättigte Heterocyclen, die als Heteroatome Stickstoff, Sauerstoff oder Schwefel enthalten können, beispielsweise Tetrahydrofuran, Tetrahydrofuranon, γ-Butylrolacton, α-Pyran, γ-Pyran, Dioxolan, Tetrahydropyran, Dioxan, Dihydrothiophen, Thiolan, Dithiolan, Pyrrolin, Pyrrolidin, Pyrazolin, Pyrazolidin, Imidazolin, Imidazolidin, Tetrazol, Piperidin, Pyridazin, Pyrimidin, Pyrazin, Piperazin, Triazin, Tetrazin, Morpholin, Thiomorpholin, Diazepan, Oxazin, Tetrahydro-oxazinyl, Isothiazol, Pyrazolidin genannt, vorzugsweise Morpholin, Pyrrolidin, Piperidin oder Piperazin, genannt, wobei der Heterocyclus gegebenenfalls Substituenten tragen kann , beispielsweise C₁-C₄-Alkyl, vorzugsweise. Methyl, Ethyl oder Propyl.

Als Polycycloalkylreste werden gegebenenfalls substituierte, Bi-, Tri-tetra- oder pentacyclische Cycloalkylreste, beispielsweise Pinan, 2,2,2-Octan, 2,2,1-Heptan oder Adamantan, bezeichnet. Als Polycycloalkenylreste werden gegebenenfalls verbrückte oder/und substituierte, 8- gliedrige Bi-, Tri-tetra- oder pentacyclische Cycloalkenyllreste, vorzugsweise Bicycloalkenyl- oder Tricycloalkenylreste, sofern sie mindestens eine Doppelbindung aufweisen, beispielsweise .Norbornen, bezeichnet.
Als Spiroalkylreste werden gegebenenfalls substituierte spirocyclische C₅-C₁₂ Alkylreste bezeichnet.

Als Halogen wird im allgemeinen Fluor, Chlor, Brom oder Jod bezeichnet, vorzugsweise Fluor, Chlor oder Brom, besonders bevorzugt Chlor.

Als Abgangsgruppe A wird, gleich oder verschieden, eine Abgangsgruppe wie beispielsweise -o-Methyl, -SCN, Chlor, Brom, lod, Methansulfonyl, Trifluormethansulfonyl oder p-Toluolsulfonyl, vorzugsweise Chlor bezeichnet.

Die erfindungsgemäßen Verbindungen können in Form der einzelnen optischen Isomeren, Mischungen der einzelnen Enantiomeren, Diastereomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren, beispielsweise Chlor- oder Bromwasserstoffsäure, oder organische Säuren, wie beispielsweise Oxal-, Fumar-, Diglycol- oder Methansulfonsäure, vorliegen.

Der Substituent R¹ kann Wasserstoff oder ein Rest ausgewählt aus der Gruppe bestehend aus gegebenenfalls substituiertem und/oder verzweigtem C₁-C₆-Alkyl, vorzugsweise Methyl oder Ethyl, besonders bevorzugt Methyl oder Ethyl.

Der Substituent R² kann Wasserstoff oder ein Rest ausgewählt aus der Gruppe bestehend aus gegebenenfalls substituiertem und/oder verzweigtem C₁-C₆-Alkyl, vorzugsweise Methyl oder Ethyl.

R¹ und R² können gemeinsam eine 2- bis 5-gliedrige Alkylbrücke, vorzugsweise eine Ethylen-, Propylen- oder Butylenbrücke, die 1 bis 2 Heteroatome enthalten kann,vorzugsweise Sauerstoff oder Stickstoff, bedeuten. besonders bevorzugt Ethylen, Propylen.

Der Substituent R³ kann Wasserstoff oder ein Rest ausgewählt aus der Gruppe bestehend aus gegebenenfalls substituiertem und/oder verzweigtem C₁-C₁₂-Alkyl, vorzugsweise Ethyl, Propyl, Butyl, Pentyl oder Hexyl, besonders bevorzugt Propyl, Butyl, Pentyl oder Hexyl, C₂-C₁₂-Alkenyl, vorzugsweise C₅-C₇-Alkenyl, C₂-C₁₂-Alkinyl, vorzugsweise C₅-C₇-Alkinyl und C₆-C₁₄-Aryl, vorzugsweise Phenyl ein Rest ausgewählt aus der Gruppe bestehend aus gegebenenfalls substituiertem und/oder verbrücktem C₃-C₁₂-Cycloalkyl, vorzugsweise Cyclopentyl oder Cyclohexyl, C₃-C₁₂-Cycloalkenyl, vorzugsweise. C₅-C₇-Cycloalkenyl, C₇-C₁₂-Polycycloalkyl, C₇-C₁₂-Polycycloalkenyl, C₅-C₁₂-Spirocycloalkyl, C₃-C₁₂-Heterocycloalkyl, vorzugsweise Pyranyl oder Piperinyl, Pyrrolidinyl, Pyrazinyl oder Morpholinyl, das 1 bis 2 Heteroatome enthält, vorzugsweise Sauerstoff oder Stickstoff, und C₃-C₁₂-Heterocycloalkenyl, das 1 bis 2 Heteroatome enthält, vorzugsweise Sauerstoff oder Stickstoff, bedeuten. Insbesondere bevorzugt bedeutet der Substituent R³ Isopropyl, lsobutyl, Isopentyl, Cyclopentyl, Phenyl oder Cyclohexyl .

R¹ und R³ oder R² und R³ können gemeinsam eine gesättigte oder ungesättigte C₃-C₄-Alkylbrücke, die 1 Heteroatom enthalten kann, vorzugsweise Sauerstoff oder Stickstoff, bedeuten.

Der Substituent R⁴ kann ein Rest ausgewählt aus der Gruppe bestehend aus Wasserstoff, -CN, Hydroxy, -NR⁶R⁷ und Halogen, vorzugsweise Chlor oder Fluor, besonders bevorzugt Chlor oder
ein Rest ausgewählt aus der Gruppe bestehend aus gegebenenfalls substituiertem C₁-C₆-Alkyl, vorzugsweise Methyl, Ethyl oder Propyl, C₂-C₆-Alkenyl, vorzugsweise Ethenyl oder Propenyl, C₂-C₆-Alkinyl, vorzugsweise Etyhinyl, Propinyl oder Butinyl, C₁-C₅-Alkyloxy, vorzugsweise Methoxy, Ethoxy oder Propargyloxy, C₂-C₅-Alkenyloxy, C₂-C₅-Alkinyloxy, C₁-C₆-Alkylthio, C₁-C₆-Alkylsulfoxo und C₁-C₆-Alkylsulfonyl bedeuten.

Insbesondere bevorzugt bedeutet der Substituent R⁴ Methoxy, Methyl, Ethoxy, Ethyl, Propargyloxy oder Chlor.

L kann einen Linker ausgewählt aus der Gruppe bestehend aus gegebenenfalls substituiertem C₂-C₁₀-Alkyl, vorzugsweise Ethyl, Propyl, Butyl oder Pentyl, C₂-C₁₀-Alkenyl, C₆-C₁₄-Aryl, vorzugsweise Phenyl, -C₂-C₄-Alkyl-C₆-C₁₄-Aryl, -C₆-C₁₄-Aryl-C₁-C₄-Alkyl, vorzugsweise -Phenyl-methyl, gegebenenfalls verbrücktem C₃-C₁₂-Cycloalkyl, vorzugsweise Cyclohexyl, und Heteroaryl, das 1 oder 2 Stickstoffatome enthält, bedeuten.
n bedeutet 0 oder 1
m bedeutet 1 oder 2, vorzugsweise 1.
R⁵ kann ein Rest, ausgewählt aus der Gruppe bestehend aus gegebenenfalls substituiertem Morpholinyl, Piperidinyl, Piperazinyl, Piperazinylcarbonyl, Pyrrolidinyl, Tropenyl, R⁸-Diketomethylpiperazinyl, Sulfoxomorpholinyl, Sulfonylmorpholinyl, Thiomorpholinyl, -NR⁸R⁹ und Azacycloheptyl, vorzugsweise Piperidinyl, Morpholinyl, Pyrrolidinyl, Sulfoxomorpholiny, Piperazinyl, Thiomorpholinyl oder Tropenyl, bedeuten.

Die Reste R⁶ und R⁷ können, gleich oder verschieden sein und Wasserstoff oder C₁-C₄-Alkyl, vorzugsweise Methyl oder Ethyl, bedeuten.
Die Reste R⁸ und R⁹ können unsubstituierte Stickstoffsubstituenten an R⁵ sein, gleich oder verschieden sein und entweder Wasserstoff oder ein Rest ausgewählt aus der Gruppe bestehend aus C₁-C₆-Alkyl, vorzugsweise Methyl, Ethyl oder Propyl, -C₁-C₄-Alkyl-C₃-C₁₀-Cycloalkyl, vorzugsweise -CH₂-Cyclopropyl, C₃-C₁₀-Cycloalkyl, C₆-C₁₄-Aryl, vorzugsweise Phenyl,-C₁-C₄-Alkyl-C₆-C₁₄-aryl, vorzugsweise Benzyl, Pyranyl, Pyridinyl, Pyrimidinyl, Pyranyl, C₁-C₄-Alkyloxycarbonyl, C₆-C₁₄-Arylcarbonyl-, C₁-C₄-Alkylcarbonyl-, C₆-C₁₄-Arylmethyloxycarbonyl-, C₆-C₁₄-Arylsulfonyl-, C₁-C₄-Alkylsulfonyl-, und C₆-C₁₄-Aryl-C₁-C₄-Alkylsulfonyl-, bedeuten.
Insbesondere bevorzugt bedeutet der Substituent R⁸ Methyl, Ethyl oder Propyl. Insbesondere bevorzugt bedeutet der Substituent R⁹ Methyl, Ethyl oder Propyl.

Der R¹⁰ kann ein Substituent ausgewählt aus der Gruppe bestehend aus OH, NH2-LR5, -O-Methyl und -O-Ethyl, vorzugsweise OH, LR5, -O-Methyl oder -O-Ethyl bedeuten.

Alle in der Bedeutung für R¹ bis R¹⁰ genannten Reste können gegebenenfalls verzweigt und/oder substituiert sein.

Die Herstellung der erfindungsgemäßen Verbindungen kann nach den im folgenden beschriebenen Syntheseverfahren A erfolgen, wobei die Substituenten der allgemeinen Formeln (A1) bis (A9) die zuvor genannten Bedeutungen haben. Dieses Verfahren ist als Erläuterung der Erfindung zu verstehen ohne selbige auf deren Gegenstand zu beschränken.

### Verfahren A

### Stufe 1 A

Eine Verbindung der Formel (A1) wird mit einer Verbindung der Formel (A2) zu einer Verbindung der Formel (A3) umgesetzt (Schema 1A). Diese Reaktion kann gemäß WO 0043369 oder WO 0043372 durchgeführt werden. Verbindung (A1) ist kommerziell, beispielsweise bei City Chemical LLC, 139 Allings Crossing Road, West Haven, CT, 06516, USA, erhältlich. Verbindung (A2) kann nach literaturbekannten Vorschriften *(a)* F. Effenberger, U. Burkhart, J. Willfahrt *Liebigs Ann. Chem.* **1986,** 314-333. b) T. Fukuyama, C.-K. Jow, M. Cheung, *Tetrahedron Lett.* **1995,** *36*, 6373-6374. c) R. K. Olsen, *J. Org. Chem.* **1970,** *35*,1912-1915. d) F.E. Dutton, B.H. Byung *Tetrahedron Lett.* **1998,** *30,* 5313-5316. e) J. M. Ranajuhi, M. M. Joullie *Synth. Commun.* **1996**, *26,* 1379-1384. hergestellt werden.

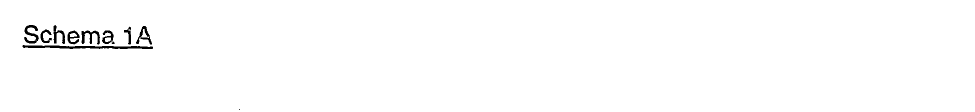

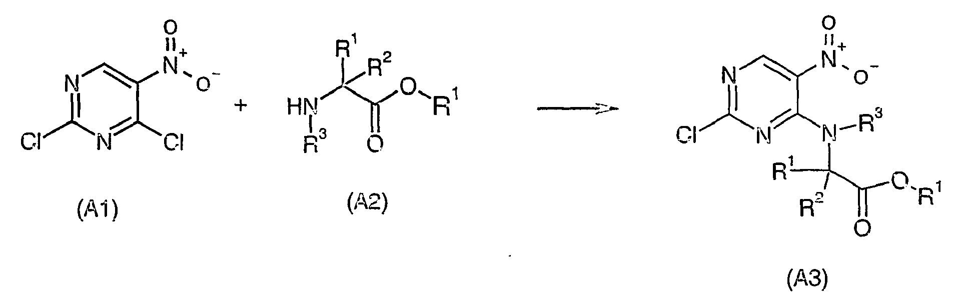

In Stufe 1A werden 1 Äquivalent der Verbindung (A1) und 1 bis 1,5 Äquivalente, bevorzugt 1,1 Äquivalente einer Base, vorzugsweise Kaliumcarbonat, Kaliumhydrogencarbonat, Natriumcarbonat oder Natriumhydrogencarbonat, Calciumcarbonat, besonders bevorzugt Kaliumcarbonat, in einem Verdünnungsmittel gegebenfalls mit Wasser gemischt, beispielsweise Aceton, Tetrahydrofuran, Diethylether, Cyclohexan, Petrolether oder Dioxan, vorzugsweise Cyclohexan oder Diethylether, gerührt.
Bei einer Temperatur von 0 bis 15 °C, bevorzugt 5 bis 10 °C, wird 1 Äquivalent einer Aminosäure der Formel (A2) gelöst in einem organischen Lösungsmittel, beispielsweise Aceton, Tetrahydrofuran, Diethylether, Cyclohexan oder Dioxan, zugetropft. Das Reaktionsgemisch wird unter Rühren auf eine Temperatur von 18°C bis 30 °C, vorzugsweise etwa 22°C, erwärmt und anschließend weitere 10 bis 24 Stunden, vorzugsweise etwa 12 Stunden, weitergerührt. Danach wird das Verdünnungsmittel abdestilliert, der Rückstand mit Wasser versetzt und das Gemisch zwei bis dreimal eines organischen Lösungsmittels beispielsweise, Diethylether oder Ethylacetat, vorzugsweise Ethylacetat extrahiert. Die vereinigten organischen Extrakte werden getrocknet und das Lösungsmittel abdestilliert. Der Rückstand (Verbindung A3) kann ohne vorherige Aufreinigung in Stufe 2 eingesetzt werden.

### Stufe 2A

Die in Stufe 1A erhaltene Verbindung (A3) wird an der Nitrogruppe reduziert und zur Verbindung der Formel (A4) zyklisiert (Schema 2A).

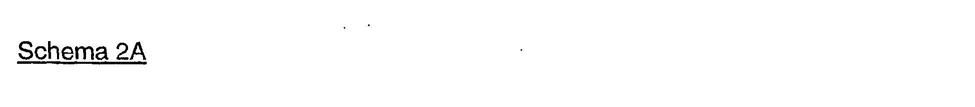

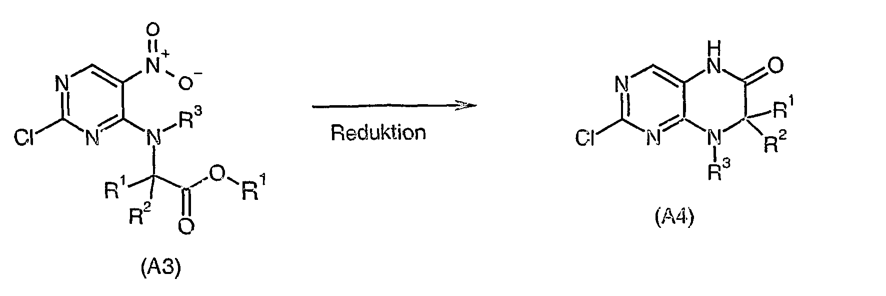

In Stufe 2A werden 1 Äquivalent der Nitroverbindung (A3) in einer Säure gelöst, vorzugsweise Eisessig, Ameisensäure oder wässrige Salzsäure, bevorzugt Eisessig, und auf 50 bis 70 °C, vorzugsweise etwa 60 °C, erwärmt. Anschließend wird ein Reduktionsmittel, beispielsweise Zink, Zinn, oder Eisen, bevorzugt Eisenpulver, bis zum Abschluß der exothermen Reaktion hinzugefügt und 0,2 bis 2 Stunden, vorzugsweise 0,5 Stunden, bei 100 bis 125 °C, vorzugsweise bei etwa 117 °C, gerührt. Nach Abkühlung auf Raumtemperatur wird das Eisensalz abfiltriert und das Lösungsmittel abdestilliert. Der Rückstand wird in einem Lösungsmittel oder Lösungsmittelgemisch, beispielsweise Ethylacetat oder Dichlormethan/ Methanol 9/1 und halbgesättigte NaCl-Lösung, aufgenommen und beispielsweise über Kieselgur filtriert. Die organische Phase wird getrocknet und eingeengt. Der Rückstand (Verbindung (A4)) kann chromatographisch oder mittels Kristallisation gereinigt oder als Rohprodukt in Stufe 3A der Synthese eingesetzt werden.

### Stufe 3A

Die in Stufe 2A erhaltene Verbindung (A4) kann durch elektrophile Substitution gemäß Schema 3A zur Verbindung der Formel (A5) umgesetzt werden.

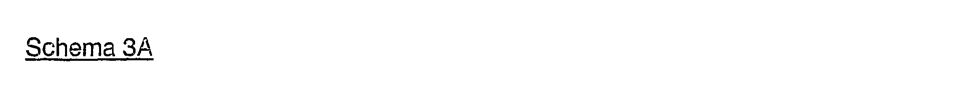

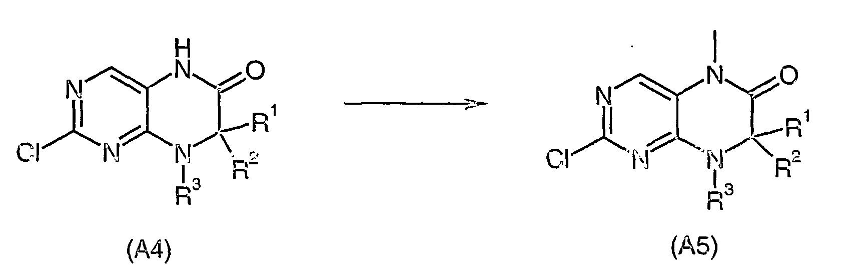

In Stufe 3A werden 1 Äquivalent des Amides der Formel (A4) in einem organischen Lösungsmittel, beispielsweise Dimethylformamid oder Dimethylacetamid, vorzugsweise Dimethylacetamid, gelöst und auf etwa -5 bis 5 °C, vorzugsweise 0°C, abgekühlt.
Anschließend wird 0,9 bis 1,3 Äquivalente Natriumhydrid und 0,9 bis 1,3 Äquivalente eines Methylierungsreagenzes, beispielsweise Methyliodid zugegeben. Das Reaktionsgemisch wird 0,1 - 3 Stunden, vorzugsweise etwa 1 Stunde, bei etwa 0 bis 10 °C, vorzugsweise bei etwa 5 °C, gerührt und kann gegebenenfalls weitere 12 Stunden bei diesem Temperaturbereich stehengelassen werden. Die Reaktionsmischung wird auf Eiswasser gegossen und der Niederschlag isoliert. Der Rückstand (Verbindung (A5)) kann chromatographisch, vorzugsweise über Kieselgel, oder mittels Kristallisation gereinigt oder als Rohprodukt in Stufe 4A der Synthese eingesetzt werden.

### Stufe 4A

Die Aminierung der in Stufe 3A erhaltenen Verbindung (A5) zur Verbindung der Formel (A9) (Schema 4A) kann nach den literaturbekannten Methoden der Varianten 4.1 A (a) M.P.V. Boarland, J.F.W. McOmie *J. Chem. Soc.* **1951**, 1218-1221; b) F. H. S. Curd, F. C. Rose *J. Chem. Soc.* **1946**, 343-348., 4.2 A (a) Banks *J. Am. Chem. Soc.* **1944**, *66,* 1131; b) Ghosh and Dolly *J*. *Indian Chem. Soc.* **1981**, *58*, 512-513 durchgeführt werden. c) N. P. Reddy and M. Tanaka *Tetrahedron Lett.* **1997**, *38*, 4807-4810.

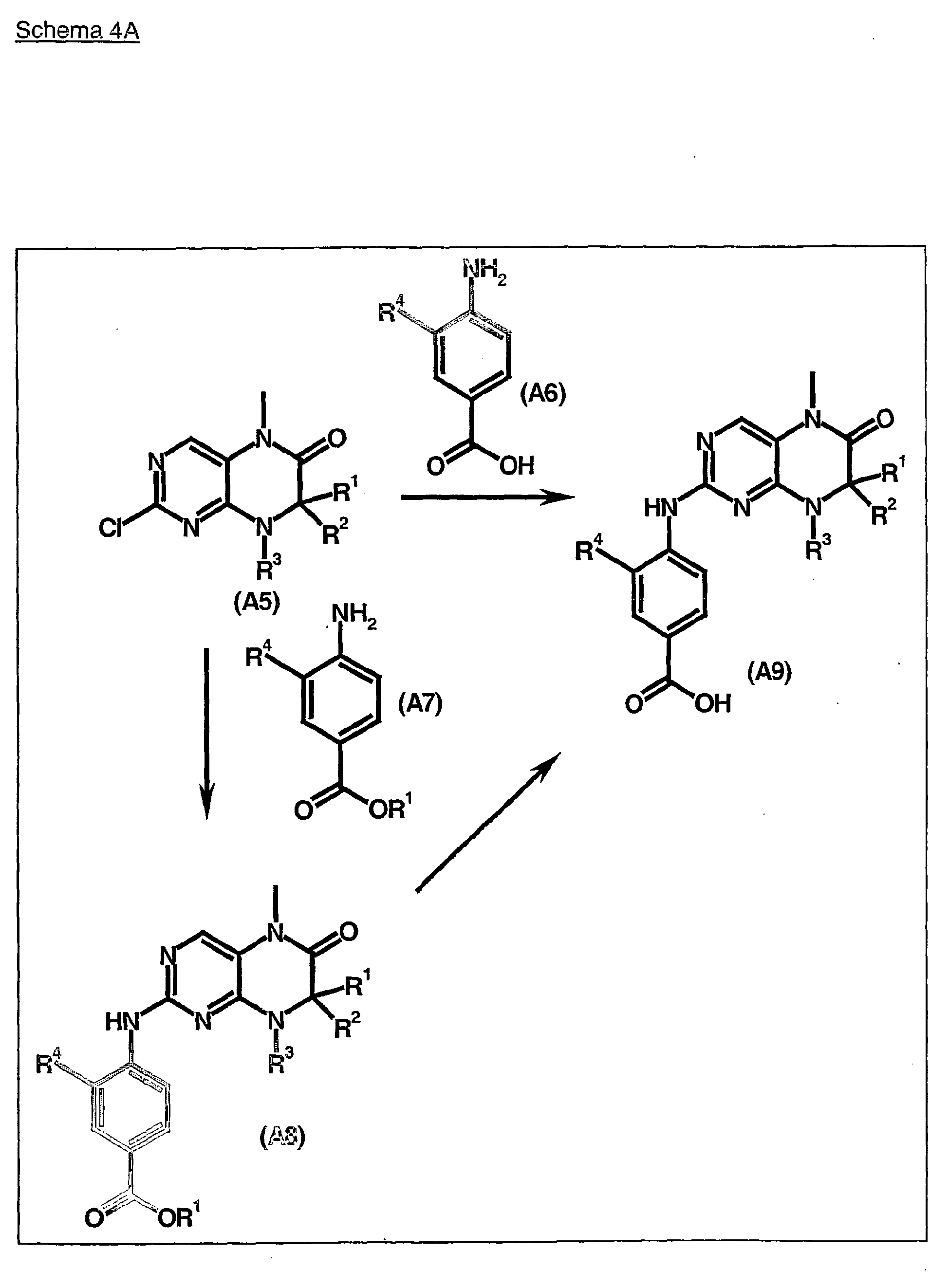

Beispielsweise werden in Variante 4.1 A 1 Äquivalent der Verbindung (A5) und 1 bis 3 Äquivalente, vorzugsweise etwa 2 Äquivalente der Verbindung (A6) ohne Lösungsmittel oder einem organischen Lösungsmittel wie beispielsweise Sulfolan, Dimethyliorrnamid, Dimethylacetamid, Toluol, N-ethylpyrrolidon, Dimethylsulfoxid, oder Dioxan, bevorzugt Sulfolan über 0,1 bis 4 Stunden, vorzugsweise 1 Stunde, bei 100 bis 220 °C, vorzugsweise bei etwa 160 °C erhitzt. Nach dem Abkühlen wird durch Zugabe von org. Lösungsmitteln oder Lösungsmittelgemischen bespielsweise Diethylether/Methanol, Ethylacetat, Methylenchlorid, oder Diethylether, vorzugsweise Diethylether/Methanol 9/1, das Produkt (A9) kristallisiert oder chromatographisch gereinigt.

Beispielsweise wird in Variante 4.2 A 1 Äquivalent der Verbindung (A5) und 1 bis 3 Äquivalente der Verbindung (A6) mit Säure, beispielsweise 1-10 Äquivalente 10-38%ige Salzsäure und/oder einem Alkohol beispielsweise Ethanol, Propanol, Butanol, bevorzugt Ethanol unter Rückfluss 1 bis 48 Stunden, vorzugsweise etwa 5 Stunden, gerührt.
Das ausgefallene Produkt (A9) wird abfiltriert und gegebenenfalls mit Wasser gewaschen, getrocknet und aus einem geeigneten org. Lösungsmittel kristallisiert.

Beispielsweise wird in Variante 4.3 A 1 Äquivalent der Verbindung (A5) und 1 bis 3 Äquivalente der Verbindung (A7), in einem Lösungsmittel, beispielsweise Toluol oder Dioxan gelöst und mit einem Phophinliganden, beispielsweise 2,2'-Bis-(diphenylphosphino)-1,1 `-binaphthyl und einem Palladiumkatalysator, beispielsweise Tris(dibenzylidenaceton)-dipalladium(0) und einer Base, beispielsweise Cäsiumcarbonat versetzt und 1-24h, vorzugsweise 17 h unter Rückfluss gekocht. Die Reaktionsmischung wird beispielsweise über Kieselgel gereinigt und das Produkt (A8) aus der Lösung isoliert oder durch geeignete Kristallisation erhalten.

Das Produkt (A8) wird in einem geeigneten Lösungsmittel, beispielsweise Dioxan gelöst und mit Säure, beispielsweise halbkonzentrierter Salzsäure, beispielsweise im Verhältnis Lösungsmittel zu Säure 3: 1 zugegeben. Anschließend wird für 1 - 48 h unter Rückfluss erhitzt, beispielsweise 12 h und der ausgefallene Niederschlag isoliert. Gegebenfalls wird das Produkt (A9) durch Kristallisation gereinigt.

### Stufe 5A

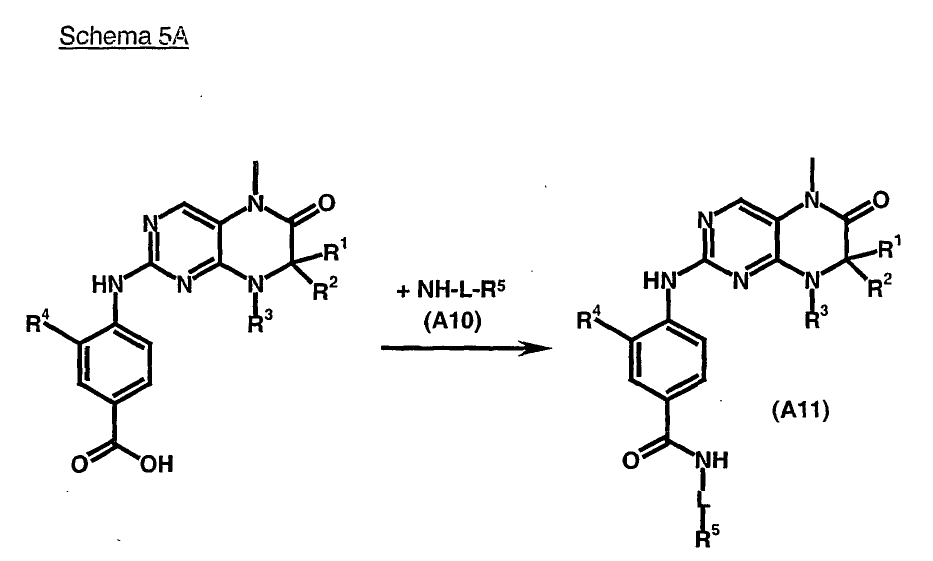

### Variante 5.1 A:

Beispielsweise werden 1 Äquivalent der Verbindung (A9) mit 1 Äquivalent eines Aktivierungsreagenzes, beispielesweise O-Benzotriazolyl-N,N,N',N'-tetramethyluroniumtetrafluoroborat (TBTU) und einer Base, beispielsweise etwa 1,5 Äquivalente, Diisopropylethylamin (DIPEA) in einem organischen Verdünnungsmittels, beispielsweise Dichlormethan, Tetrahydrofuran, Dimethylformamid, N-Methylpyrrolidion, Dimethylacetamid, vorzugsweise Dichlormethan oder Dimethylformamid, gelöst. Nach Zugabe von 1 Äquivalent des Amins (A10) wird das Reaktionsgemisch 0,1 bis 24 Stunden, vorzugsweise etwa 2 Stunden bei 20°C bis 100°C gerührt. Über beispielsweise Kristallisation oder chromatographische Reinigung wird das Produkt der Formel (A11) erhalten.

Die neuen Verbindungen der allgemeinen Formel (I) können in Analogie zu nachfolgenden Synthesebeispielen synthetisiert werden. Diese Beispiele sind allerdings nur als exemplarische Vorgehensweise zur weitergehenden Erläuterung der Erfindung zu verstehen, ohne selbige auf dessen Gegenstand zu beschränken.

Die Herstellung einiger zur Synthese der Beispiele eingesetzten Zwischenverbindungen wird ebenfalls im folgenden beschrieben.

### Herstellung der Säuren

Zur Synthese der Verbindungen Bsp. 94 und Bsp. 95 wird zunächst eine 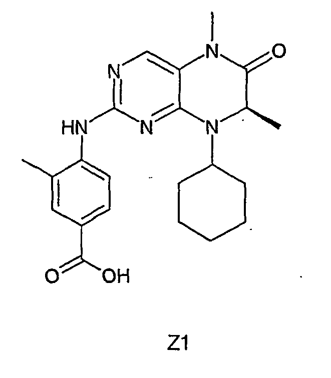 Zwischenverbindung Z1
wie im folgenden beschrieben hergestellt.

50.0 g (0.48 mol) D-Alaninmethylester x HCl und 49.1 g (0.50 mol) Cyclohexanon wurden in 300 mL Dichlormethan vorgelegt und anschließend mit 41.0 g (0.50 mol) Natriumacetat und 159.0 g (0.75 mol) Natriumtriacetoxyborhydrid versetzt. Es wurde über Nacht gerührt und dann 300 mL 10%ige Natriumhydrogencarbonat-Lösung zugegeben. Die wässrige Phase wurde mit Dichlormethan extrahiert. Die vereinten organischen Phasen wurden mit 10%iger Natriumhydrogencarbonat-Lösung gewaschen, über Na₂SO₄ getrocknet und eingeengt.
Ausbeute: 72.5 g einer Verbindung Z1a (klare Flüssigkeit)

72.5 g der Verbindung Z1a wurden in 500 mL Wasser vorgelegt und 76.6 g (0.39 mol) 2,4-Dichlor-5-nitropyrimidin in 500 mL Diethylether zugegeben. Bei einer Temperatur von-5°C wurden 100 mL 10%ige Kaliumhydrogencarbonat-Lösung zugetropft. Es wurde 3 h bei -5°C und weitere 12 h bei Raumtemperatur gerührt. Die organische Phase wurde abgetrennt und über Na₂SO₄ getrocknet. Beim Einengen kristallisierte das Produkt aus.
Ausbeute: 48.0 g einer Verbindung Z1b (gelbe Kristalle)

48.0 g der Verbindung Z1b wurden in 350 mL Eisessig gelöst und auf 60°C erwärmt. Es wurden portionsweise 47.5 g Eisen-Pulver zugegeben, wobei die Temperatur auf 105°C stieg. Das Reaktionsgemisch wurde drei Stunden bei 80°C gerührt, anschließend heiß über Cellulose filtriert und eingeengt. Der Rückstand wurde in Wasser und Essigsäureethylester ausgerührt, abgesaugt und der hellgraue Niederschlag mit Essigsäureethylester gewaschen. Das Filtrat wurde mit verdünntem Ammoniak und Wasser gewaschen, die organische Phase über Na₂SO₄ getrocknet, über Aktivkohle filtriert und eingeengt. Es wurde weiterer hellgraue Feststoff erhalten.
Ausbeute: 29.5 g einer Verbindung Z1c (hellgraue Kristalle)

32.1 g der Verbindung Z1c wurden in 300 mL Dimethylacetamid vorgelegt und mit 13 mL (0.2 mol) Methyliodid versetzt. Bei -5°C wurden portionsweise 6.4 g (0.16 mol) Natriumhydrid als 60%ige Dispersion in Mineralöl zugegeben. Nach 2 h wurde das Reaktionsgemisch auf 800 mL Eiswasser gegossen. Der ausgefallene Niederschlag wurde abgesaugt und mit Petrolether gewaschen.
Ausbeute: 33.0 g einer Verbindung Z1d (beige Kristalle)

4.0 g der Verbindung Z1d und 2.3 g (15 mmol) 4-Amino-3-methylbenzoesäure wurden in 50 mL Ethanol und 120 mL Wasser suspendiert, mit 2 mL konz. Salzsäure versetzt und 48 h unter Rückfluss gekocht. Der beim Abkühlen ausgefallene Niederschlag wurde abgesaugt und mit Wasser, Ethanol und Diethylether gewaschen.
Ausbeute: 2.9 g einer Verbindung Z1 (farblose Kristalle)

Zur Synthese der Verbindungen Bsp. 188 und Bsp. 203 wird zunächst eine Zwischenverbindung Z2 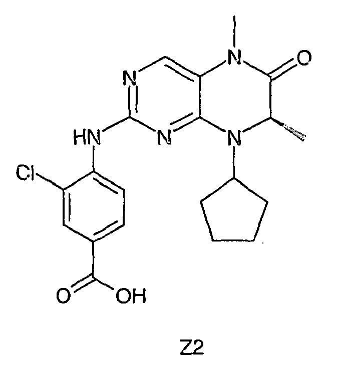 wie im folgenden beschrieben hergestellt.

Eine Lösung von128.2 g (0.83 mol) D-Alaninethylester x HCI und 71.5 g (0.85 mol) Cyclopentanon in 1500 mL Dichlormethan wurde mit 70.1 (0.85 mol) Natriumacetat und 265.6 g (1.25 mol) Natriumtriacetoxyborhydrid versetzt. Die Reaktionsmischung wurde 12 h gerührt und dann in 1.5 L einer 10%igen Natriumhydrogencarbonat-Lösung gegossen. Die wässrige Phase wurde mit Dichlormethan extrahiert. Die vereinten organischen Phasen wurden über Na₂SO₄ getrocknet und eingeengt.
Ausbeute: 143.4 g einer Verbindung Z2a (farbloses Öl)

66.0 g der Verbindung Z2a wurden in 500 mL Wasser vorgelegt und mit 85.0 g (0.44 mol) 2,4-Dichlor-5-nitropyrimidin in 500 mL Diethylether versetzt. Bei -5°C wurden 100 mL 10%ige Kaliumhydrogencarbonat-Lösung zugetropft und die Reaktionsmischung 48 h bei Raumtemperatur gerührt. Die wässrige Phase wurde mit Diethylether extrahiert, die vereinten organischen Phasen über Na₂S0₄ getrocknet und eingeengt. Der dunkelrote Feststoff wurde mit Petrolether ausgerührt und abgesaugt.
Ausbeute: 88.0 g einer Verbindung Z2b (gelbe Kristalle)

88.0 g der Verbindung Z2b wurden in 1000 mL Eisessig gelöst und bei 60°C portionsweise mit 85 g Eisen-Pulver versetzt, wobei die Temperatur auf 110°C anstieg. Es wurde 1 h.bei 60°C gerührt, anschließend heiß über Cellulose abgesaugt und eingeengt Der braune Feststoff wurde mit 700 mL Wasser ausgerührt und abgesaugt.
Ausbeute: 53.3 g einer Verbindung Z2c (leicht braune Kristalle)

53.3 g der Verbindung Z2c wurden in 300 mL Dimethylacetamid gelöst und mit 13 mL (0.21 mol) Methyliodid versetzt. Bei -5°C wurden 5.0 g (0.21 mol) Natriumhydrid als 60%ige Dispersion in Mineralöl portionsweise zugegeben. Nach 12 h wurde das Reaktionsgemisch auf 1000 mL Eiswasser gegossen und der entstandene Niederschlag abgesaugt.
Ausbeute: 40.0 g einer Verbindung Z2d (farblose Kristalle)

4.0 g der Verbindung Z2d und 2.8 g (16 mmol) 4-Amino-3-chlorbenzoesäure wurden in 25 mL Ethanol und 60 mL Wasser suspendiert, mit 3 mL konz. Salzsäure versetzt und 43 h unter Rückfluss gekocht. Der beim Abkühlen ausgefallene Niederschlag wurde abgesaugt und mit Wasser, Ethanol und Diethylether gewaschen.
Ausbeute: 0.9 g einer Verbindung Z2 (farblose Kristalle)

Zur Synthese der Verbindungen Bsp. 19, 21, 22, 23, 45, 55, 58,116,128, 131, 133, 134, 136, 138, 177, 217, 231, 239, 46, 134, 166 und 187 wird zunächst eine Zwischenverbindung Z3 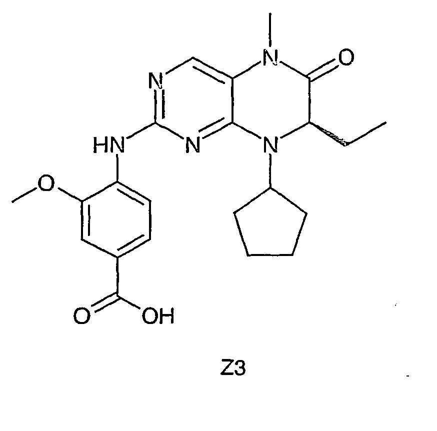 wie im folgenden beschrieben hergestellt.

54.0 g (0.52 mol) D-2-Aminobuttersäure wurden in 540 mL Methanol suspendiert und unter Eiskühlung langsam mit 132 g (1.1 mol) Thionylchlorid versetzt. Es wurde 1.5 h unter Rückfluß gekocht und anschließend eingeengt. Das verbliebene Öl wurde mit 540 mL tert-Butylmethylether versetzt und die entstandenen farblosen Kristalle abgesaugt.
Ausbeute: 78.8 g einer Verbindung Z3a (farblose Kristalle)

74.2 g der Verbindung Z3a und 43.5 mL (0.49 mol) Cyclopentanon wurden in 800 mL Dichlormethan gelöst. Nach Zugabe von 40.0 g (0.49 mol) Natriumacetat und 150.0 g (0.71 mol) Natriumtriacetoxyborhydrid bei 0°C wurde 12 h bei Raumtemperatur gerührt und dann 500 mL 20%ige Natriumhydrogencarbonat-Lösung zugegeben. Die wässrige Phase wurde mit Dichlormethan extrahiert. Die vereinten organischen Phasen wurden mit Wasser gewaschen, über MgSO₄ getrocknet und eingeengt.
Ausbeute: 85.8 g einer Verbindung Z3b (leicht gelbes Öl)

40.0 g der Verbindung Z3b und 30.0 g (0.22 mol) Kaliumcarbonat wurden in 600 mL Aceton suspendiert und unter Eiskühlung mit 45.0 g (0.23 mol) 2,4-Dichlor-5-nitropyrimidin in 200 mL Aceton versetzt. Nach 12 h wurden weitere 5.0 g 2,4-Dichlor-5-nitropyrimidin zugegeben und 3 h gerührt. Die Reaktionsmischung wurde eingeengt, in 800 mL Essigsäureethylester und 600 mL Wasser aufgenommen und die wässrige Phase mit Essigsäureethylester extrahiert. Die vereinten organischen Phasen wurden mit Wasser gewaschen, über MgSO₄ getrocknet und eingeengt.
Ausbeute: 75.0 g einer Verbindung Z3c (braunes Öl)

100 g der Verbindung Z3c wurden in 650 mL Eisessig gelöst und bei 70°C mit 20 g Eisen-Pulver portionsweise versetzt. Es wurde 1 h bei 70°C, dann 1.5 h bei 100°C gerührt und anschließend heiß über Kieselgur abfiltriert. Die Reaktionsmischung wurde eingeengt, in Methanol/Dichlormethan aufgenommen, auf Kieselgel aufgezogen und durch Soxhlet-Extraktion mit Essigsäureethylester gereinigt. Das Lösungsmittel wurde entfernt und der Rückstand mit Methanol ausgerührt.
Ausbeute: 30.0 g einer Verbindung Z3d (hellbraune Kristalle)

25.0 g der Verbindung Z3d und 6.5 mL (0.1 mol) Methyliodid wurden in 250 mL Dimethylacetamid vorgelegt und bei -10°C mit 3.8 g (0.95 mol) Natriumhydrid als 60%ige Dispersion in Mineralöl versetzt. Es wurde 20 min bei 0°C, dann 30 min bei Raumtemperatur gerührt und schließlich Eis zugegeben. Die Reaktionsmischung wurde eingeengt und mit 300 mL Wasser versetzt. Der entstandene Niederschlag wurde abgesaugt und mit Petrolether gewaschen.
Ausbeute: 23.0 g einer Verbindung Z3e (farbloser Feststoff)

6.0 g der Verbindung Z3e und 5.1 g (31 mmol) 4-Amino-3-methoxybenzoesäure wurden in 90 mL Ethanol und 350 mL Wasser suspendiert, mit 3.5 mL konz. Salzsäure versetzt und 48 h unter Rückfluss gekocht. Die Reaktionsmischung wurde eingeengt, der Rückstand mit Methanol/Diethylether verrührt und der entstandene Niederschlag abgesaugt.
Ausbeute: 6.3 g einer Verbindung Z3 (hellbeige Kristalle)

Zur Synthese der Verbindung Bsp. 81, 82, 93,137 wird zunächst eine Zwischenverbindung Z4 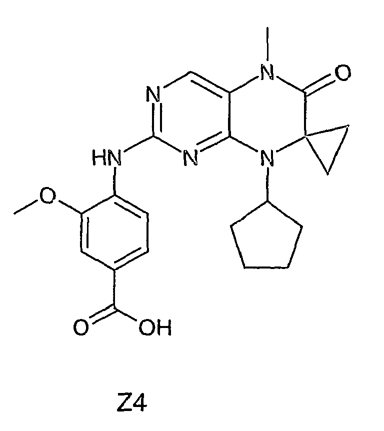 wie im folgenden beschrieben hergestellt.

25.0 g (0.19 mol) 1-Aminocyclopropan-1-carbonsäureethylester x HCl und 16.8 g (0.20 mol) Cyclopentanon wurden in 300 mL Dichlormethan gelöst und mit 16.4 g (0.20 mol) Natriumacetat und 61.7 g (0.29 mol) Natriumtriacetoxyborhydrid versetzt. Es wurde über Nacht gerührt und die Reaktionsmischung dann auf 400 mL 10%ige Natriumhydrogencarbonat-Lösung gegossen. Die wässrige Phase wurde mit Dichlormethan extrahiert. Die vereinten organischen Phasen wurden über Na₂SO₄ getrocknet und eingeengt.
Ausbeute: 34.5 g einer Verbindung Z4a (farbloses Öl)

Zu einer Mischung von 34.5 g der Verbindung Z4a in 350 mL Wasser wurden 42.5 g (0.22 mol) 2,4-Dichlor-5-nitropyrimidin in 350 mL Diethylether gegeben. Bei -5°C wurde mit 80 mL 10%iger Katiumhydrogencarbonat-Lösung versetzt und über Nacht bei Raumtemperatur gerührt. Die wässrige Phase wurde mit Diethylether extrahiert. Die vereinten organischen Phasen wurden über Na₂SO₄ getrocknet und eingeengt.
Ausbeute: 53.8 g einer Verbindung Z4b (braunes Öl)

20.1 g der Verbindung Z4b wurden in 200 mL Eisessig gelöst und bei 60°C mit 19.1 g Eisen-Pulver portionsweise versetzt, wobei die Temperatur auf 10h°C anstieg. Es wurde 3 h bei 60°C gerührt, anschließend über Cellulose abgesaugt und eingeengt. Der Rückstand wurde in Wasser und Essigsäureethylester ausgerührt und der gelbe Niederschlag abgesaugt. Das Filtrat wurde mit verdünntem Ammoniak und Wasser gewaschen, die organische Phase über Na₂SO₄ getrocknet und eingeengt. Nach Zugabe von Diethylether kristallisierte weiteres Produkt.
Ausbeute: 4.0 g einer Verbindung Z4c (gelbe Kristalle)

7.8 g der Verbindung Z4c und 2.6 mL (0.04 mol) Methyliodid wurden in 100 mL Dimethylacetamid gelöst und bei -5°C mit 1.5 g (0.04 mol) Natriumhydrid als 60%ige Dispersion in Mineralöl portionsweise versetzt. Nach 2 h wurde das Reaktionsgemisch auf Eiswasser gegossen und der entstandene Niederschlag abgesaugt.
Ausbeute: 7.5 g einer Verbindung Z4d (leicht braune Kristalle)

3.0 g der Verbindung Z4d und 1.9 g (11 mmol) 4-Amino-3-methoxybenzoesäure wurden in 40 mL Ethanol und 80 mL Wasser suspendiert, mit 2 mL konz. Salzsäure versetzt und 20 h unter Rückfluss gekocht. Es wurden weitere 0.5 g 4-Amino-3-methoxybenzoesäure zugegeben und 48 h unter Rückfluss gekocht. Der beim Abkühlen ausgefallene Niederschlag wurde abgesaugt und mit Wasser, Ethanol und Diethylether gewaschen.
Ausbeute: 2.1 g einer Verbindung Z4 (farblose Kristalle) Smp.: 222-223°C

Zur Synthese der Verbindungen Bsp. 162, 43, 53, 161, 202, 211, 215 und 212 wird zunächst eine Zwischenverbindung Z5 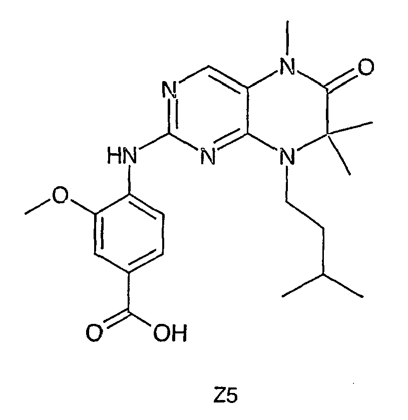 wie im folgenden beschrieben hergestellt.

Eine Mischung aus 73.4 mL (0.5 mol) 2-Bromisobuttersäureethylester, 87.1 mL (0.75 mol) 3-Methyl-1-butylamin, 82.5 g (0.6 mol) Natriumiodid und 76.0 g (0.6 mol) Kaliumcarbonat in 1000 mL Essigsäureethylester wurde 3 Tage unter Rückfluss gekocht. Vorhandene Salze wurden abfiltriert und das Filtrat eingeengt.
Ausbeute: 97.0 g einer Verbindung Z5a (rotes Öl)

49.0 g (0.25 mol) 2,4-Dichlor-5-nitropyrimidin und 38.3 g (0.28 mol) Kaliumcarbonat wurden in 500 mL Aceton suspendiert und bei 0°C mit 93.0 g der Verbindung Z5a in 375 mL Aceton versetzt. Die Reaktionsmischung wurde über Nacht bei Raumtemperatur gerührt, filtriert und eingeengt. Der in Essigsäureethylester gelöste Rückstand wurde mit Wasser gewaschen und die organische Phase über MgSO₄ getrocknet und eingeengt.
Ausbeute: 102.7 g einer Verbindung Z5b (braunes Öl)

22.7 g der Verbindung Z5b wurden in 350 mL Eisessig gelöst und bei 60°C mit 17.4 g Eisen-Pulver portionsweise versetzt. Nach beendeter Zugabe wurde 0.5 h unter Rückfluss gekocht, heiß abfiltriert und eingeengt. Der Rückstand wurde in 200 mL Dichlormethan/Methanol (9:1) aufgenommen und mit NatriumchloridLösung gewaschen. Die organische Phase wurde über Kieselgur abgesaugt, über NgSO₄ getrocknet, eingeengt und mittels Säulenchromatographie (Laufmittel: Essigsäureethylester/Cyclohexan 1:1) gereinigt.
Ausbeute: 1.9 g einer Verbindung Z5c (farblose Kristalle)

1.9 g der Verbindung Z5c wurden in 32 mL Dimethylacetamid gelöst und unter Eiskühlung mit 0.3 g (7 mmol) Natriumhydrid als 60%ige Dispersion in Mineralöl versetzt. Nach 10 min wurden 0.5 mL (7 mmol) Methyliodid zugegeben und 3 h bei Raumtemperatur gerührt. Die Reaktionsmischung wurde eingeengt und mit Wasser versetzt. Der entstandene Niederschlag wurde abgesaugt und mit Petrolether gewaschen.
Ausbeute: 1.6 g einer Verbindung Z5d (farblose Kristalle)

14.0 g der Verbindung Z5d und 10.0 g (0.06 mol) 4-Amino-3-methoxybenzoesäure wurden in 200 mL Dioxan und 80 mL Wasser suspendiert, mit 10 mL konz. Salzsäure versetzt und 40 h unter Rückfluss gekocht. Der beim Abkühlen ausgefallene Niederschlag wurde abgesaugt und mit Wasser, Dioxan und Diethylether gewaschen.
Ausbeute: 13.9 g einer Verbindung Z5 (farblose Kristalle)

Zur Synthese der Verbindungen Bsp. 88, 194, 229 und 89 wird zunächst eine Zwischenverbindung Z6 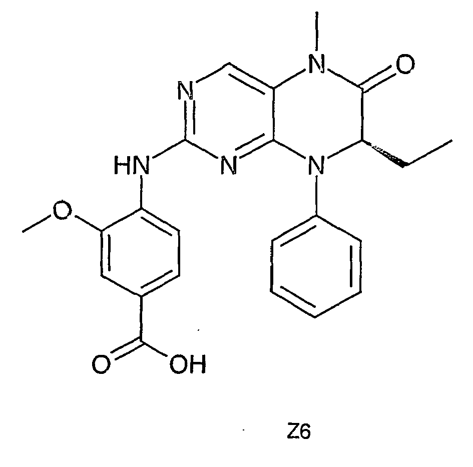 wie im folgenden beschrieben hergestellt.

6.0 g (0.06 mol) L-2-Aminobuttersäure wurde in 80 mL 0.5 M Schwefelsäure vorgelegt und bei 0°C mit 5.5 g (0.08 mol) Natriumnitrit in 15. mL Wasser versetzt. Die Reaktionsmischung wurde 22 h bei 0°C gerührt, mit Ammoniumsulfat versetzt und filtriert. Das Filtrat wurde mit Diethylether extrahiert und die vereinten organischen über MgSO₄ getrocknet und eingeengt.
Ausbeute: 6.0 g einer Verbindung Z6a (gelbes Öl)

200 mL Methanol wurden unter Eiskühlung nacheinander mit 65.0 mL (0.89 mol) Thionylchlorid und 76.0 g der Verbindung Z6a in 50 mL Methanol versetzt. Es wurde 1 h bei 0°C und 2 h bei Raumtemperatur gerührt und dann das Methanol und restliches Thionylchlorid bei 0°C im Vakuum entfernt.
Ausbeute: 40.0 g einer Verbindung Z6b (gelbes Öl)

30.0 mL (0.17 mol) Trifluormethansulfonsäureanhydrid wurden in 150 mL Dichlormethan vorgelegt und unter Eiskühlung innerhalb einer Stunde mit einer Lösung von 20.0 g der Verbindung Z6b und 14.0 mL (0.17 mol) Pyridin in 50 mL Dichlormethan versetzt. Es wurde 2 h bei Raumtemperatur gerührt, entstandene Salze abgesaugt und dann mit 100 mL Wasser gewaschen. Die organische Phase wurde über MgSO₄ getrocknet und eingeengt.
Ausbeute: 42.0 g einer Verbindung Z6c (hellgelbes Öl)

Zu einer Lösung von 15.5 mL (0.17 mol) Anilin und 24.0 mL (0.17 mol) Triethylamin in 400 mL Dichlormethan wurde unter Eiskühlung innerhalb einer Stunde 42.0 g der Verbindung Z6c in 200 mL Dichlormethan getropft. Es wurde 1 h bei Raumtemperatur und weitere 2 h bei 35°C gerührt. Die Reaktionsmischung wurde mit Wasser gewaschen, über MgSO₄ getrocknet und eingeengt. Der verbliebene Rückstand wurde durch Destillation (95-100°C, 1*10⁻³ mbar) gereinigt.
Ausbeute: 14.0 einer Verbindung Z6d (farbloses Öl)

14.0 g der Verbindung Z6d und 16.0 g (0.1 mol) Kaliumcarbonat wurden in 100 mL Aceton suspendiert und bei 10°C mit 16.0 g (0.08 mol) 2,4-Dichlor-5-nitropyrimidin versetzt. Es wurde 4 h bei 40°C gerührt, entstandene Salze abgesaugt und das Filtrat eingeengt. Der Rückstand wurde in 300 mL Essigsäureethylester aufgenommen und mit Wasser gewaschen. Die organische Phase wurde über MgSO₄ getrocknet und eingeengt.
Ausbeute: 31.0 g einer Verbindung Z6e (braunes Öl)

31.0 g der Verbindung Z6e wurden in 200 mL Eisessig gelöst und bei 60°C mit 10 g Eisen-Pulver portionsweise versetzt, wobei die Temperatur auf 85°C anstieg. Es wurde eine weitere Stunde bei 60°C gerührt, über Kieselgur filtriert und eingeengt. Der Rückstand wurde mit Methanol ausgerührt.
Ausbeute: 4.5 g einer Verbindung Z6f (braune Kristalle)

Zu einer Mischung von 4.5 g der Verbindung Z6f und 1.0 mL (16 mmol) Methyliodid in 100 mL Dimethylacetamid wurden bei -20°C 0.6 g (16 mmol) Natriumhydrid als 60%ige Dispersion in Mineralöl portionsweise gegeben. Nach 1 h wurde das Reaktionsgemisch mit 50 mL Wasser versetzt und eingeengt. Der Rückstand wurde mit 200 mL Wasser verrührt, der Niederschlag abgesaugt und mit Petrolether gewaschen.
Ausbeute: 4.5 g einer Verbindung Z6g (farblose Kristalle)

Eine Suspension von 1.5 g der Verbindung Z6g und 1.4 g (8 mmol) 4-Amino-3-methoxybenzoesäuremethylester in 30 mL Toluol wurde mit 0.4 g (0.6 mmol) 2,2'-Bis-(diphenylphosphino)-1,1'-binaphthyl, 0.23 g (0.3 mmol) Tris(dibenzylidenaceton)-dipalladium(0) und 7.0 g (21 mmol) Cäsiumcarbonat versetzt und 17 h unter Rückfluss gekocht. Die Reaktionsmischung wurde auf Kieselgel aufgezogen und mittels Säulenchromatographie (Laufmittel: Dichlormethan/Methanol 9:1) gereinigt.
Ausbeute: 1.7 g einer Verbindung Z6h (gelbe Kristalle)

1.7 g der Verbindung Z6h wurden in 50 mL Dioxan gelöst, mit 15 mL halbkonz. Salzsäure versetzt und 12 h unter Rückfluss gekocht. Nach dem Abkühlen wurde der entstandene Niederschlag abgesaugt.
Ausbeute: 1.1 g einer Verbindung Z6 (farbloser Feststoff)

Zur Synthese der Verbindung Bsp. 26, 20,32, 56, 101, 112, 209 wird zunächst eine Zwischenverbindung Z7 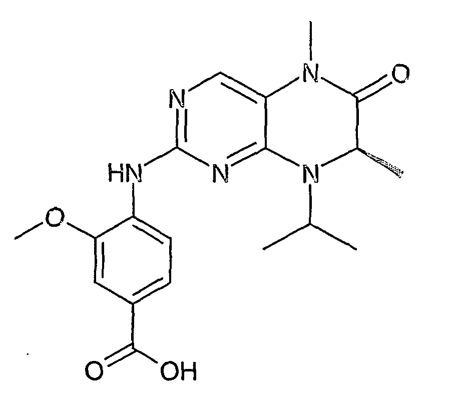 wie im folgenden beschrieben hergestellt.

50.0 g (0.36 mol) D-Alaninmethylester x HCl wurde in 500 mL Dichlormethan und 35 mL Aceton suspendiert und mit 30.0 g (0.37 mol) Natriumacetat und 80.0 g (0.38 mol) Natriumtriacetoxyborhydrid versetzt. Es wurde 12 h gerührt und anschließend auf 400 mL 10%ige Natriumhydrogencarbonat-Lösung gegossen. Die organische Phase wurde über Na₂SO₄ getrocknet und eingeengt.
Ausbeute: 51.0 g einer Verbindung Z7a (gelbes Öl)

Eine Suspension von 51.0 g der Verbindung Z7a in 450 mL Wasser wurde mit 80.0 g (0.41 mol) 2,4-Dichlor-5-nitropyridin in 450 mL Diethylether versetzt. Bei - 5°C wurden 100 mL 10%ige Kaliumhydrogencarbonat-Lösung zugetropft. Das Reaktionsgemisch wurde 3 h gerührt, die organische Phase über Na₂SO₄ getrocknet und eingeengt.
Ausbeute: 74 g einer Verbindung Z7b (gelbes Öl)

18.6 g der Verbindung Z7b wurden in 200 mL Eisessig gelöst und bei 60°C mit 20.0 g Eisen-Pulver portionsweise versetzt. Es wurde 2 h bei 60°C gerührt und dann über Cellulose abgesaugt. Der Rückstand wurde in Essigsäureethylester gelöst und mit Wasser und konz. Ammoniak gewaschen. Die organische Phase wurde über Na₂SO₄ getrocknet und eingeengt. Der Rückstand wurde aus Diethylether kristallisiert.
Ausbeute: 9.8 g einer Verbindung Z7c (farblose Kristalle)

17.0 g der Verbindung Z7c und 7 mL (0.1 mol) Methyliodid wurden in 200 mL Dimethylacetamid gelöst und bei -5°C mit 4.0 g (0.1 mol) Natriumhydrid als 60%ige Dispersion in Mineralöl versetzt. Die Reaktionsmischung wurde 30 min gerührt und dann auf 300 mL Eiswasser gegossen. Der entstandene Niederschlag wurde abgesaugt und mit Petrolether ausgerührt.
Ausbeute: 14.8 g einer Verbindung Z7d (beige Kristalle)

0.9 g der Verbindung Z7d und 1.5 g (9 mmol) 4-Amino-3-methoxybenzoesäure wurden 30 min auf 210°C erhitzt. Nach dem Abkühlen wurde der Rückstand mit Essigsäureethylester ausgerührt und der erhaltene Niederschlag abgesaugt. Ausbeute: 1.2 g einer Verbindung Z7 (graue Kristalle)

Analog zu den beschriebenen Synthesen wurden beispielesweise folgende Säuren hergestellt:

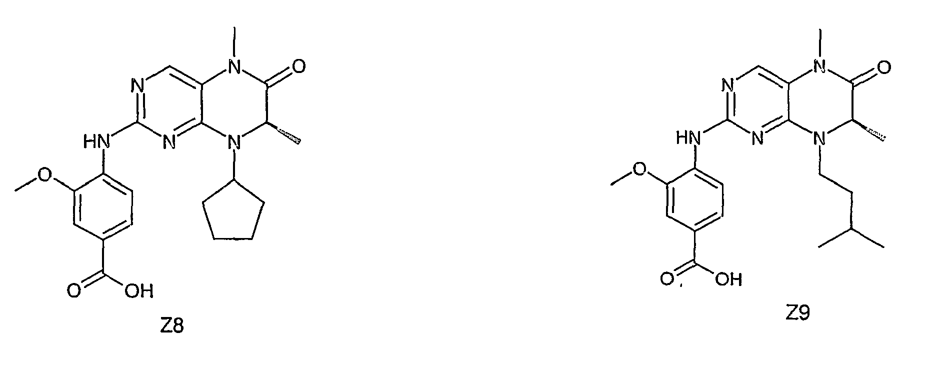

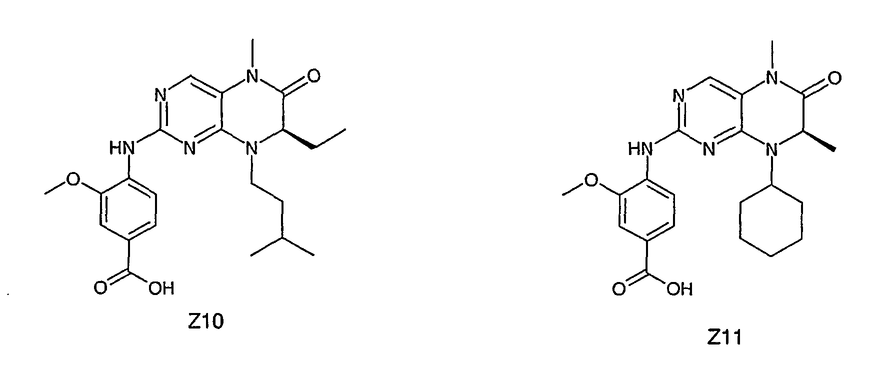

### Synthese der Aminbausteine L-R5

Die folgenden Amine wurden wie folgt erhalten:

### 1,1-Dimethyl-2-dimethylamino-1-yl-ethylamin und 1,1-Dimethyl-2-piperidin-1-yl-ethylamin

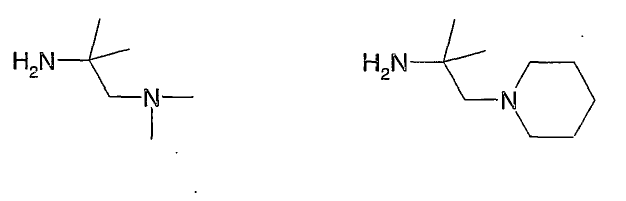

Die Verbindungen wurden hergestellt nach folgenden Literaturstellen: a) S. Schuetz et al. *Arzneimittel-Forschung* **1971,** *21*, 739-763 b) V. M. Belikov et al. *Tetrahedron* **1970,** *26*, 1199-1216. c) E.B. Butler and McMillan *J. Amer. Chem. Soc*. **1950**, *72*, 2978.

Weitere Amine wurden in zu der oben beschriebenen Literatur modifizierter Art und Weise wie folgt hergestellt.

### 1,1-Dimethyl-2-morpholin-1-yl-ethylamin

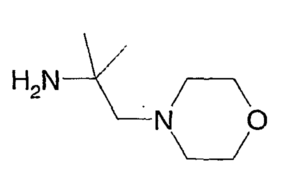

8.7 mL Morpholin und 9.3 mL 2-Nitropropan wurden unter Eiskühlung vorgelegt, 7.5 mL Formaldehyd (37%) und 4 mL einer 0.5 mol/L NaOH-Lsg. wurden langsam zugetropft (<10°C). Danach wurde 1 h bei 25°C und 1 h bei 50°C gerührt. Die Lösung wurde mit Wasser und Ether behandelt und die wäßrige Phase 3x mit Ether extrahiert. Die vereinte org. Phase wurde über NaSO4 getrocknet und mit HCl in Dioxan (4mol/l) versetzt, der entstandene Niederschlag wurde abgesaugt.
Ausbeute: 21,7 weißes Pulver
5 g des weißen Pulvers wurden in 80 mL Methanol gelöst und unter Zusatz von 2 g RaNi mit Wasserstoff bei 35°C und 50 psi für 40 Minuten behandelt. Dies ergab 3.6 g 1,1-Dimethyl-2-morpholin-1-yl-ethylamin.

Analog zu dieser Vorschrift wurden die folgenden Amine hergestellt:

### 1,1-Dimethyl-N-methylpiperazin-1-yl-ethylamin

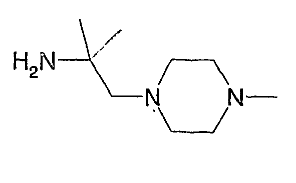

### 1,1-Dimethyl-2-pyrrolidin-1-yl-ethylamin

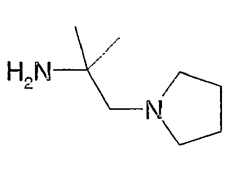

### Synthese von 1,3 Dimorpholin-2-amino-propan

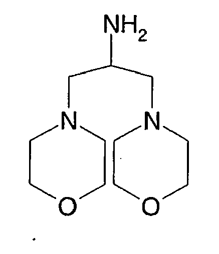

5 g 1,3 Dimorpholin-2-nitropropan der Fa. Aldrich wurde in 80 mL Methanol gelöst und unter Zusatz von 2 g RaNi mit Wasserstoff bei 30°C und 50 psi für 5.5 h behandelt. Dies ergab 4.2 g 1,3 Dimorpholin-2-amino-propan.

### 4-Aminobenzylmorpholin

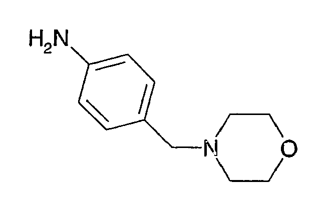

Die Herstellung dieses Amines ist in folgender Literaturstelle beschrieben: S. Mitsuru et al. *J. Med. Chem.* **2000,** *43,* 2049-2063

### 4-Amino-1-tertahydro-4H-pyran-4-yl-piperidin

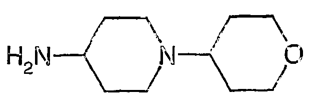

20 g (100 mmol)) 4-tert-Butyloxycarbony-aminopiperidin wurden in 250 mL CH₂Cl₂ gelöst und mit 10 g (100 mmol) Tetrahydro-4H-pyran-4-on und 42 g (200 mmol) NaBH(OAc)₃ 12 h bei RT gerührt. Anschließend wurde mit Wasser und Kaliumcarbonat versetzt, die org. Phase abgetrennt, getrocknet und im Vakuum das Lösungsmittel abgezogen. Der Rückstand wurde in 200 mL CH₂Cl₂ gelöst und mit 100 mL Trifluoressigsäure 12 h bei RT gerührt. Das Lösungsmittel wurde im Vakuum abgezogen, der Rückstand mit CHCl₃ aufgenommen und erneut eingedampft, anschließend in Aceton aufgenommen und mit etherischer HCl das Hydrochlorid gefällt. Ausbeute: 14,3 g (56%).

### cis- und trans-4-Morpholino-cyclohexylamin

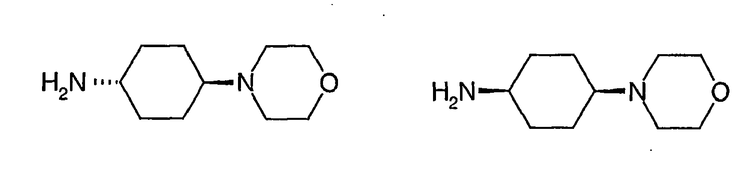

### Dibenzyl-4-morpholino-cyclohenlamin

3,9 g (30 mmol)) 4-Dibenzylcyclohexanon wurden in 100 mL CH₂Cl₂ gelöst und mit 3,9 g (45 mmol) Morpholin und 9,5 g (45 mmol) NaBH(OAc)₃ 12 h bei RT gerührt. Anschließend wurde mit Wasser und Kaliumcarbonat versetzt, die org. Phase abgetrennt, getrocknet und im Vakuum das Lösungsmittel abgezogen. Der Rückstand wurde über eine Kieselgelsäule gereinigt (ca 20 mL Kieselgel; ca 500 mL Essigester 90/ Methanol 10 + 1 % konz. Ammoniak). Die geeigneten Fraktionen wurden im Vakuum eingeengt. Ausbeute 6,6 g (60%) cis-lsomer und 2 g (18%) trans-lsomer.

Alternativ kann das trans-Dibenzyl-4-morpholino-cyclohexylamin nach folgendem Weg dargestellt werden:

33 g (112 mmol) 4-Dibenzylcyclohexanon wurden in 300 mL MeOH gelöst, mit 17,4 g (250 mmol) Hydroxylaminhydrochlorid versetzt und 4. h bei 60°C gerührt. Das Lösungsmittel wurde im Vakuum eingeengt, mit 500 mL Wasser und 50 g Kaliumcarbonat versetzt und zweimal mit je 300 mL Dichlormethan extrahiert. Die org. Phase wurde getrocknet, im Vakuum eingeengt, der Rückstand aus Petrolether kristallisiert, in 1,5 L EtOH gelöst und auf 70°C erwärmt. Es wurden 166 g Natrium portionsweise hinzugefügt und bis zur Auflösung des Natriums unter Rückfluß gekocht. Das Lösungsmittel wird im Vakuum abgezogen, der Rückstand mit 100 mL Wasser versetzt und zweimal mit je 400 mL Ether extrahiert. Die org. Phase wird mit Wasser gewaschen, getrocknet, im Vakuum eingeengt und über eine Säule das trans-Isomere isoliert (ca .1,5 L Kieselgel; ca. 2 L Essigester 80/ Methanol 20 + 2 % konz. Ammoniak). Ausbeute: 12,6 g (41,2%).
6,8 g (23 mmol) trans-lAmino-4-Dibenzylaminocyclohexan wurde in 90 mL DMF gelöst und mit 5 mL (42 mmol) 2,2'-Dichlorethylether und 5 g Kaliumcarbonat 8 h bei 100°C gerührt. Nach Abkühlung wurde mit mit 30 mL Wasser versetzt, ausgefallene Kristalle abgesaugt und über eine kurze Säule (ca. 20 mL Kieselgel, ca. 100 mL Essigester) gereinigt. Der Rückstand wird aus Methanol und konz. HCl als Dihydrochlorid kristallisiert. Ausbeute: 7,3 g (72,4%).

### trans-4-Morpholino-cyclohexylamin

7,2 g (16,4 mmol) trans-Dibenzyl-4-morpholino-cyclohexylamin wurden in 100 mL MeOH gelöst und an 1,4 g Pd/C (10%) bei 30-50 °C hydriert. Das Lösungsmittel wurde im Vakuum abgezogen und der Rückstand aus Ethanol und konz. HCl kristallisiert. Ausbeute: 3,9 g (93%); Schmp. 312 °C.

Das cis-lsomer kann analog dargestellt werden.

### cis- und trans-4-Piperidino-cyclohexylamin

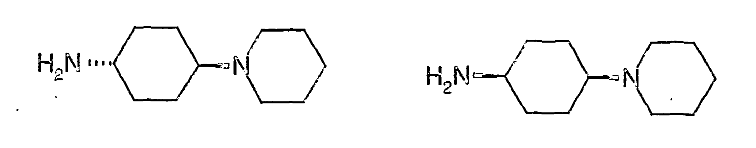

### trans-Dibenzyl-4-piperidino-cyclohexylamin

2,0 g (6,8 mmol) trans-1Amino-4-Dibenzylaminocyclohexan (s. Bsp. 2) wurde in 50 mL DMF gelöst und mit 1,6 g (7 mmol) 1,5-Dibrompentan und 2 g Kaliumcarbonat 48 h bei RT gerührt. Es wurde abgekühlt, mit Wasser versetzt, zweimal mit je 100 mL Dichlormethan extrahiert, getrocknet und das Lösungsmittel im Vakuum abgezogen. Der Rückstand wird über eine Säule gereinigt (ca. 100 mL Kieselgel, ca. 500 mL Essigester 80/Methanol 20 +1 % konz. Ammoniak). Die geeignete Fraktionen wurden im Vakuum eingeengt und aus Petrolether kristallisiert. Ausbeute: 1,2 g (49%).

### trans-4-Piperidino-cyclohexylamin

1,7 g (4,8 mmol) trans-Dibenzyl-4-piperidino-cyclohexylamin wurden in 35 mL MeOH gelöst und an 350 mg Pd/C (10%) bei 20 °C hydriert. Das Lösungsmittel wurde im Vakuum abgezogen und der Rückstand aus Ethanol und konz. HCl kristallisiert. Ausbeute: 1,1 g (78%).

Das cis-Isomer kann analog dargestellt werden.

### cis- und trans-4-(4-Phenyl-piperazin-1-yl)-cyclohexylamine

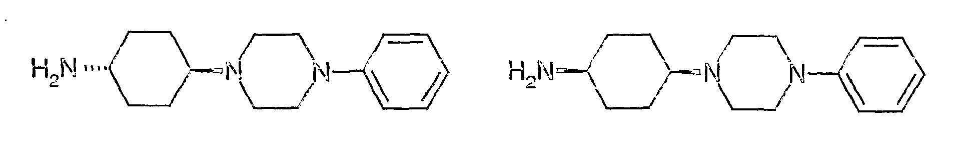

4,1 g (25,3 mmol) 4-Dibenzylcyclohexanon wurde in 50 mL Dichlormethan gelöst und mit 7,4 g (25,3 mmol) N-Phenylpyperazin und 7,4 g (35 mmol) NaBH(OAc)₃ 12 h bei RT gerührt. Anschließend wurde mit Wasser und Kaliumcarbonat versetzt, die org. Phase abgetrennt, getrocknet und das Lösungsmittel im Vakuum abgezogen. Der Rückstand wurde über Kieselgelsäule gereinigt (Essigester 80/Methanol 20 + 0,5% konz. Ammoniak). Ausbeute: 1,7 g (15,8%) cis-lsomer und 0,27 (2,5%) trans-Isomer.

### trans-4-(4-Phenyl-piperazin-1-yl)-cyclohexylamine

270 mg (0,61 mmol) trans-Dibenzyl-[4-(4-phenyl-piperazin-1-yl)-cyclohexyl]-amine wurden in 5 mL MeOH gelöst und an 40 mg Pd/C (10%) bei 20-30 °C hydriert. Das Lösungsmittel wurde im Vakuum abgezogen und der Rückstand aus Ethanol und konz. HCl kristallisiert. Ausbeute: 110 mg (69%).

Das cis-Isomer kann analog dargestellt werden.

### cis- und trans-4-(4-Cyclopropylmethyl-piperazin-1-yl)-cyclohexylamine

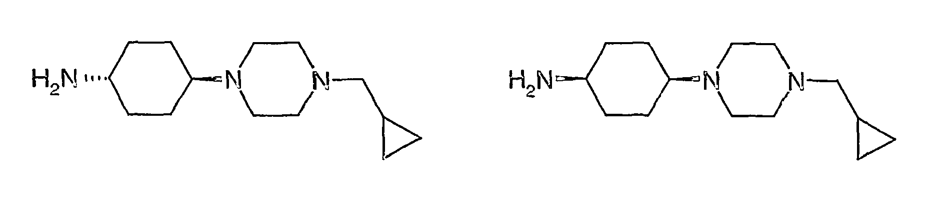

9,8 g (33,4 mmol) 4-Dibenzylcyclohexanon wurde in 100 mL Dichlormethan gelöst und mit 5,6 g (40 mmol) N-Cyclopropylmethylpyperazin und 8,5 g (40 mmol) NaBH(OAc)₃ 12 h bei RT gerührt. Anschließend wurde mit Wasser und Kaliumcarbonat versetzt, die org. Phase abgetrennt, getrocknet und das Lösungsmittel im Vakuum abgezogen. Der Rückstand wurde über Kieselgelsäule gereinigt (ca. 50 mL Kieselgel, ca. 3 L Essigester 95/ Methanol 5 + 0,25% konz. Ammoniak. Die geeigneten Fraktionen wurden im Vakuum eingeengt. Die schneller eluierende cis-Verbindung kristallisiert aus Essigester. Die trans-Verbindung wurde aus Ethanol + konz. HCl kristallisiert. Ausbeute: 8,5 g (61 %) cis-Isomer und 2,2 (13%) trans-Isomer.

### cis-4-(4-Cyclopropylmethyl-piperazin-1-yl)-cyclohexylamine

8,5 g (20 mmol) cis-Dibenzyl-[4-(4-cyclopropylmethyl-piperazin-1-yl)-cyclohexyl]-amine wurden in 170 mL MeOH gelöst und an 1,7 g Pd/C (10%) bei 30-50 °C hydriert. Das Lösungsmittel wurde im Vakuum abgezogen und der Rückstand aus Ethanol und konz. HCl kristallisiert. Ausbeute: 4,4 g (91%).

Das trans-Isomer kann analog dargestellt werden.

### Synthese der Beispiele

### Beispiel 152

0.15g der Verbindung Z10, 0.14 g TBTU, 0.13 mL DIPEA wurden in Dichlormethan gelöst und 20 Minuten bei 25°C gerührt. Dann wurde 90 µL 1-(3-Aminopropyl)-4-Methylpiperazin zugegeben und für weitere 2 Stunden Bei 25°C gerührt. Die Lösung wurde dann mit Dichlormethan verdünnt und mit Wasser extrahiert. Durch Zugabe von Petrolether, Ether und Etyhlacetat zur organischen Phase wurde das Produkt gefällt. Ausbeute: 0.16 g beiger Feststoff

### Beispiel 164

0.10g der Verbindung Z10, 0.1 g TBTU, 0.08 mL DIPEA wurden in 4 mL Dichlormethan gelöst und 20 Minuten bei 25°C gerührt. Dann wurden 44 µL Dimethylaminopropylamin zugegeben und für weitere 2 Stunden Bei 25°C gerührt. Die Lösung wurde dann mit Dichlormethan verdünnt und mit Wasser extrahiert. Durch Zugabe von Petrolether, Ether und Aceton zur organischen Phase wurde das Produkt gefällt. Ausbeute: 0.08 g gelber Feststoff.

### Beispiel 242

0.15g der Verbindung Z10, 0.14 g TBTU, 0.13 mL DIPEA wurden in 5 mL Dichlormethan gelöst und 20 Minuten bei 25°C gerührt. Dann wurden 75 *µ*L 1-(2-Aminoethyl) piperidin zugegeben und für weitere 2 Stunden Bei 25°C gerührt. Die Lösung wurde dann mit Dichlormethan verdünnt und mit Wasser extrahiert. Durch Zugabe von Petrolether, Ether und Ethylacetat zur organischen Phase wurde das Produkt gefällt. Ausbeute: 0.14 g gelber Feststoff.

### Beispiel 188

0.1 g der Verbindung Z2, 0.09 g TBTU, 0.05 mL DIPEA wurden in 15 mL Dichlormethan gelöst und 20 Minuten bei 25°C gerührt. Dann wurden 33 mg 1-Methyl-4-aminopiperidin zugegeben und für weitere 3 Stunden Bei 25°C gerührt. Die Lösung wurde mit 20 mL Wasser extrahiert, dann im Vakkum eingeengt. Mit Hilfe von Ether wurde das Produkt kristallisiert. Ausbeute: 0.04.7 g weiße Kristalle.

### Beispiel 203

0.1 g der Verbindung Z2, 0.09 g TBTU, 0.5 mL DIPEA wurden in 15 mL Dichlormethan gelöst und 30 Minuten bei 25°C gerührt. Dann wurden 50 mg 4-Amino-1-benzylpiperidin zugegeben und für weitere 3 Stunden Bei 25°C gerührt. Die Lösung wurde mit 20 mL Wasser extrahiert, dann im Vakkum eingeengt. Anschließend wurde über Kieselgel chromatographiert und das isolierte Produkt mit Hilfe von Ether kristallisiert. Ausbeute: 0.015 g weiße Kristalle.

### Beispiel 94

0.17 g der Verbindung Z1, 0.19 g TBTU, 0.11 mL DIPEA wurden in 50 mL Dichlormethan gelöst und 30 Minuten bei 25°C gerührt. Dann wurden 63 mg 1-Methyl-4-aminopiperidin zugegeben und für weitere 17 Stunden Bei 25°C gerührt. Zur Lösung wurden 50 mL Wasser und 1 g Kaliumcarbonat gegeben und die organische Phase mittels Phasentrennkartusche abgetrennt, dann im Vakkum eingeengt. Anschließend wurde das Produkt durch Kieselgelchromatograhie gereinigt und das gereinigte Produkt mit Hilfe von Ether kristallisiert. Ausbeute: 0.1 g weiße Kristalle.

### Beispiel 95

0.17 g der Verbindung Z1, 0.19 g TBTU, 0.11 mL DIPEA wurden in 50 mL Dichlormethan gelöst und 30 Minuten bei 25°C gerührt. Dann wurden 77 mg exo - 3-β-Amino-Tropan zugegeben und für weitere 17 Stunden Bei 25°C gerührt. Zur Lösung wurden 50 mL Wasser und 1 g Kaliumcarbonat gegeben und die organische Phase mittels Phasentrennkartusche abgetrennt, dann im Vakkum eingeengt. Anschließend wurde das Produkt durch Kieselgelchromatograhie gereinigt und das gereinigte Produkt mit Hilfe von Ether kristallisiert. Ausbeute: 0.03 g weiße Kristalle.

### Beispiel 46

0.15 g der Verbindung Z3, 0.12 g TBTU, 0.12 mL DIPEA wurden in 5 mL Dichlormethan gelöst und 30 Minuten bei 25°C gerührt. Dann wurden 50 mg 1-Methyl-4-aminopiperidin zugegeben und für weitere 2.5 Stunden Bei 25°C gerührt. Die Lösung wurde dann mit Wasser extrahiert und anschließend eingeengt. Der Rückstand wurde in warmen Ethylacetat gelöst und durch Ether und Petrolether kristallisiert. Ausbeute: 0.025 g weiße Kristalle. Schmp.: 203°C als Base

### Beispiel 80

0.2 g der Verbindung Z8, 0.2 g TBTU, 0.1 mL DIPEA wurden in 10 mL Dichlormethan gelöst und 30 Minuten bei 25°C gerührt. Dann wurden 100 mg 1-Methyl-4-aminapiperidin zugegeben und für weitere 17 Stunden bei 25°C gerührt. Die Lösung wurde dann mit einer verdünnten Kaliumcarbonatlösung extrahiert und eingeengt. Der Rückstand wurde mit Hilfe von Ether kristallisiert. Ausbeute: 0.12 g weiße Kristalle

### Beispiel 190

0.2 g Verbindung Z8, 0.2 g TBTU, 0.3 mL DIPEA wurden in 5 mL Dichlormethan gelöst und 1h bei 25°C gerührt. Dann wurden 0.13 g 4-Amino-1-benzylpiperidin zugegeben und für eine weiter Stunde bei 25°C gerührt. Die Lösung wurde dann mit 10 mL Methylenchlorid verdünnt und mit 20 mL Wasser extrahiert. Anschließend wurde das Produkt über Kieselgel gereinigt und mittels Ethylacetat und Ether kristallisiert. Ausbeute: 0.23 g der Verbindung Z8
0.23 g des Benzylamines Z8 werden in 10 mL Methanol gelöst, mit 50 mg Pd/C versetzt und 3h bei 3 bar bei 25°C hydriert. Duch Zugabe von Petrolether und Ethylacetat werden weiße Kristalle erhalten. Diese werden über Kieselgel chromatographiert und mit Hilfe von Ethylacetat und Ether kristallisiert. Ausbeute: 0.075 g weiße Kristalle.

### Beispiel 196

0.1g Verbindung Z10, 0.09 g TBTU, 0.3 mL DIPEA wurden in 4 mLDichlormethan gelöst und 20 Minuten bei 25°C gerührt. Dann wurde 67 mg xx Amin zugegeben und für weitere 2 Stunden Bei 25°C gerührt. Die Lösung wurde dann mit Dichlormethan verdünnt und mit Wasser extrahiert. Anschließend wurde über Kieselgel chromtografiert und der Rückstand in Aceton gelöst, mit etherischer HCl versetzt und der entstandene Niederschlag isoliert. Ausbeute: 0.09 g hellgelber Feststoff

### Beispiel 166

0.1 g der Verbindung Z10, 0.11 g TBTU, 0.14 mL DIPEA wurden in 2 mL Dimethylformamid gelöst und 3h bei 50°C gerührt. Dann wurde 55 mg 4-Morpholinomethylphenylamin zugefügt. Anschließend wurde die Reaktion innerhalb von 17 h auf Raumtemperatur abgekühlt. Dann wurde das Dimethylformamid im Vakkum entfernt, der Rückstand in Dichlormethan aufgenommen und mit Wasser extrahiert. Anschließend wurde über Kieselgel chromatographiert und das Produkt aus Ethylacetat und Ether kristallisiert. Ausbeute: 0.06 g gelbliche Kristalle

### Beispiel 81

0.2 g der Verbindung Z4, 0.2 g TBTU, 0.1 mL DIPEA wurden in 10 mL Dichlormethan gelöst und 30 Minuten bei 25°C gerührt. Dann wurden 0.1 g 1-Methyl-4-aminopiperidin zugegeben und für weitere 17 Stunden bei 25°C gerührt. Die Lösung wurde dann mit wässriger Kaliumcarbonatlösung extrahiert und anschließend eingeengt. Der Produkt wurde mit Hilfe von Ether kristallisiert. Ausbeute: 0.16 g weiße Kristalle.

### Beispiel 162

0.1 g der Verbindung Z5, 0.07 g TBTU, 0.15 mL DIPEA wurden in 5 mL Dichlormethan gelöst und 20 Minuten bei 25°C gerührt. Dann wurden 0.04 g 1-Methyl-4-aminopiperidin zugegeben und für weitere 2 Stunden bei 25°C gerührt. Die Lösung wurde dann mit 15 mL Dichlormethan verdünnt und mit 20 mL Wasser extrahiert. Der Rückstand wurde in MeOH und Aceton gelöst, mit 1 mL etherische HCI versetzt und eingeengt. Mit Hilfe von Ether, Ethylacetat und wenig MeOH wurde ein kristallines Produkt erhalten. Ausbeute: 0.1 g weiße Kristalle.

### Beispiel 88

0.1 g der Verbindung Z6, 0.12 g TBTU, 0.12 mL DIPEA wurden in 10 mL Dichlormethan gelöst und 30 Minuten bei 25°C gerührt. Dann wurden 0.04 g 1-Methyl-4-aminopiperidin zugegeben und für weitere 2 Stunden bei 25°C gerührt. Die Lösung wurde dann mit 10 mL Dichlormethan verdünnt und mit 10 mL Wasser extrahiert. Mit Hilfe von Ethylacetat, Ether und Petrtolether wurde ein kristallines Produkt erhalten. Ausbeute: 0.6 g weiße Kristalle.

### Beispiel 89

0.1 g der Verbindung Z6, 0.08 g TBTU, 0.08 mL DIPEA wurden in 10 mL Dichlormethan gelöst und 30 Minuten bei 25°C gerührt. Dann wurden 37 µL g N,N-Dimethylneopentandiamin zugegeben und für weitere 2 Stunden bei 25°C gerührt. Die Lösung wurde dann mit 10 mL Dichlormethan verdünnt und mit 10 mL Wasser extrahiert. Das Produkt wurde dann über Kieselgel chromatografiert und mit Hilfe von Ethylacetat, Ether und Petrtoletherkristallisiert. Ausbeute: 0.005 g weiße Kristalle.

### Beispiel 26

0.15 g der Verbindung Z7, 0.16 g TBTU, 1 mL DIPEA wurden in 5 mL Dichlormethan gelöst und 30 Minuten bei 25°C gerührt. Dann wurden 0.1 g 4-Morpholinocyclohexylamin zugegeben und für weitere 17 Stunden bei 25°C gerührt. Der Rückstand wurde dann mit 10 mL 10%iger Kaliumcarbonatlösung versetzt, der Niederschlag isoliert und mit Wasser gewaschen. Dann wurde in Dichlormethan gelöst und erneut eingeengt. Mit Hilfe von Ethylacetat, wurde das Produkt kristallisiert. Ausbeute: 0.1 g weiße Kristalle.

### Beispiel 9

150 mg der Verbindung Z9 und 93 mg Amin wurden in 5 mL Dichlormethan gelöst und mit 160 mg TBTU und 1 mL DlPEA 12 h bei RT gerührt. Das Lösungsmittel wurde im Vakuum abgezogen, der Rückstand mit 10 mL 10%ige Kaliumcarbonatlösung versetzt. Der Niederschlag wurde abgesaugt, mit Wasser gewaschen, in Dichlormethan aufgenommen, getrocknet und das Lösungsmittel im Vakuum abgezogen. Der Rückstand wurde aus Essigester kristallisiert. Ausbeute: 82,0 mg; Schmp. 253 °C (als Base).

### Beispiel 16

150 mg der Verbindung Z8 und 73 mg trans-4-Piperidino-cyclohexylamin wurden in 5 mL Dichlormethan gelöst und mit 160 mg (0,50 mmol) TBTU und 1 mL DIPEA 12 h bei RT gerührt. Das Lösungsmittel wurde im Vakuum abgezogen, der Rückstand mit 10 mL 10%ige Kaliumcarbonatlösung versetzt. Der Niederschlag wurde abgesaugt, mit Wasser gewaschen, in Dichlormethan aufgenommen, getrocknet und das Lösungsmittel im Vakuum abgezogen. Der Rückstand wurde aus Essigester kristallisiert. Ausbeute: 87,0 mg; Schmp. 237 °C (als Base).

### Beispiel 37

100 mg der Verbindung Z9 und 42 mg 3-Amino-1-ethyl-pyrolidine wurden in 10. mL Dichlormethan gelöst und mit 90 mg TBTU und 0,5 mL DIPEA 12 h bei RT gerührt. Das Lösungsmittel wurde im Vakuum abgezogen, der Rückstand mit 10 mL 10%ige Kaliumcarbonatlösung versetzt. Der Niederschlag wurde abgesaugt, mit Wasser gewaschen, in Dichlormethan aufgenommen, getrocknet und das Lösungsmittel im Vakuum abgezogen. Der Rückstand wurde aus Essigester/Petrolether kristallisiert. Ausbeute: 24,0 mg .

### Beispiel 120

100 mg der Verbindung Z11 und 73 mg 4-Amino-1tetrahydro-4H-pyran-4-yl-piperidin wurden in 10 mL Dichlormethan gelöst und mit 90 mg TBTU und 0,5 mL DIPEA 1 h bei RT gerührt. Das Lösungsmittel wurde im Vakuum abgezogen, der Rückstand mit 10 mL 10%ige Kaliumcarbonatlösung versetzt. Der Niederschlag wurde abgesaugt, mit Wasser gewaschen, in Dichlormethan aufgenommen, getrocknet und das Lösungsmittel im Vakuum abgezogen. Der Rückstand wurde aus Essigester/Petrolether kristallisiert. Ausbeute: 89 mg.

### Beispiel 212

150 mg der Verbindung Z5 und 150 mg trans-4-(4-Cyclopropylmethyl-piperazin-1-yl)-cyclohexylamin (als Hydrochlorid) wurden in 5 mL Dichlormethan gelöst und mit 160 mg TBTU und 2 mL DIPEA 2 h bei RT gerührt. Das Lösungsmittel wurde im Vakuum abgezogen, der Rückstand mit 10 mL 10%ige Kaliumcarbonatlösung versetzt. Der Niederschlag wurde abgesaugt, mit Wasser gewaschen, in Dichlormethan aufgenommen, getrocknet und das Lösungsmittel im Vakuum abgezogen. Der Rückstand wurde über eine Säule gereinigt (20 mL Kieselgel, 300 mL Essigester 90/ Methanol 10 + 2% konz. Ammoniak). Die geeigneten Fraktionen wurden i.Vak eingeengt und aus Essigester kristallisiert. Ausbeute: 140 mg; Schmp. 187 °C (als Base).

### Beispiel 232

390 mg der Verbindung Z11 und 240 mg trans-4-(4-tButyloxycarbonyl-piperazin-1-yl)-cyclohexylamin wurden in 2,5 mL NMP gelöst und mit 482 mg TBTU und 1 mL Triethylamin 2 h bei RT gerührt. Anschließend wurde mit 100 mL Wasser und 200 mg Kaliumcarbonat versetzt, der Niederschlag wurde abgesaugt, mit Wasser gewaschen und über eine Kieselgelsäule gereinigt. Die geeignete Fraktionen wurden im Vakuum eingeengt, in 2 mL Dichlormethan gelöst, mit 2 mL Trifluoessigsäure versetzt und 2 h bei RT gerührt, erneut mit 100ml Wasser und 200 mg Kaliumcarbonat versetzt und der Niederschlag abgesaugt und mit Wasser gewaschen. Anschließend wurde der Niederschlag über eine Kieselgelsäule gereinigt. Die geeignete Fraktionen wurden im Vakuum eingeengt und der Rückstand aus Ethanol und konz. Salzsäure kristallisiert. Ausbeute: 95 mg; Schmp. 291°C.

### Beispiel 213

60 mg der Verbindung Beispiel 232 wurde in 10 mL Essigester gelöst und mit 1 mL Essigsäureanhydrid und 1 mL Triethylamin 30 Min. bei RT gerührt. Das Lösungsmittel wurde im Vakuum abgezogen, der Rückstand mit Wasser und Ammoniak versetzt, die ausgefallene Kristalle abgesaugt und mit Wasser und wenig kaltem Aceton gewaschen. Ausbeute: 40 mg; Schmp.248 °C.

### Beispiel 218

1,2 g der Verbindung Z9 und 0,5g 1,4-Dioxaspiro[4.5]dec-8-ylamin wurden in 20 mL Dichlormethan gelöst und mit 1,28 g TBTU und 4 mL Triethylamin 12 h bei RT gerührt. Anschließend wurde mit 50 mL Wasser und 0,5 g Kaliumcarbonat versetzt, die org. Phase abgetrennt, getrocknet und im Vakuum eingeengt. Der Rückstand wurde aus Essigester kristallisiert, mit 25 mL 1 N Salzsäure und 20 mL Methanol versetzt und 30 Min bei 50°C gerührt. Das Methanol wurde im Vakuum abgezogen, der Niederschlag abgesaugt, mit Wasser gewaschen und getrocknet.
Der Rückstand wurde in 20 mL Dichlormethan aufgenommen, mit 0,5 g Thiomorpholin und 0,5 g NaBH(OAc)₃ 12 h bei RT gerührt. Anschließend wurde mit Wasser und Kaliumcarbonat versetzt, die org. Phase abgetrennt, getrocknet und das Lösungsmittel im Vakuum abgezogen. Der Rückstand wurde über Kieselgelsäule. Die geeigneten Fraktionen wurden im Vakuum eingeengt und mit etherischer HCl das Hydrochlorid gefällt. Ausbeute: 86 mg trans-Isomer; amorphes Pulver.

### Beispiel 187

200 mg der Verbindung Z3 in 5 mL Dichlormethan wurde mit 0.1 mL Diisopropylethylamin und 180 mg TBTU versetzt und 30 min gerührt. Anschließend wurden 191 mg 4-(4-Methyl-piperazin-1-yl)-phenylamin zugegeben und über Nacht gerührt. Die Reaktionsmischung wurde mit Wasser versetzt und die wässrige Phase mit Dichlormethan extrahiert. Die vereinten organischen
Z9

Phasen wurden über Na₂SO₄ getrocknet und eingeengt. Der Rückstand wurde mittels Säulenchromatographie (Laufmittel: Dichlormethan/Methanol 100:7) gereinigt.
Ausbeute: 128 mg (leicht gelbe Kristalle)

Analog zu vorstehend beschriebener Vorgehensweise werden u.a. die in Tabelle 1 aufgeführten Verbindungen der Formel (I) erhalten.
Die in Tabelle 1 verwendeten Abkürzungen X₁, X₂, X₃, X₄ und X₅ stehen jeweils für eine Verknüpfung mit einer Position in der unter Tabelle 1 aufgeführten allgemeinen Formel anstelle der entsprechenden Reste R¹, R², R³, R⁴ und L-R⁵.

**Tabelle 1**

| | | | | | | | | | |
|---|---|---|---|---|---|---|---|---|---|
| 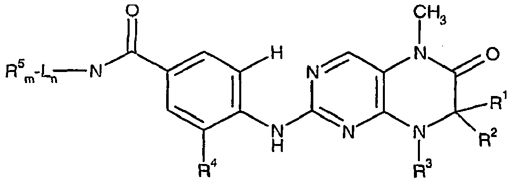 | | | | | | | | | |
| | | | | | | | | | |

| Bsp. | HELA | H460VINC | R¹ | R² | Konfig. R¹ od. R² | R³ | R⁴ | Lₙ-R⁵ₘ | Smp. |
|---|---|---|---|---|---|---|---|---|---|
| 1 | 0,046 | 0,604 | H | 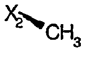 | R | 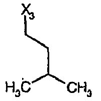 | 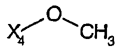 | 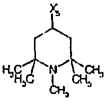 | |
| 2 | 0,056 | 0,971 | H | 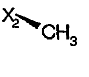 | R | 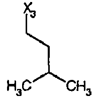 | 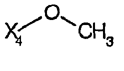 | 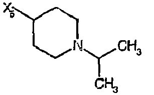 | |
| 3 | 0,061 | | H | 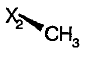 | R | 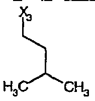 | H | 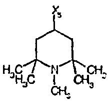 | |
| 4 | 0,098 | | H | 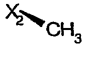 | R | 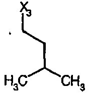 | H | 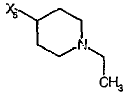 | |
| 5 | 0,052 | 0,863 | H | 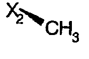 | R | 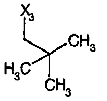 | 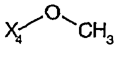 | 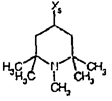 | |
| 6 | 0,053 | 0,822 | H | 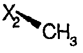 | R | 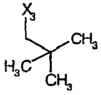 | 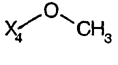 | 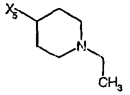 | |
| 7 | 0,084 | 0,865 | H | 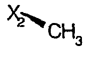 | R | 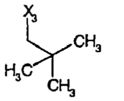 | 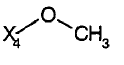 | 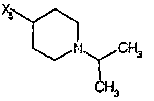 | |
| 8 | 0,063 | | H | 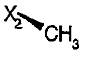 | R | 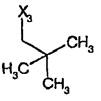 | H | 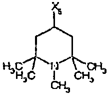 | |
| 9 | 0,057 | 0,207 | H | 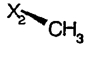 | R | 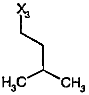 | 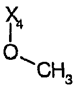 | 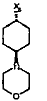 | 253 |
| 10 | 0,091 | | H | 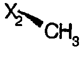 | R | 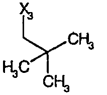 | H | 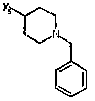 | |
| 11 | 0,086 | 1,300 | H | 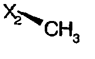 | R | 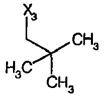 | H | 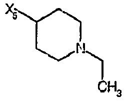 | |
| 12 | 0,099 | 1,069 | H | 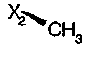 | R | 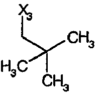 | H | 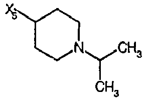 | |
| 13 | 0,026 | 1,320 | H | 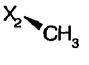 | R | 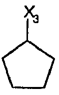 | 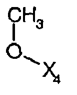 | 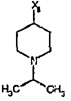 | |
| 14 | 0,055 | 3,000 | H | 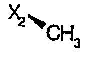 | R | 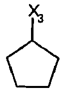 | H | 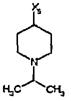 | |
| 15 | 0,020 | 1,000 | H | 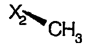 | R | 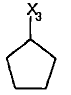 | 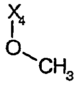 | 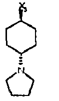 | 231 |
| 16 | 0,017 | 0,483 | H | 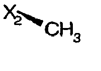 | R | 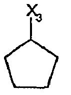 | 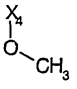 | 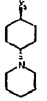 | 237 |
| 17 | 0,024 | 0,730 | H | 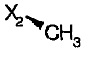 | R | 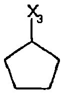 | 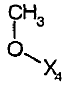 | 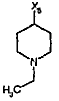 | |
| 18 | 0,040 | 1,764 | H | 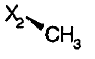 | R | 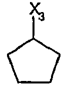 | H | 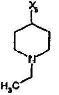 | |
| 19 | 0,013 | 0,747 | H | 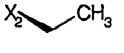 | R | 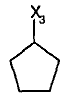 | 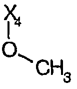 | 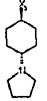 | 254 |
| 20 | 0,083 | 3,000 | H | 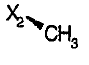 | R | 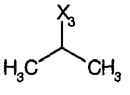 | 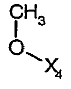 | 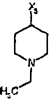 | |
| 21 | 0,009 | 0,488 | H | 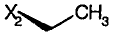 | R | 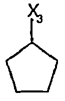 | 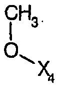 | 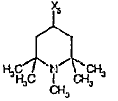 | |
| 22 | 0,008 | 0,347 | H | 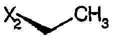 | R | 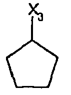 | 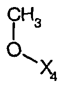 | 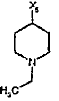 | |
| 23 | 0,013 | 0,679 | H | 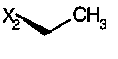 | R | 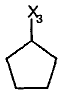 | 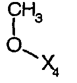 | 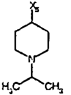 | |
| 24 | 0,017 | 0,277 | H | 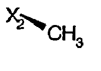 | R | 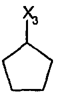 | 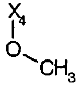 | 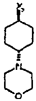 | 152 |
| 25 | 0,038 | 1,000 | H | 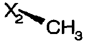 | R | 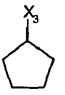 | 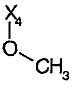 | 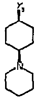 | 254 |
| 26 | 0,059 | 0,424 | H | 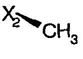 | R | 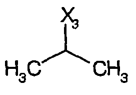 | 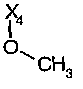 | 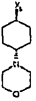 | |
| 27 | 0,014 | 0,164 | H | 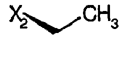 | R | 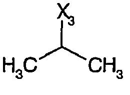 | 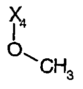 | 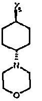 | 292 |
| 28 | 0,018 | 0,160 | H | 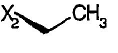 | R | 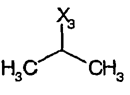 | 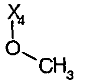 | 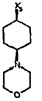 | 160 |
| 29 | 0,049 | 1,000 | H | 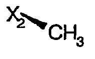 | R | 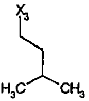 | 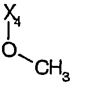 | 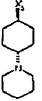 | 251 |
| 30 | 0,012 | 0,574 | H | 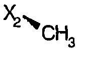 | R | 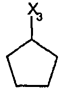 | 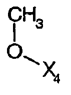 | 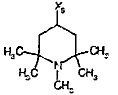 | |
| 31 | 0,023 | 3,000 | H | 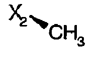 | R | 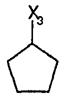 | H | 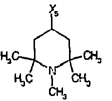 | |
| 32 | 0,049 | 3,000 | H | 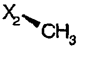 | R | 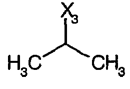 | 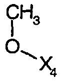 | 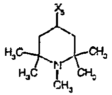 | |
| 33 | 0,097 | 3,000 | H | 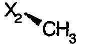 | R | 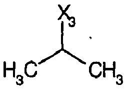 | H | 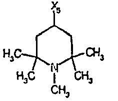 | |
| 34 | 0,055 | 1,000 | H | 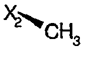 | R | 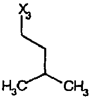 | 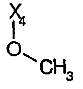 | 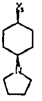 | 242 |
| 35 | 0,088 | 1,000 | H | 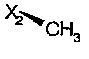 | R | 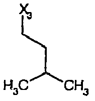 | 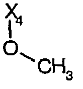 | 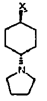 | 213 |
| 36 | 0,021 | 1,000 | H | 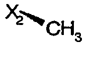 | R | 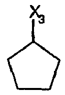 | 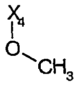 | 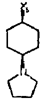 | 232 |
| 37 | 0,072 | 0,762 | H | 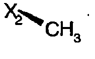 | R | 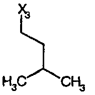 | 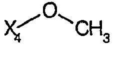 | 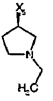 | 160 |
| 38 | 0,087 | 0,278 | H | 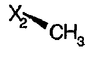 | R | 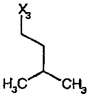 | 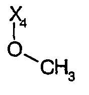 | 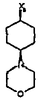 | 225 |
| 39 | 0,075 | 1,000 | H | 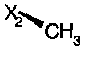 | R | 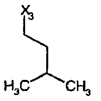 | H | 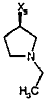 | |
| 40 | 0,038 | 0,187 | H | 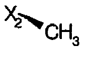 | R | 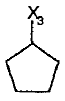 | 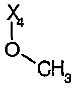 | 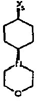 | 226 |
| 41 | 0,083 | 3,000 | H | 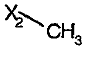 | R | 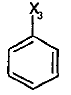 | 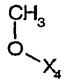 | 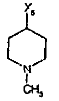 | |
| 42 | 0,076 | 2,310 | H | 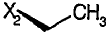 | R | 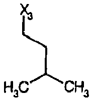 | 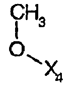 | 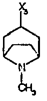 | |
| 43 | 0,094 | 3,000 | X₁-CH₃ | 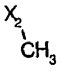 | | 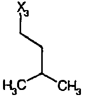 | 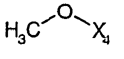 | 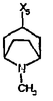 | 218 |
| 44 | 0,009 | 0,204 | H | 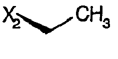 | R | 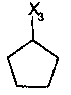 | H | 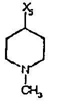 | |
| 45 | 0,022 | 0,792 | H | 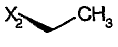 | R | 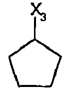 | 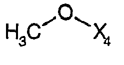 | 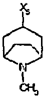 | |
| 46 | 0,0_{'}12 | 0,208 | H | 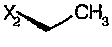 | R | 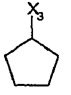 | 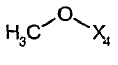 | 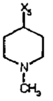 | 203 |
| 47 | 0,028 | | H | 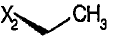 | R | 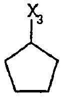 | H | 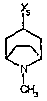 | |
| 48 | 0,083 | 3,000 | H | 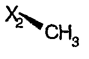 | R | 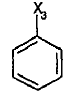 | H | 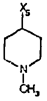 | |
| 49 | 0,059 | 0,529 | H | 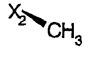 | R | 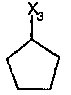 | 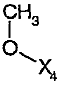 | 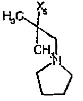 | |
| 50 | 0,081 | 0,436 | H | 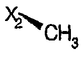 | R | 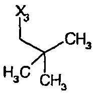 | 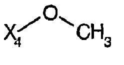 | 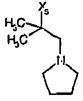 | |
| 51 | 0,080 | 0,150 | H | 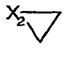 | R | 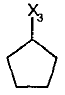 | 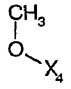 | 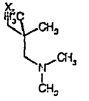 | |
| 52 | 0,079 | 0,153 | H | 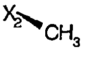 | R | 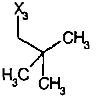 | 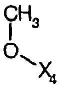 | 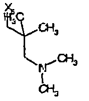 | |
| 53 | 0,076 | 0,245 | 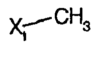 | 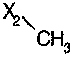 | | 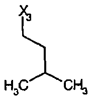 | 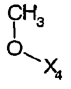 | 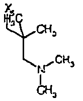 | |
| 54 | 0,031 | 0,226 | H | 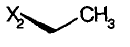 | R | 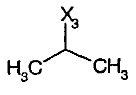 | 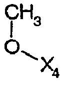 | 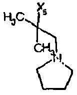 | |
| 55 | 0,013 | 0,086 | H | 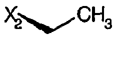 | R | 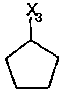 | 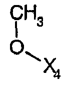 | 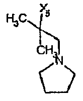 | |
| 56 | 0,064 | 0,324 | H | 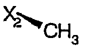 | R | 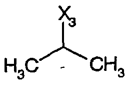 | 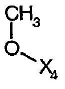 | 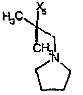 | |
| 57 | 0,038 | 0,067 | H | 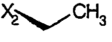 | R | 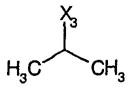 | 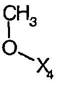 | 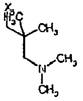 | |
| 58 | 0,010 | 0,023 | H | 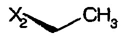 | R | 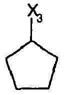 | 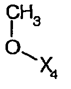 | 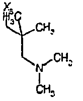 | |
| 59 | 0,095 | 1,000 | H | 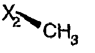 | R | 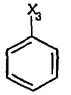 | 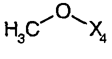 | 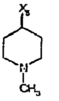 | |
| 60 | 0,041 | 0,189 | H | 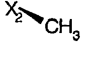 | R | 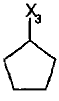 | 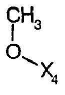 | 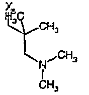 | |
| 61 | 0,065 | 1,040 | 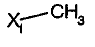 | 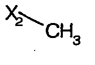 | | 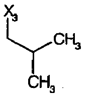 | 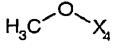 | 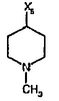 | 217 |
| 62 | 0,058 | 0,538 | H | 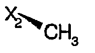 | R | 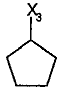 | 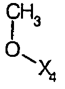 | 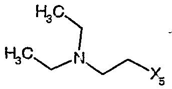 | 259 |
| 63 | 0,081 | 0,206 | H | 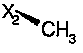 | R | 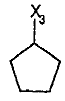 | 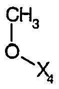 | 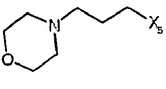 | 184 |
| 64 | 0,064 | 0,894 | H | 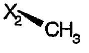 | R | 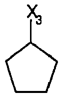 | 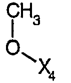 | 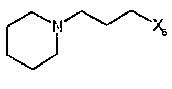 | 175 |
| 65 | 0,052 | 0,216 | H | 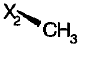 | R | 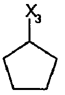 | 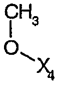 | 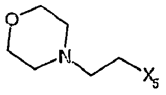 | 172 |
| 66 | 0,064 | 0,250 | H | 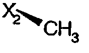 | R | 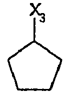 | 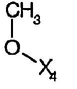 | 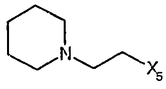 | 164 |
| 67 | 0,052 | 0,566 | H | 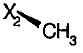 | R | 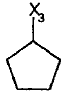 | 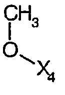 | 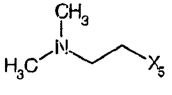 | 115 |
| 68 | 0,092 | 1,242 | H | 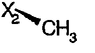 | R | 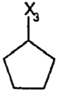 | H | 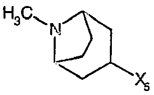 | |
| 69 | 0,055 | 0,633 | H | 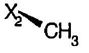 | R | 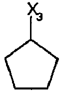 | H | 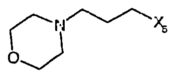 | |
| 70 | 0,092 | 1,000 | H | 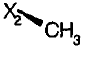 | R | 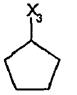 | H | 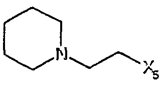 | |
| 71 | 0,035 | 0,227 | H | 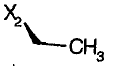 | R | 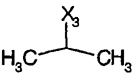 | 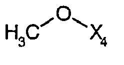 | 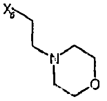 | 146 |
| 72 | 0,050 | | H | 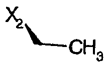 | R | 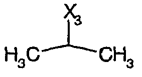 | 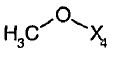 | 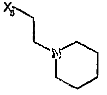 | |
| 73 | 0,091 | | H | 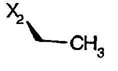 | R | 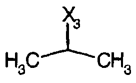 | H | 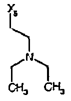 | |
| 74 | 0,043 | | H | 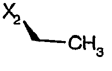 | R | 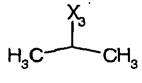 | 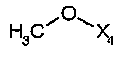 | 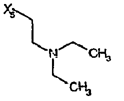 | |
| 75 | 0,046 | 0,557 | H | 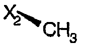 | R | 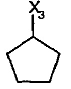 | 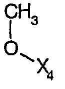 | 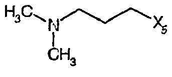 | |
| 76 | 0,068 | | H | 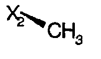 | R | 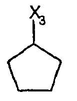 | 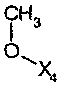 | 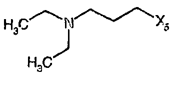 | |
| 77 | 0,055 | 1,000 | H | 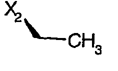 | R | 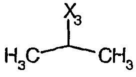 | H | 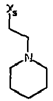 | |
| 78 | 0,035 | 0,610 | H | 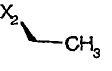 | R | 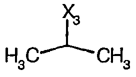 | H | 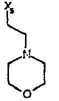 | |
| 79 | 0,043 | | H | 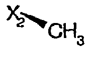 | R | 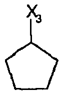 | H | 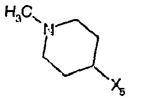 | |
| 80 | 0,038 | 0,286 | H | 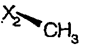 | R | 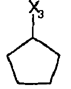 | 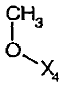 | 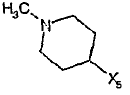 | |
| 81 | 0,050 | 0,237 | H | 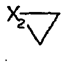 | R | 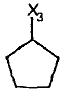 | 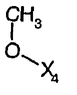 | 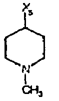 | 194 |
| 82 | 0,083 | 1,580 | H | 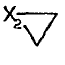 | R | 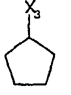 | 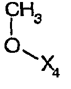 | 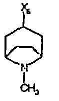 | |
| 83 | 0,069 | 0,263 | H | 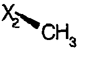 | R | 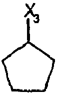 | 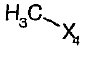 | 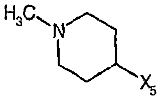 | |
| 84 | 0,015 | 0,312 | H | 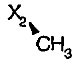 | R | 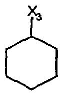 | 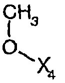 | 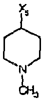 | |
| 85 | 0,025 | 0,754 | H | 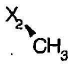 | R | 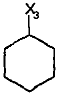 | H | 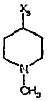 | |
| 86 | 0,038 | 0,024 | H | 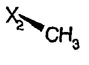 | R | 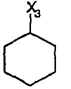 | 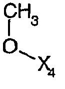 | 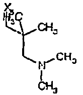 | |
| 87 | 0,029 | 0,066 | H | 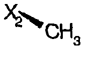 | R | 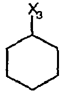 | 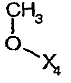 | 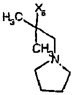 | |
| 88 | 0,020 | 1,018 | H | 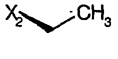 | R | 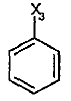 | 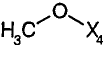 | 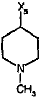 | |
| 89 | 0,047 | 0,245 | H | 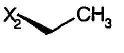 | R | 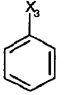 | 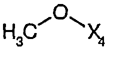 | 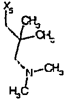 | |
| 90 | 0,032 | 0,137 | H | 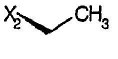 | R | 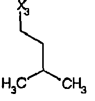 | 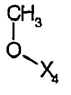 | 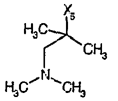 | |
| 91 | 0,041 | 1,780 | H | 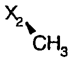 | R | 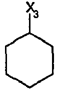 | 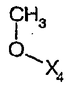 | 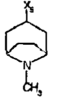 | |
| 92 | 0,043 | | H | 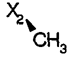 | R | 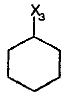 | H | 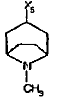 | 131 |
| 93 | 0,060 | | H | 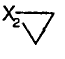 | R | 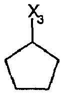 | H | 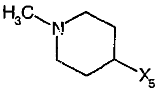 | |
| 94 | 0,018 | 0,510 | H | 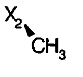 | R | 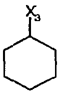 | 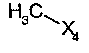 | 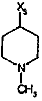 | 178 |
| 95 | 0,047 | 1,000 | H | 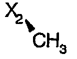 | R | 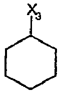 | 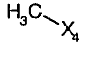 | 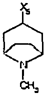 | |
| 96 | 0,011 | 0,577 | H | 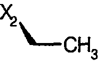 | R | 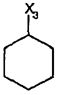 | 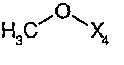 | 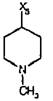 | 203 |
| 97 | 0,032 | 0,066 | H | 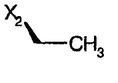 | R | 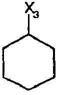 | 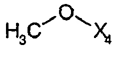 | 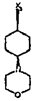 | 179 |
| 98 | 0,077 | 1,319 | H | 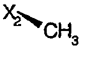 | R | 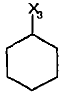 | 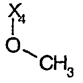 | 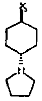 | |
| 99 | 0,025 | 0,209 | H | 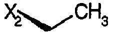 | R | 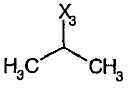 | 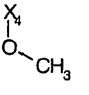 | 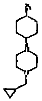 | 280 |
| 100 | 0,058 | 0,124 | H | 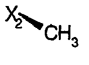 | R | 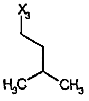 | 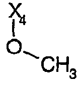 | 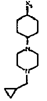 | 233 |
| 101 | 0,031 | 0,124 | H | 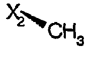 | R | 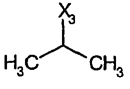 | 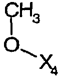 | 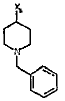 | |
| 102 | 0,006 | 0,053 | H | 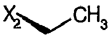 | R | 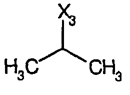 | 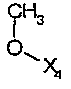 | 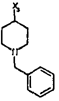 | |
| 103 | 0,005 | 0,076 | H | 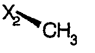 | R | 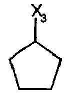 | 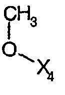 | 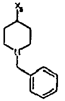 | |
| 104 | 0,041 | 0,133 | H | 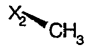 | R | 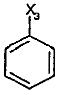 | 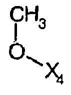 | 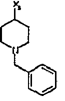 | |
| 105 | 0,015 | 0,055 | H | 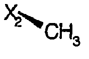 | R | 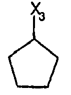 | 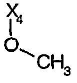 | 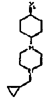 | 229 |
| 106 | 0,025 | 1,000 | H | 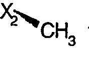 | R | 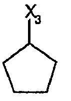 | 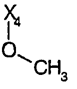 | 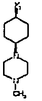 | 213 |
| 107 | 0,043 | 0,363 | H | 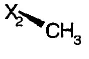 | R | 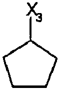 | 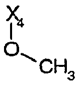 | 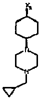 | 285 |
| 108 | 0,016 | 1,000 | H | 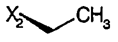 | R | 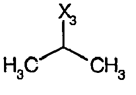 | 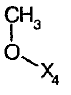 | 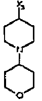 | |
| 109 | 0,031 | 0,760 | H | 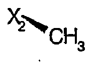 | R | 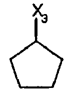 | 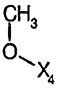 | 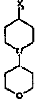 | |
| 110 | 0,027 | 0,122 | H | 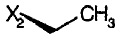 | R | 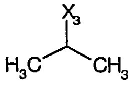 | 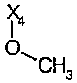 | 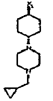 | 278 |
| 111 | 0,023 | 0,503 | H | 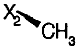 | R | 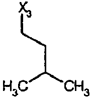 | 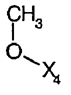 | 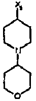 | |
| 112 | 0,075 | 0,345 | H | 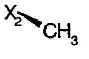 | R | 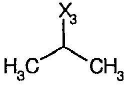 | 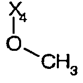 | 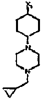 | 264 |
| 113 | 0,037 | 0,687 | H | 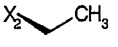 | R | 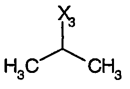 | 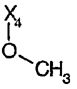 | 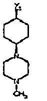 | 279 |
| 114 | 0,064 | 0,766 | H | 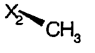 | R | 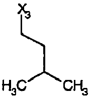 | 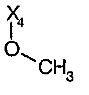 | 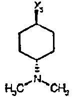 | 234 |
| 115 | 0,016 | 0,076 | H | 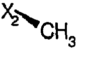 | R | 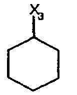 | 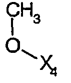 | 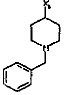 | |
| 116 | 0,006 | 0,174 | H | 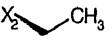 | R | 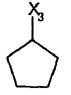 | 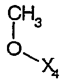 | 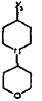 | |
| 117 | 0,010 | 0,370 | H | 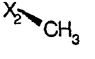 | R | 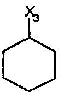 | 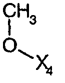 | 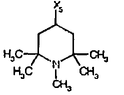 | |
| 118 | 0,010 | 0,279 | H | 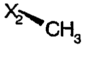 | R | 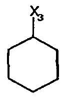 | 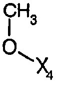 | 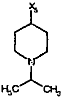 | |
| 119 | 0,008 | 0,438 | H | 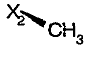 | R | 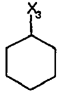 | 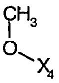 | 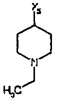 | |
| 120 | 0,014 | 0,374 | H | 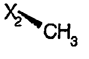 | R | 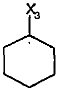 | 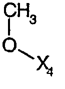 | 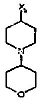 | 154 |
| 121 | 0,011 | 0,427 | H | 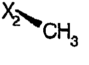 | R | 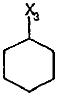 | 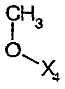 | 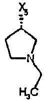 | |
| 122 | 0,009 | 0,261 | H | 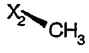 | R | 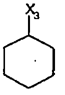 | 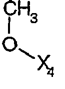 | 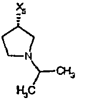 | |
| 123 | 0,043 | 0,490 | H | 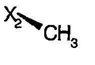 | R | 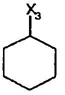 | 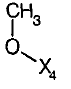 | 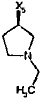 | |
| 124 | 0,006 | 0,391 | H | 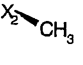 | R | 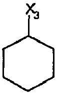 | 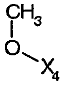 | 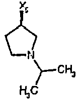 | |
| 125 | 0,049 | 0,795 | H | 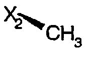 | R | 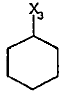 | 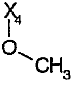 | 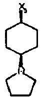 | 283 |
| 126 | 0,051 | 0,742 | H | 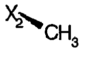 | R | 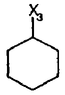 | 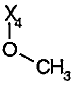 | 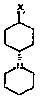 | 238 |
| 127 | 0,045 | 0,787 | H | 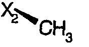 | R | 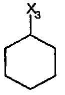 | 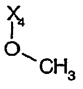 | 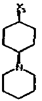 | 221 |
| 128 | 0,015 | 0,282 | H | 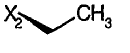 | R | 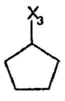 | 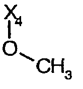 | 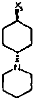 | 257 |
| 129 | 0,035 | 0,646 | H | 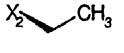 | R | 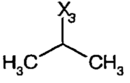 | 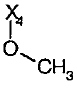 | 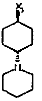 | 284 |
| 130 | 0,015 | 0,091 | H | 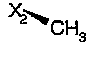 | R | 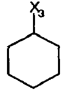 | 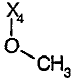 | 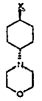 | 236 |
| 131 | 0,023 | 0,101 | H | 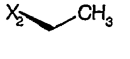 | R | 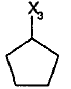 | 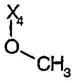 | 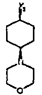 | 141 |
| 132 | 0,062 | 1,000 | H | 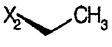 | R | 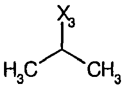 | 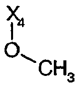 | 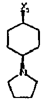 | 268 |
| 133 | 0,011 | 0,075 | H | 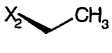 | R | 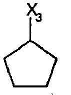 | 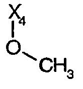 | 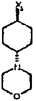 | 272 |
| 134 | 0,015 | 0,063 | H | 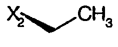 | R | 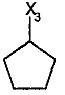 | 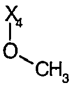 | 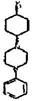 | 319 |
| 135 | 0,012 | 0,699 | H | 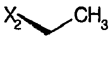 | R | 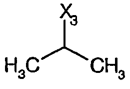 | 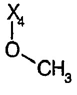 | 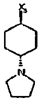 | 289 |
| 136 | 0,041 | 0,821 | H | 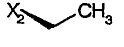 | R | 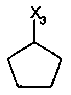 | 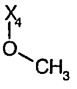 | 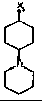 | 201 |
| 137 | 0,039 | 0,148 | H | 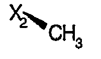 | R | 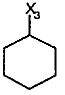 | 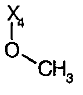 | 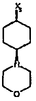 | 223 |
| 138 | 0,043 | 1,137 | H | 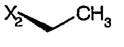 | R | 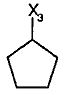 | 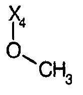 | 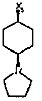 | 217 |
| 139 | 0,097 | 3,000 | H | 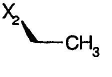 | R | 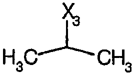 | 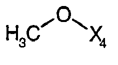 | 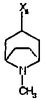 | 112 |
| 140 | 0,029 | 1,970 | H | 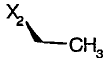 | R | 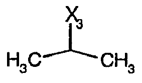 | 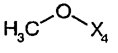 | 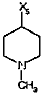 | 215 |
| 141 | 0,057 | | H | 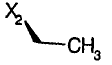 | R | 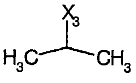 | 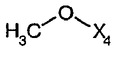 | 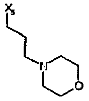 | 198 |
| 142 | 0,030 | | H | 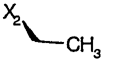 | R | 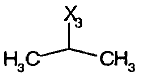 | 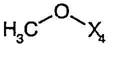 | 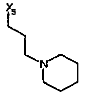 | 192 |
| 143 | 0,086 | | H | 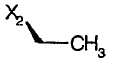 | R | 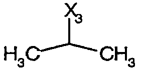 | H | 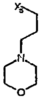 | 139 |
| 144 | 0,044 | | H | 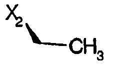 | R | 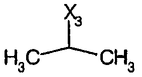 | H | 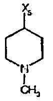 | 191 |
| 145 | 0,043 | 3,000 | H | 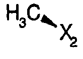 | R | 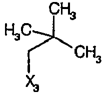 | 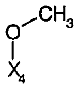 | 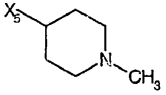 | |
| 146 | 0,066 | 0,966 | H | 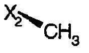 | R | 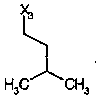 | 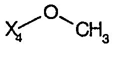 | 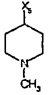 | |
| 147 | 0,055 | 1,760 | H | 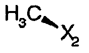 | R | 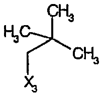 | H | 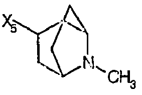 | |
| 148 | 0,087 | | H | 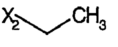 | R | 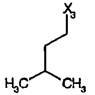 | 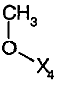 | 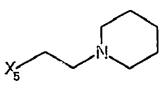 | |
| 149 | 0,085 | | H | 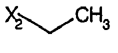 | R | 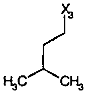 | 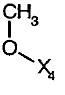 | 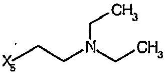 | |
| 150 | 0,043 | | H | 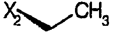 | R | 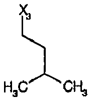 | H | 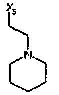 | |
| 151 | 0,043 | | H | 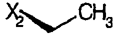 | R | 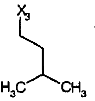 | 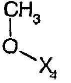 | 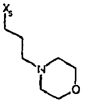 | 155 |
| 152 | 0,081 | | H | 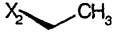 | R | 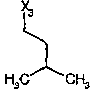 | 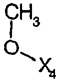 | 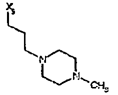 | 147 |
| 153 | 0,072 | 2,640 | H | 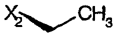 | R | 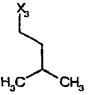 | 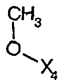 | 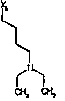 | 128 |
| 154 | 0,097 | | H | 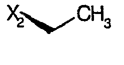 | R | 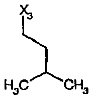 | H | 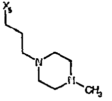 | |
| 155 | 0,079 | | H | 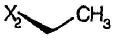 | R | 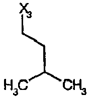 | H | 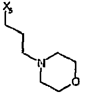 | |
| 156 | 0,048 | 0,481 | H | 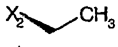 | R | 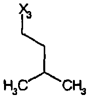 | 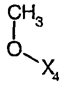 | 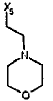 | 173 |
| 157 | 0,051 | | H | 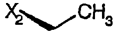 | R | 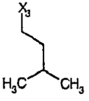 | 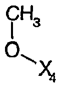 | 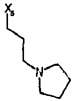 | 134 |
| 158 | 0,043 | 0,536 | H | 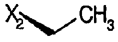 | R | 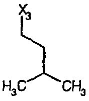 | 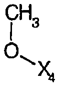 | 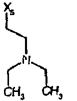 | 230 |
| 159 | 0,029 | 1,000 | H | 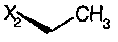 | R | 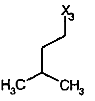 | 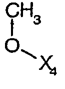 | 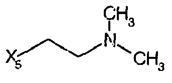 | 260 |
| 160 | 0,037 | 0,728 | H | 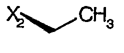 | R | 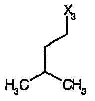 | 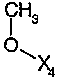 | 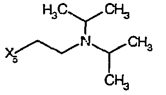 | 193 |
| 161 | 0,081 | | 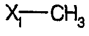 | 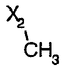 | | 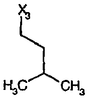 | 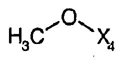 | 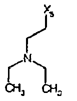 | 199 |
| 162 | 0,071 | 0,903 | 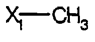 | 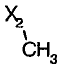 | | 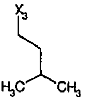 | 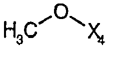 | 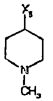 | 254 |
| 163 | 0,071 | 1,000 | H | 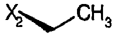 | R | 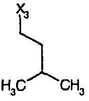 | 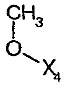 | 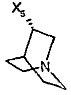 | 249 |
| 164 | 0,030 | 0,975 | H | 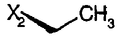 | R | 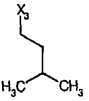 | 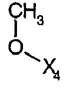 | 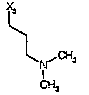 | |
| 165 | 0,046 | 0,668 | H | 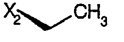 | R | 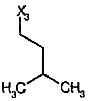 | 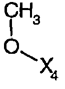 | 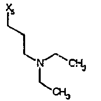 | |
| 166 | | | H | 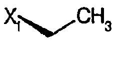 | R | 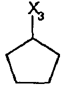 | 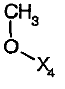 | 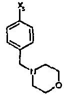 | |
| 167 | 0,034 | 0,817 | H | 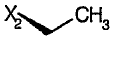 | R | 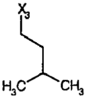 | 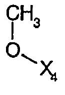 | 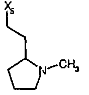 | |
| 168 | 0,042 | 0,088 | H | 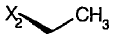 | R | 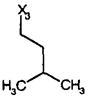 | 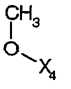 | 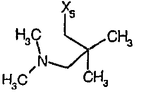 | |
| 169 | 0,044 | 0,503 | H | 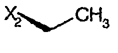 | R | 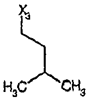 | 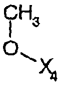 | 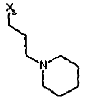 | |
| 170 | 0,045 | 0,427 | H | 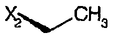 | R | 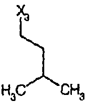 | 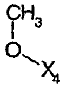 | 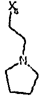 | 170 |
| 171 | 0,031 | 0,790 | H | 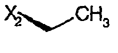 | R | 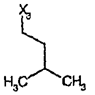 | 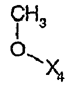 | 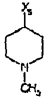 | 196 |
| 172 | 0,020 | 0,772 | H | 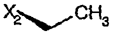 | R | 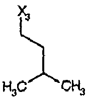 | 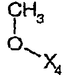 | 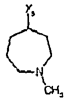 | 166 |
| 173 | 0,070 | 0,871 | H | 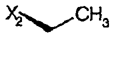 | R | 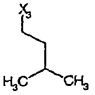 | 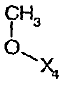 | 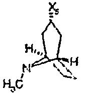 | |
| 174 | 0,000 | 0,155 | H | 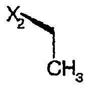 | R | 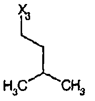 | 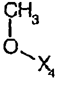 | 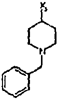 | |
| 175 | 0,000 | 0,296 | H | 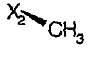 | R | 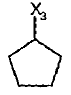 | 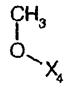 | 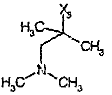 | |
| 176 | 0,000 | 0,301 | H | 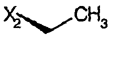 | R | 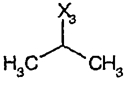 | 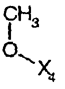 | 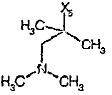 | |
| 177 | 0,000 | 0,082 | H | 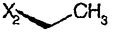 | R | 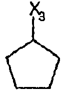 | 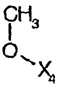 | 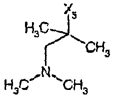 | |
| 178 | 0,000 | 1,000 | H | 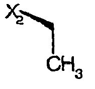 | R | 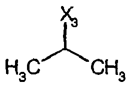 | 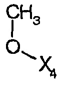 | 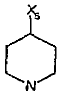 | |
| 179 | 0,000 | 1,000 | H | 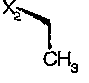 | R | 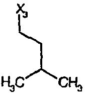 | 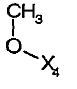 | 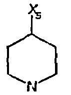 | |
| 180 | 0,000 | 0,147 | H | 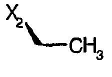 | R | 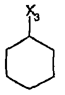 | 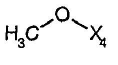 | 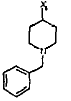 | 136 |
| 181 | 0,000 | 0,754 | H | 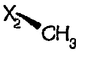 | R | 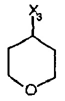 | 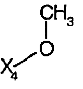 | 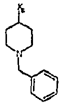 | |
| 182 | | | H | 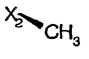 | R | 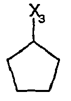 | 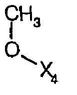 | 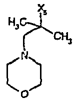 | |
| 183 | | | H | 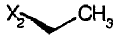 | R | 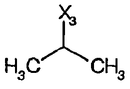 | 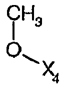 | 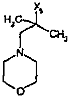 | |
| 184 | | | H | 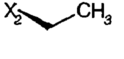 | R | 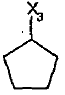 | 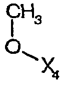 | 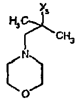 | |
| 185 | | | H | 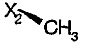 | R | 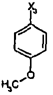 | 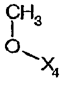 | 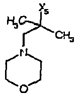 | 217 |
| 186 | | | H | 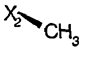 | R | 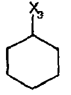 | 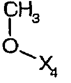 | 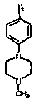 | 237 |
| 187 | | | H | 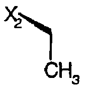 | R | 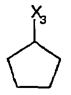 | 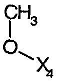 | 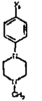 | 223 |
| 188 | | | H | 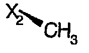 | R | 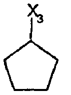 | 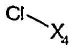 | 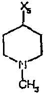 | 161 |
| 189 | | | H | 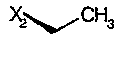 | R | 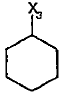 | 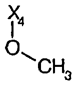 | 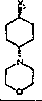 | 267 |
| 190 | | | H | 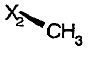 | R | 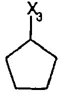 | 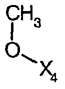 | 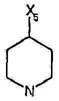 | |
| 191 | | | H | 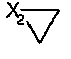 | R | 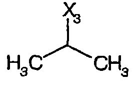 | 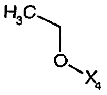 | 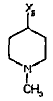 | |
| 192 | | | H | 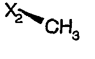 | R | 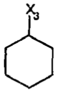 | 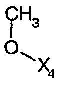 | 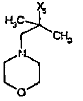 | |
| 193 | | | H | 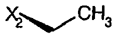 | R | 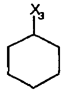 | 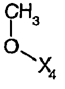 | 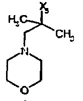 | |
| 194 | | | H | 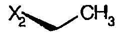 | R | 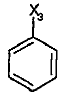 | 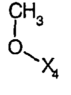 | 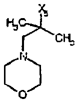 | |
| 195 | | | H | 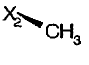 | R | 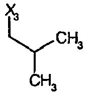 | 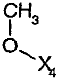 | 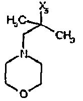 | |
| 196 | | | H | 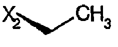 | R | 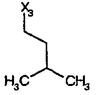 | 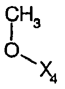 | 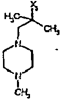 | |
| 197 | | | H | 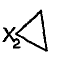 | | 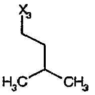 | 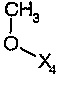 | 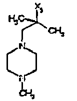 | |
| 198 | | | H | 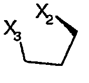 | R | | 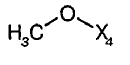 | 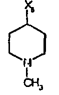 | 301 |
| 199 | | | H | 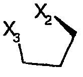 | R | | 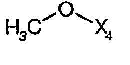 | 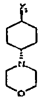 | 291 |
| 200 | | | H | 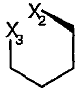 | R | | 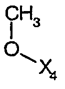 | 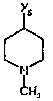 | |
| 201 | | | H | 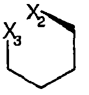 | R | | 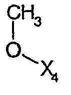 | 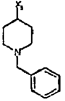 | |
| 202 | | | 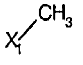 | 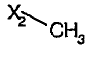 | | 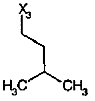 | 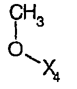 | 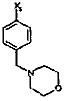 | |
| 203 | | | H | 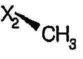 | R | 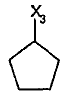 | 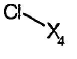 | 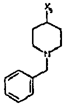 | |
| 204 | | | H | 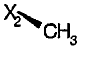 | R | 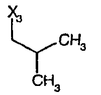 | 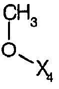 | 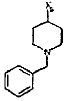 | |
| 205 | | | H | 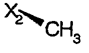 | R | 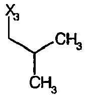 | 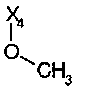 | 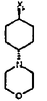 | 255 |
| 206 | | | H | 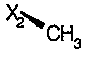 | R | 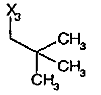 | 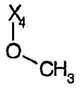 | 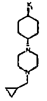 | 190 |
| 207 | | | H | 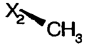 | R | 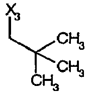 | 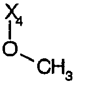 | 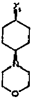 | 236 |
| 208 | | | H | 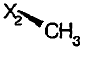 | R | 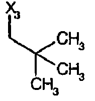 | 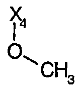 | 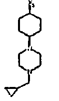 | 192 |
| 209 | | | H | 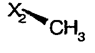 | R | 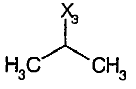 | 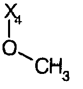 | 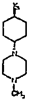 | 211 |
| 210 | | | H | 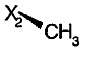 | R | 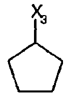 | 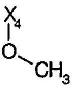 | 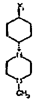 | 210 |
| 211 | | | 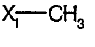 | 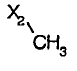 | | 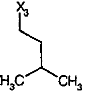 | 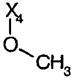 | 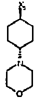 | 182 |
| 212 | | | 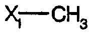 | 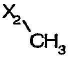 | | 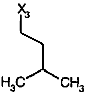 | 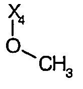 | 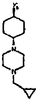 | 187 |
| 213 | | | H | 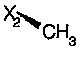 | R | 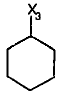 | 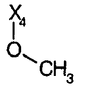 | 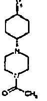 | 248 |
| 214 | | | H | 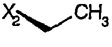 | R | 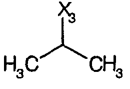 | 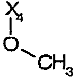 | 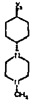 | 338 |
| 215 | | | 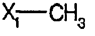 | 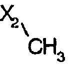 | | 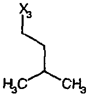 | 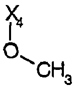 | 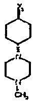 | 232 |
| 216 | | | H | 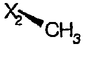 | R | 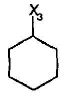 | 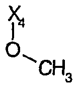 | 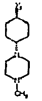 | 217 |
| 217 | | | H | 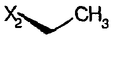 | R | 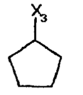 | 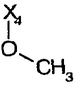 | 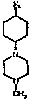 | 139 |
| 218 | | | H | 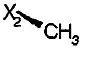 | R | 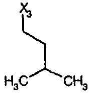 | 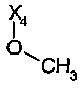 | 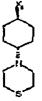 | |
| 219 | | | H | 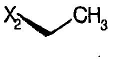 | R | 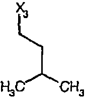 | 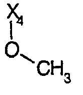 | 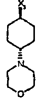 | 298 |
| 220 | | | H | 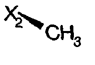 | R | 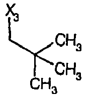 | 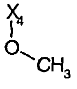 | 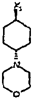 | 279 |
| 221 | | | H | 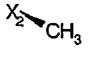 | R | 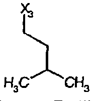 | 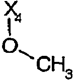 | 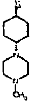 | 336 |
| 222 | | | H | 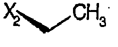 | R | 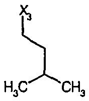 | 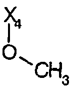 | 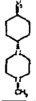 | 241 |
| 223 | | 1 | H | 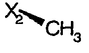 | R | 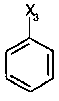 | 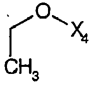 | 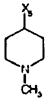 | |
| 224 | 1,056 | 1 | H | 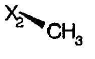 | R | 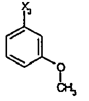 | 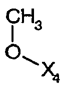 | 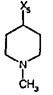 | |
| 225 | 1,18 | 1 | H | 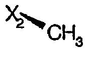 | R | 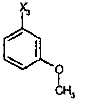 | H | 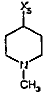 | |
| 226 | 0,295 | 1 | H | 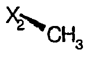 | R | 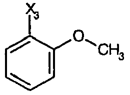 | 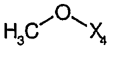 | 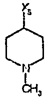 | |
| 227 | 0,853 | 0,946 | H | 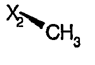 | R | 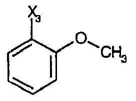 | 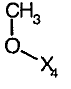 | 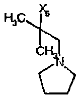 | |
| 228 | | 1 | H | 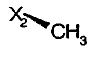 | R | 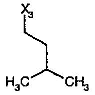 | 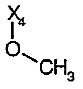 | 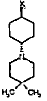 | |
| 229 | | 0,381 | H | 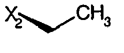 | R | 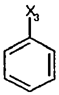 | 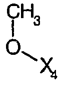 | 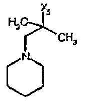 | |
| 230 | | 0,193 | H | 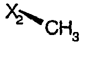 | R | 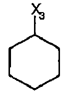 | 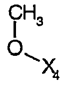 | 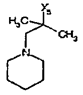 | |
| 231 | | 0,17 | H | 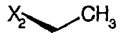 | R | 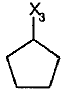 | 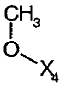 | 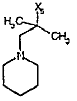 | |
| 232 | | 1 | H | 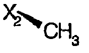 | R | 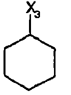 | 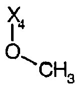 | 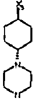 | |
| 233 | | | H | 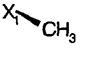 | R | 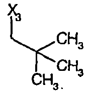 | 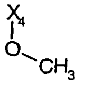 | 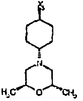 | |
| 234 | | | H | 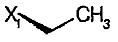 | R | 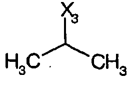 | 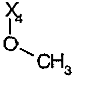 | 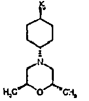 | |
| 235 | | | H | 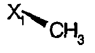 | R | 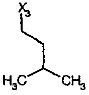 | 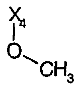 | 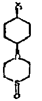 | |
| 236 | | | H | 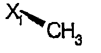 | R | 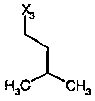 | 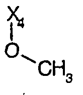 | 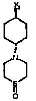 | |
| 237 | 0,012 | 1 | H | 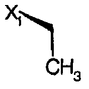 | R | 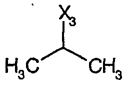 | 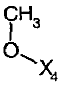 | 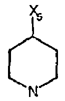 | |
| 238 | 0,015 | 1 | H | 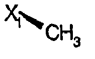 | R | 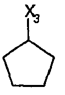 | 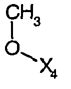 | 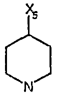 | |
| 239 | 0,051 | 0,07 | H | 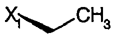 | R | 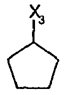 | 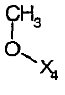 | 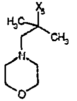 | |
| 240 | 0,017 | 0,054 | H | 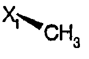 | R | 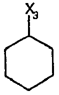 | 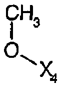 | 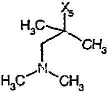 | |
| 241 | 0,044 | 0,093 | H | 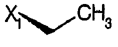 | R | 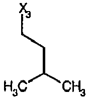 | 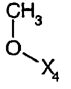 | 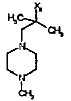 | |
| 242 | 0,019 | 0,524 | H | 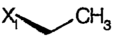 | R | 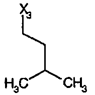 | 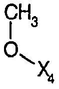 | 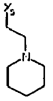 | |
| 243 | 0,018 | 0,975 | H | 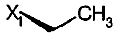 | R | 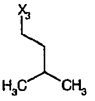 | 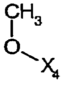 | 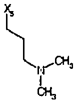 | |
| 244 | 0,0115 | | H | 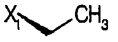 | R | 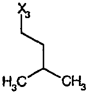 | 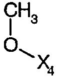 | 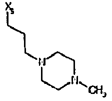 | |

Wie gefunden wurde, zeichnen sich die Verbindungen der allgemeinen Formel (I) durch vielfältige Anwendungsmöglichkeiten auf therapeutischem Gebiet aus. Hervorzuheben sind solche Anwendungsmöglichkeiten, für welche die Inhibition spezifischer Zellzykluskinasen, insbesondere die inhibierende Wirkung auf die Proliferation kultivierter humaner Tumorzellen, aber auch auf die Proliferation anderer Zellen, wie z.B. Endothelzellen, eine Rolle spielen.

Wie durch FACS Analyse gezeigt werden konnte, ist die, durch die erfindungsgemäßen Verbindungen bewirkte, Proliferationsinhibition vermittelt durch einen Arrest der Zellen vor allem in der G2/M Phase des Zellzyklus. Die Zellen arretieren abhängig von den verwendeten Zellen für eine bestimmte Zeitspanne in dieser Zellzyklus Phase, bevor der programmierte Zelltod eingeleitet wird. Ein Arrest in der G2/M Phase des Zellzyklus wird z.B. durch die Inhibition spezifischer Zellzykluskinasen ausgelöst. Studien in Modellorganismen wie *Schizosaccharomyces pombe* oder *Xenopus,* oder Untersuchungen in menschlichen Zellen haben gezeigt, das der Übergang von der G2 Phase zur Mitose durch die CDK1/Cyclin B Kinase reguliert wird (Nurse, 1990). Diese Kinase, die auch als "mitosis promoting factor" (MPF) bezeichnet wird, phosphoryliert und reguliert dadurch eine Vielzahl von Proteinen, wie z.B. nukleäre Lamine, Kinesin-ähnliche Motorproteine, Kondensine und Golgi Matrix Proteine, die eine wichtige Rolle beim Abbau der Kernhülle, bei der Centrosomen Separation, dem Aufbau des mitotischen Spindelapparates, der Chromosomen Kondenation und Abbau des Golgi Apparates, spielen (Nigg. E., 2001). Eine murine Zell-Linie mit einer temperatursensitiven CDK1 Kinasemutante zeigt nach Temperaturerhöhung einen raschen Abbau der CDK1-Kinase und einen darauffolgenden Arrest in der G2/M Phase (Th'ng et al., 1990). Die Behandlung von humanen Tumorzellen mit Inhibitoren gegen CDK1/Cyclin B wie z.B. Butyrolacton führt ebenfalls zu einen Arrest in der G2/M Phase und anschließender Apoptose (Nishio, et al. 1996). Eine weitere Kinase, die in der G2 und Mitose Phase eine Rolle spielt, ist Polo-like Kinase 1 (Plk1), die für die Reifung der Centrosomen, für die Aktivierung der Phosphatase Cdc25C, sowie für die Aktivierung des Anaphase Promoting Complex verantwortlich ist (Glover et al., 1998, Qian, et al., 2001). Die Injektion von Plk1 Antikörpern führt zu einem G2 Arrest in nicht transformierten Zellen während Tumorzellen in der Mitosephase arretieren (Lane und Nigg, 1996). Darüber hinaus wurde auch für die Proteinkinase aurora B eine essentielle Funktion beim Eintritt in die Mitose beschrieben. Aurora B phosphoryliert Histon H3 an Ser11 und leitet damit die Chromosomenkondensation ein (Hsu, J.Y. et al., 2000). Ein spezifischer Zellzylzlusarrest in der G2/M Phase kann aber auch z.B. durch Inhibition von spezifischen Phosphatasen wie z.B. Cdc25C (Russell and Nurse, 1986) ausgelöst werden. Hefen mit defektem cdc25 Gen arretieren in der G2 Phase, während eine Überexpression von cdc25 zu einem verfrühten Eintritt in die Mitosephase führt (Russell und Nurse, 1987). Ein Arrest in der G2/M Phase kann aber auch durch Inhibition von bestimmten Motorproteinen, sogenannten Kinesinen wie z.B. Eg5 (Mayer et al., 1999), oder durch Mikrotubuli stabilisierende oder destabilisierende Agentien (z.B. Colchicin, Taxol, Etoposid, Vinblastin, Vincristin) ausgelöst werden (Schiff und Horwitz, 1980).

Auf Grund ihrer biologischen Eigenschaften eignen sich die erfindungsgemäßen Verbindungen der allgemeinen Formel I, deren Isomere und deren physiologisch verträgliche Salze zur Behandlung von Erkrankungen, die durch exzessive oder anomale Zellproliferation charakterisiert sind.

Zu solchen Erkrankungen gehören beispielsweise: Virale Infektionen (z.B. HIV und Kaposi Sarkoma); Entzündung und Autoimmun-Erkrankungen (z.B. Colitis, Arthritis, Alzheimer Erkrankung, Glomerulonephritis und Wund-Heilung); bakterielle, fungale und/oder parasitäre Infektionen; Leukämien, Lymphoma und solide Tumore; Haut-Erkrankungen (z.B. Psoriasis); Knochen-Erkrankungen; kardiovaskuläre Erkrankungen (z.B. Restenose und Hypertrophie). Ferner sind sie nützlich als Schutz von proliferierenden Zellen (z.B. Haar-, Intestinal-, Blut- und Progenitor-Zellen) gegen DNA-Schädigung durch Strahlung, UV-Behandlung und/oder zytostatischer Behandlung (Davis et al., 2001). Die neuen Verbindungen können zur Prävention, Kurz- oder Langzeitbehandlung der vorstehend genannten Erkrankungen, auch in Kombination mit anderen Wirkstoffen, die für dieselben Indikationen Verwendung finden, z.B. Cytostatika, verwendet werden.

Die Wirkung der erfindungsgemäBen Verbindungen wurde im Cytotoxizitätstest an kultivierten humanen Tumorzellen und/oder in einer FACS-Analyse, beispielsweise an HeLaS3-Zellen, bestimmt. Die Verbindungen zeigten in beiden Testmethoden eine gute bis sehr gute Wirksamkeit, d.h. beispielsweise einen EC₅ₒ-Wert im HeLaS3-Cytotoxizitätstest kleiner 5 µmol/L, in der Regel kleiner 1 µmol/L.

### Messung der Cytotoxizität an kultivierten humanen Tumorzellen

Zur Messung der Cytotoxizität an kultivierten humanen Tumorzellen wurden Zellen der zervikalen Carcinoma Tumorzell-Linie HeLaS3 (erhalten von American Type Culture Collection (ATCC)) in Ham's F12 Medium (Life Technologies) und 10% fötalem Rinderserum (Life Technologies) kultiviert und in der log-Wachstumsphase geerntet. Anschließend wurden die HeLaS3 Zellen in 96-well Platten (Costar) mit einer Dichte von 1000 Zellen pro well eingebracht und über Nacht in einem Inkubator (bei 37°C und 5 % CO₂) inkubiert, wobei auf jeder Platte 6 wells nur mit Medium gefüllt wurden (3 wells zur Mediumkontrolle, 3 wells zur Inkubation mit reduzierten AlamarBlue). Die Wirksubstanzen wurden in verschiedenen Konzentrationen (gelöst in DMSO; Endkonzentration: 1%) zu den Zellen zugegeben (jeweils als Dreifachbestimmung) Nach 72 Stunden Inkubation wurden zu jeden well 20 *µ*l AlamarBlue (AccuMed International) zugesetzt, und die Zellen für weitere 7 Stunden inkubiert. Zur Kontrolle wurde zu 3 wells je 20 µl reduziertes Alamar Blue gegeben (AlamarBlue Reagenz, das für 30 min autoklaviert wurde). Nach 7 h Inkubation wurde der Farbumsatz des AlamarBlue Reagenz in den einzelnen wells in einem Perkin Elmer Fluoreszenspektrophotometer bestimmt (Exitation 530 nm, Emission 590 nm, Slits 15, Integrate time 0.1). Die Menge an umgesetzten AlamarBlue Reagenz repräsentiert die metabolische Aktivität der Zellen. Die relative Zellaktivität wurde in Prozent der Kontrolle (HeLa S3 Zellen ohne Inhibitor) berechnet und die Wirkstoffkonzentration, die die Zellaktivität zu 50% hemmt (IC⁵⁰) abgeleitet. Die Werte wurden hierbei aus dem Mittelwert von drei Einzelbestimmungen - unter Korrektur des Leerwertes (Mediumkantrolle)- berechnet.

### FACS- Analyse

Propidium Iodid (P1) bindet stöchiometrisch an doppelsträngige DNA, und ist damit geeignet den Prozentsatz an Zellen in der G1, S, und G2/M Phase des Zellzykluses auf der Basis des zellulären DNA Gehaltes zu bestimmen. Zellen in der G0 und G1 Phase haben einen diploiden DNA Gehalt (2N), während Zellen in der G2 oder Mitose einen 4N DNA Gehalt haben.
Für eine Pl-Färbung wurden beispielsweise 0.4 Mio HeLaS3 Zellen auf eine 75 cm² Zellkulturflasche ausgesät, nach 24 h wurde entweder 1 % DMSO als Kontrolle zugesetzt, bzw. die Substanz in verschiedenen Konzentrationen (in 1 % DMSO). Die Zellen wurden für 24 h mit der Substanz bzw. mit DMSO inkubiert, bevor die Zellen mit 2 x PBS gewaschen und mit Trypsin /EDTA abgelöst wurden. Die Zellen wurden zentrifugiert (1000 Upm, 5 min, 4°C), und das Zellpellet 2 x mit PBS gewaschen, bevor die Zellen in 0.1 ml PBS resuspendiert wurden. Anschließend wurden die Zellen für 16 Stunden bei 4°C oder alternativ für 2 Stunden bei -20°C mit 80% Ethanol fixiert. Die fixierten Zellen (10⁶ Zellen) wurden zentrifugiert (1000 Upm, 5min, 4°C), mit PBS gewaschen und anschließend nochmals zentrifugiert . Das Zellpellet wurde in 2 ml Triton X-100 in 0.25 % PBS resuspendiert, und 5 min auf Eis inkubiert, bevor 5 ml PBS zugeben wurden und erneut zentrifugiert wurde. Das Zellpellet wurde in 350 µl Pl Färbelösung (0.1 mg/ml RNase A, 10 µg/ml Prodium lodid in 1 x PBS) resuspendiert. Die Zellen wurden für 20 min im Dunkeln mit dem Färbepuffer inkubiert, bevor sie in Probenmessgefäße für das FACS Scan überführt werden. Die DNA Messung erfolgte in einem Becton Dickinson FACS Analyzer, mit einen Argonlaser (500 mW, Emission 488 nm), und dem DNA Cell Quest Programm (BD). Die logarithmische Pl Fluoreszenz wurde mit einem band-pass Filter (BP 585/42) bestimmt. Die Quantifizierung der Zellpopulationen in den einzelnen Zellzyklusphasen erfolgte mit dem ModFit LT Programm von Becton Dickinson.

Die Verbindungen der allgemeinen Formel (I) können allein oder in Kombination mit anderen erfindungsgemäßen Wirkstoffen, gegebenenfalls auch in Kombination mit weiteren pharmakologisch aktiven Wirkstoffen, zur Anwendung gelangen. Geeignete Anwendungsformen sind beispielsweise Tabletten, Kapseln, Zäpfchen, Lösungen, - insbesondere Lösungen zur Injektion (s.c., i.v., i.m.) und Infusion - Säfte, Emulsionen oder dispersible Pulver. Hierbei soll der Anteil der pharmazeutisch wirksamen Verbindung(en) jeweils im Bereich von 0,1 - 90 Gew.-%, bevorzugt 0,5 - 50 Gew.-% der Gesamtzusammensetzung liegen, d.h. in Mengen die ausreichend sind, um den unten angegebenen Dosierungsbereich zu erreichen. Die genannten Dosen können, falls erforderlich, mehrmals täglich gegeben werden. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektions- und Infusionslösungen werden in üblicher Weise, z.B. unter Zusatz von Isotonantien, Konservierungsmitteln, wie p-Hydroxybenzoaten, oder Stabilisatoren, wie Alkalisalzen der Ethylendiaminfietraessigsäure, gegebenenfalls unter Verwendung von Emulgiermitteln und /oder Dispergiermitteln, wobei beispielsweise bei der Verwendung von Wasser als Verdünnungsmittel gegebenenfalls organische Lösemittel als Lösevermittler bzw. Hilflösemittel eingesetzt werden können, hergestellt und in Injektionsflaschen oder Ampullen oder Infusionsflaschen abgefüllt.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.
Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.
Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuß- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Die Applikation erfolgt in üblicher Weise, vorzugsweise oral oder transdermal, insbesondere bevorzugt oral. Im Falle der oralen Anwendung können die Tabletten selbstverständlich außer den genannten Trägerstoffen auch Zusätze, wie z.B. Natriumcitrat, Calciumcarbonat und Dicafciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.
Für den Fall der parenteralen Anwendung können Lösungen der Wirkstoffe unter Verwendung geeigneter flüssiger Trägermaterialien eingesetzt werden. Die Dosierung für die intravenöse Anwendung liegt bei 1 - 1000 mg pro Stunde, vorzugsweise zwischen 5 - 500 mg pro Stunde.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über der Tag zu verteilen.

Die nachfolgenden Formulierungsbeispiele illustrieren die vorliegende Erfindung ohne sie jedoch in ihrem Umfang zu beschränken:

### Pharmazeutische Formulierungsbeispiele

| A) | Tabletten | pro Tablette |
|---|---|---|
| | Wirkstoff | 100 mg |
| | Milchzucker | 140 mg |
| | Maisstärke | 240 mg |
| | Polyvinylpyrrolidon | 15 mg |
| | Magnesiumstearat | 5 mg |
| | | 500 mg |

Der feingemahlene Wirkstoff, Milchzucker und ein Teil der Maisstärke werden miteinander vermischt. Die Mischung wird gesiebt, danach mit einer Lösung von Polyvinylpyrrolidon in Wasser befeuchtet, geknetet, feuchtgranuliert und getrocknet. Das Granulat, der Rest der Maisstärke und das Magnesiumstearat werden gesiebt und miteinander vermischt. Das Gemisch wird zu Tabletten geeigneter Form und Größe verpreßt.

| B) | Tabletten | pro Tablette |
|---|---|---|
| | Wirkstoff | 80 mg |
| | Milchzucker | 55 mg |
| | Maisstärke | 190 mg |
| | Mikrokristalline Cellulose | 35 mg |
| | Polyvinylpyrrolidon | 15 mg |
| | Natrium-carboxymethylstärke | 23 mg |
| | Magnesiumstearat | 2 mg |
| | | 400 mg |

Der feingemahlene Wirkstoff, ein Teil der Maisstärke, Milchzucker, mikrolcristalline Cellulose und Polyvinylpyrrolidon werden miteinander vermischt, die Mischung gesiebt und mit dem Rest der Maisstärke und Wasser zu einem Granulat verarbeitet, welches getrocknet und gesiebt wird. Dazu gibt man die Natriumcarboxymethylstärke und das Magnesiumstearat, vermischt und verpreßt das Gemisch zu Tabletten geeigneter Größe.

| C) | Ampullenlösung | |
|---|---|---|
| | Wirkstoff | 50 mg |
| | Natriumchlorid | 50 mg |
| | Aqua pro inj. | 5 ml |

Der Wirkstoff wird bei Eigen-pH oder gegebenenfalls bei pH 5,5 - 6,5 in Wasser gelöst und mit Natriumchlorid als Isotonans versetzt. Die erhaltene Lösung wird pyrogenfrei filtriert und das Filtrat unter aseptischen Bedingungen in Ampullen abgefüllt, die anschließend sterilisiert und zugeschmolzen werden. Die Ampullen enthalten 5 mg, 25 mg und 50 mg Wirkstoff.

## Patentansprüche

1. Verbindungen der allgemeinen Formel (1), 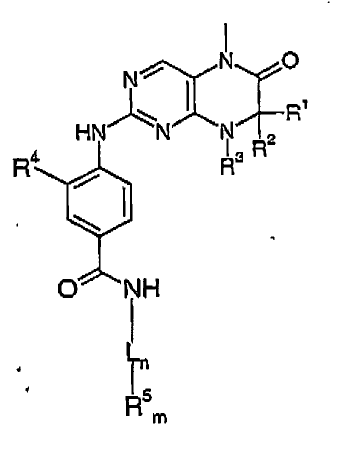 worin
R¹, R² gleich oder verschieden, Wasserstoff oder gegebenenfalls substituiertes C₁-C₈-Alkyl,
oder
R¹ und R² gemeinsam eine 2- bis 5-gliedrige Alkylbrücke, die 1 bis 2 Heteroatome enthalten kann,
R³ Wasserstoff oder ein Rest ausgewählt aus der Gruppe bestehend aus gegebenenfalls substituiertem C₁-C₁₂-Alkyl, C₂-C₁₂-Alkenyl, C₂-C₁₂-Alkinyl und C₆-C₁₄-Aryl, oder
ein Rest ausgewählt aus der Gruppe bestehend aus gegebenenfalls substituiertes C₃-C₁₂-Cycloalkyl, C₃-C₁₂-Cycloalkenyl, C₇-C₁₂-Polycycloalkyl, C₇-C₁₂-Polycycloalkenyl, C₅-C₁₂Spirocycloalkyl, gegebenenfalls substituiertes C₃-C₁₂-Heterocycloalkyl, das 1 bis 2 Heteroatome enthält, und
C₃-C₁₂-Heterocycloalkenyl, das 1 bis 2 Heteroatome enthält, oder
R¹ und R³ oder R² und R³ gemeinsam eine gesättigte oder ungesättigte C₃-C₄-Alkylbrücke, die 1 Heteroatom enthalten kann,
R⁴ ein Rest ausgewählt aus der Gruppe bestehend aus Wasserstoff, -CN, Hydroxy, -NR₆R₇ und Halogen, oder
ein Rest ausgewählt aus der Gruppe bestehend aus gegebenenfalls substituiertem C₁-C₆-Alkyl, C₂-C₆-Alkenyl, C₂-C₈-Alkinyl, C₁-C₅-Alkyloxy, C₂-C₅-Alkenyloxy, C₂-C₅-Alkinyloxy, C₁-C₆-Alkylthio, C₁-C₆-Alkylsulfoxo und C₁-C₆-Alkylsulfonyl,
L ein Linker ausgewählt aus der Gruppe bestehend aus gegebenenfalls substituiertem C₂-C₁₀-alkyl, C₂-C₁₀-Alkenyl, C₆-C₁₄-Aryl, -C₂-C₄-Alkyl-C₆-C₁₄-Aryl,-C₆-C,₄-Aryl-C₁-C₄-Alkyl, C₃-C₁₂-Cycloalkyl und Heteroaryl, das 1 oder 2 Stickstoffatome enthält,
n 0 oder 1
m 1 oder 2
R⁵ ein Rest, ausgewählt aus der Gruppe bestehend aus gegëbenenfalls substituiertem Morpholinyl, Piperidinyl, Piperazinyl, Piperazinylcarbonyl, Pyrrolidinyl, Tropenyl, R⁸-Diketomethylpiperazinyl, Sulfioxomorpholinyl, Sulfonylmorpholinyl, Thiomorpholinyl, -NR⁸R⁹ und Azacycloheptyl,
R⁶, R⁷ gleich oder verschieden, Wasserstoff oder C₁-C₄-Alkyl, und
R⁸, R⁹ unsubstituierte Stickstoffsubstituenten an R⁶, gleich oder verschieden, entweder Wasserstoff oder ein Rest ausgewählt aus der Gruppe bestehend aus C₁-C₆-Alkyl, -C₁-C₄-Alkyl-C₃-C₁₀-Cycloalkyl, C₃-C₁₀-Cycloalkyl, C₆-C₁₄-Aryl, -C₁-C₄-Alkyl-C₆-C₁₄-aryl, Pyranyl, Pyridinyl, Pyrimidinyl, C₁-C₄-Alkyloxycarbonyl, C₆-C₁₄-Arylcarbonyl-, C₁-C₄-Alkylcarbonyl-, C₆-C₁₄-Arylmethyloxycarbonyl-, C₆-C₁₄-Arylsulfonyl-, C₁-C₄-Alkylsulfonyl- und C₆-C₁₄-Aryl-C₁-C₄-Alkylsulfonyl-,
wobei der Begriff Heterocycloalkyl, soweit in den Definitionen nicht anders beschrieben, für 3 bis 12 gliedrige gesättigte oder ungesättigte Heterocyclen steht, die als Heteroatome Stickstoff, Sauerstoff oder Schwefel enthalten können,
wobei der Begriff Alkenyl, soweit es Bestandteil anderer Reste ist, für verzweigte und unverzweigte Alkylengruppen mit 2 bis 10 Kohlenstoffatomen steht, soweit sie mindestens eine Doppelbindung aufweisen,
wobei der Begriff Aryl für ein aromatisches Ringsystem mit 6 bis 14 Kohlenstoffatomen steht,
wobei die Substituenten der gegebenenfalls substituiertem Alkylgruppen, soweit nicht anders beschrieben, ausgewählt sein können aus ein oder mehrere Fluoratome,
wobei die Substituenten der gegebenenfalls substituiertem Alkenylgruppen, soweit nicht anders beschrieben, ausgewählt sein können aus ein oder mehrere Fluoratome,
wobei die Substituenten der gegebenenfalls substituiertem Alkinylgruppen, soweit nicht anders beschrieben, ausgewählt sein können aus ein oder mehrere Fluoratome,
wobei die Substituenten der gegebenenfalls substituiertem Cycloalkylgruppen, soweit nicht anders beschrieben, ausgewählt sein können aus -OH, -NO₂, -CN, - OMe, -OCHF₂, -OCF₃, -NH₂, oder Halogen,
wobei die Substituenten der gegebenenfalls substituiertem Heterocycloalkylgruppen, soweit nicht anders beschrieben, ausgewählt sein können aus C₁-C₄-Alkyl,
wobei die Substituenten der gegebenenfalls substituiertem Arylgruppen, soweit nicht anders beschrieben, ausgewählt sein können aus -OH, -NO₂, -CN, -OMe, - OCHF₂, -OCF₃, -NH₂, oder Halogen,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.

2. Verbindungen nach Anspruch 1, worin
R¹ bis R⁴, R⁶ und R⁷ die angegebene Bedeutung aufweisen, und
L ein Linker ausgewählt aus der Gruppe bestehend aus gegebenenfalls substituiertem C₂-C₁₀-Alkyl, C₂-C₁₀-Alkenyl, C₆-C₁₄-Aryl, -C₂-C₄-Alkyl-C₆-C₁₄-Aryl, - C₆-C₁₄-Aryl-C₁-C₄-Alkyl, gegebenenfalls verbrücktem C₃-C₁₂-Cycloalkyl und Heteroaryl, das 1 oder 2 Stickstoffatome enthält
n 1
m 1 oder 2
R⁵ ein Rest, der über ein Stickstoffatom an L gebunden ist, ausgewählt aus der Gruppe bestehend aus gegebenenfalls substituiertem Morpholinyl, Piperidinyl, R⁸-Piperazinyl, Pyrrolidinyl, Tropenyl, R⁸-Diketomethylpiperazinyl, Sulfoxomorpholinyl, Sulfonylmorpholinyl, Thiomorpholinyl, -NR⁸R⁹ und Azacycloheptyl,
R⁸, R⁹ unsubstituierte Stickstoffsubstituenten an R⁵, gleich oder verschieden, die Wasserstoff oder ein Rest ausgewählt aus der Gruppe bestehend aus C₁-C₆-Alkyl, -C₁-C₄-Alkyl-C₃-C₁₀-Cycloalkyl, C₃-C₁₀-Cycloalkyl, C₆-C₁₄-Aryl, -C₁-C₄-Alkyl-C₆-C₁₄-aryl, Pyranyl, Pyridinyl, Pyrimidinyl, C₁-C₄-Alkyloxycarbonyl,
C₆-C₁₄-Arylcarbonyl-, C₁-C₄-Alkylcarbonyl-, C₆-C₁₄-Arylmethyloxycarbonyl-, C₆-C₁₄-Arylsulfonyl-, C₁-C₄-Alkylsulfonyl- und C₆-C₁₄-Aryl-C₁-C₄-Alkylsulfonyl-,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.

3. Verbindungen nach Anspruch 1, worin
R¹ bis R⁴, R⁶ und R⁷ die angegebene Bedeutung aufweisen,
L ein Linker ausgewählt aus der Gruppe bestehend aus gegebenenfalls substituiertem C₂-C₁₀-Alkyl, C₂-C₁₀-Alkenyl, C₆-C₁₄-Aryl, -C₂-C₄-Alkyl-C₆-C₁₄-Aryl, - C₆-C₁₄-Aryl-C₁-C₄-Alkyl, gegebenenfalls verbrücktem C₃-C₁₂-Cycloalkyl und Heteroaryl, das 1 oder 2 Stickstoffatome enthält
n 0 oder 1
m 1 oder 2
R⁵ ein Rest, der über ein Kohlenstoffatom an L gebunden ist, ausgewählt aus der Gruppe bestehend aus R⁸- Piperidinyl-, R⁸R⁹-Piperazinyl-, R⁸-Pyrrolidinyl, R⁸-Piperazinylcarbonyl-, R⁸-Tropenyl, R⁸-Morpholinyl und R⁸-Azacycloheptyl, und
R⁸, R⁹ unsubstituierte Stickstoffsubstituenten an R⁵, gleich oder verschieden, die Wasserstoff oder ein Rest ausgewählt aus der Gruppe bestehend aus C₁-C₆-Alkyl, -C₁-C₄-Alkyl-C₃-C₁₀-Cycloalkyl, C₃-C₁₀-Cycloalkyl, C₆-C₁₄-Aryl, -C₁-C₄-Alkyl-C₆-C₁₄-aryl, Pyranyl, Pyridinyl, Pyrimidinyl, C₁-C₄-Alkyloxycarbonyl, C₆-C₁₄-Arylcarbonyl-, C₁-C₄-Alkylcarbonyl-, C₆-C₁₄-Arylmethyloxycarbonyl-, C₆-C₁₄-Arylsulfonyl-, C₁-C₄-Alkylsulfonyl- und C₆-C₁₄-Aryl-C₁-C₄-Alkylsulfonyl-,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.

4. Verbindungen nach einem der Ansprüche 1 bis 3, worin
L, m, n und R³ bis R⁹ die angegebene Bedeutung aufweisen, und
R¹, R² gleich oder verschieden, ein Rest ausgewählt aus der Gruppe bestehend aus Wasserstoff, Me, Et, Pr, oder
R¹ und R² gemeinsam eine C₂-C₄-Alkylbrücke bilden,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.

5. Verbindungen nach einem der Ansprüche 1 bis 4, worin
R¹, R², m, n und R⁵ bis R⁸ die angegebene Bedeutung aufweisen, und
R³ ein Rest ausgewählt aus der Gruppe bestehend aus gegebenenfalls substituiertem C₁-C₁₀-Alkyl, C₃-C₇-Cycloalkyl, C₃-C₆-Heterocycloalkyl und C₆-C₁₄-Aryl oder
R¹ und R³ oder R² und R³ gemeinsam eine gesättigte oder ungesättigte C₃-C₄-Alkylbrücke, die 1 bis 2 Heteroatome enthalten kann,
R⁴ ein Rest ausgewählt aus der Gruppe bestehend aus Wasserstoff, OMe, OH, Me, Et, Pr, OEt, NHMe, NH₂, F, CL, Br, Ö-Propargyl, O-Butinyl, CN, SMe, NMe₂, CONH₂, Ethinyl, Propinyl, Butinyl und Allyl, und
L ein Linker ausgewählt aus der Gruppe bestehend aus gegebenenfalls substituiertem Phenyl, Phenylmethyl, Cyclohexyl und verzweigtem C₁-C₆-Alkyl,
gegebenenfalls in Form ihrer Tautomeren, ihrer Racemate, ihrer Enantiomere, ihrer Diastereomere und ihrer Gemische, sowie gegebenenfalls ihrer pharmakologisch unbedenklichen Säureadditionssalze,
bedeuten.

6. Folgende Verbindungen der allgemeinen Formel (I) nach einem der Ansprüche 1 bis 5
| | | | | | | |
|---|---|---|---|---|---|---|
| 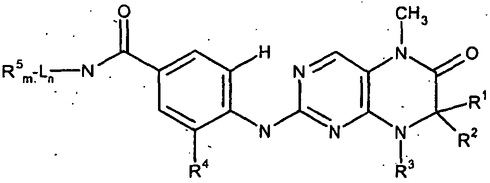 | | | | | | |
| | | | | | | |
| Bsp. | R¹ | R² | Konfig. R¹ od. R² | R³ | R⁴ | Lₙ-R⁵ₘ |
|---|---|---|---|---|---|---|
| 27 | H | 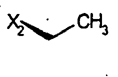 | R | 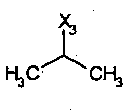 | 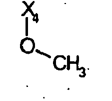 | 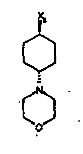 |
| 44 | H | 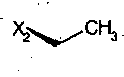 | R | 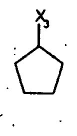 | H | 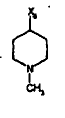 |
| 46 | H | 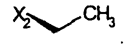 | R | 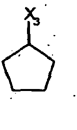 | 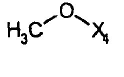 | 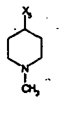 |
| 55 | H | 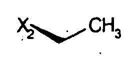 | R | 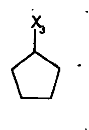 | 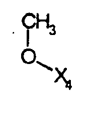 | 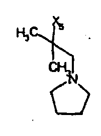 |
| 58 | H | 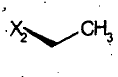 | R | 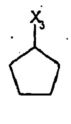 | 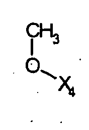 | 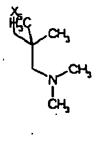 |
| 102 | H | 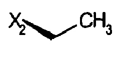 | R | 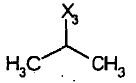 | 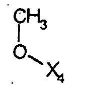 | 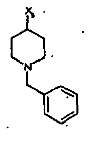 |
| 103 | H | 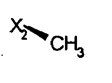 | R | 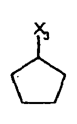 | 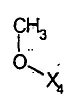 | 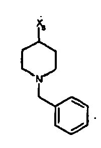 |
| 105 | H | 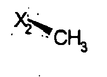 | R | 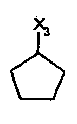 | 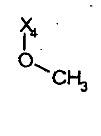 | 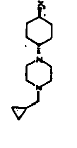 |
| 110 | H | 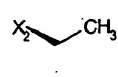 | R | 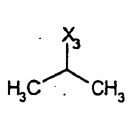 | 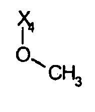 | 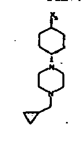 |
| 115 | H | 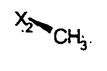 | R | 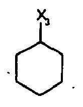 | 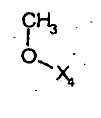 | 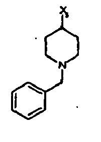 |
| 133 | H | 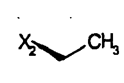 | R | 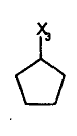 | 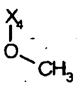 | 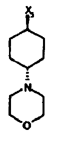 |
| 134 | H | 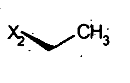 | R | 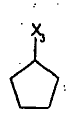 | 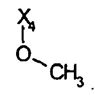 | 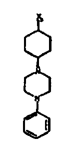 |
| 234 | H | 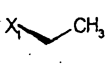 | R | 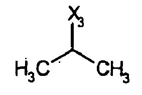 | 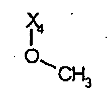 | 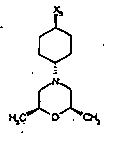 |
| 240 | H | 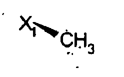 | R | 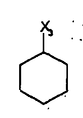 | 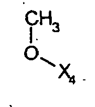 | 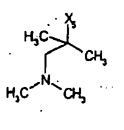 |
wobei die in der Tabelle verwendeten Abkürzungen X₁, X₂, X₃, X₄ und X₅ stehen jeweils für eine Verknüpfung mit einer Position in der unter der Tabelle aufgeführten allgemeinen Formel anstelle der entsprechenden Reste R¹, R², R³, R⁴ und L-R⁵.

7. Verbindung der Formel I nach einem der Ansprüche 1 bis 6 zur Verwendung als Arzneimittel.

8. Verbindung der Formel nach einem der Ansprüche 1 bis 6 zur Verwendung als Arzneimittel mit antiproliferativer Wirkung.

9. Verwendung einer Verbindung der Formel 1 gemäß einem der Ansprüche 1 bis 6 zur Herstellung eines Arzneimittels zur Behandlung und/oder Prävention von Krebs, Infektionen, Entzündungs- und Autoimmunerkrankungen.

10. Pharmazeutische Zubereitungen, enthaltend als Wirkstoff eine oder mehrere Verbindungen der allgemeinen Formel (I) gemäß einem der Ansprüche 1 bis 6
oder deren physiologisch verträgliche Salze gegebenenfalls in Kombination mit üblichen Hilfs- und/oder Trägerstoffen.

11. Verfahren zur Herstellung einer Verbindung der allgemeinen Formel (I), 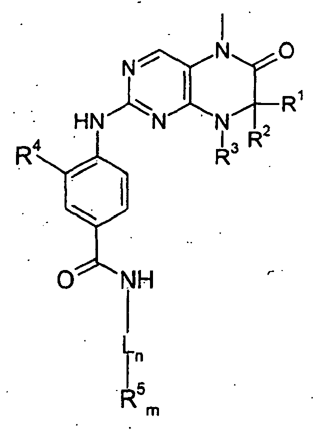 worin
R¹-R⁵,m ,n und L die in den Ansprüchen 1 bis 5 angegebene Bedeutung aufweisen,
**dadurch gekennzeichnet, daß** eine Verbindung der allgemeinen Formel (II) 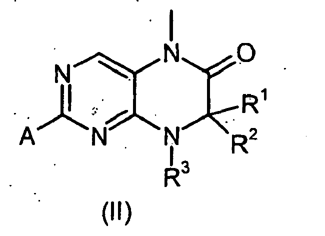 worin
R¹-R³ die in den Ansprüchen 1 bis 4 angegebene Bedeutung aufweisen und A eine Abgangsgruppe ist,
mit einer gegebenenfalls substituierten Verbindung der allgemeinen Formel (III), 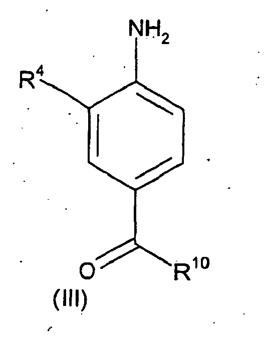 worin
R⁴ die in den Ansprüchen 1 bis 5 angegebene Bedeutung aufweist und
R¹⁰ OH, NH-L-R⁵, -O-Methyl, -O-Ethyl bedeutet,
umgesetzt wird, und gegebenenfalls anschließend das Produkt der allgemeinen Formel (IV) 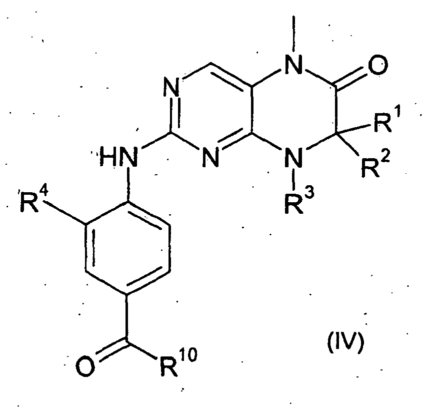 worin
R¹ bis R⁴ die in den Ansprüchen 1 bis 5 angegebene Bedeutung aufweist und .
R¹⁰ OH, -NH-L-R⁵, -O-Methyl oder -O-Ethyl bedeutet,
gegebenenfalls nach vorhergehender Hydrolyse der Estergruppe -COR¹⁰, mit einem Amin der allgemeinen Formel (V)
NH₂-L-R⁵ₘ (V)
worin
R⁵ die in den Ansprüchen 1 bis 5 angegebene Bedeutung aufweist,
umgesetzt wird.

12. Verbindung der Formel (II), 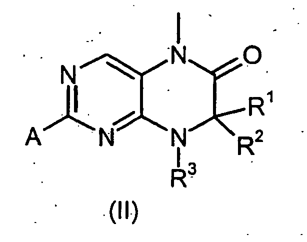 worin
R¹-R³ die in den Ansprüchen 1 bis 5 angegebene Bedeutung aufweisen und A eine Abgangsgruppe ist.

13. Cis- oder trans-4-(4-Cyclopropylmethyl-piperazin-1-yl)-cyclohexylamine

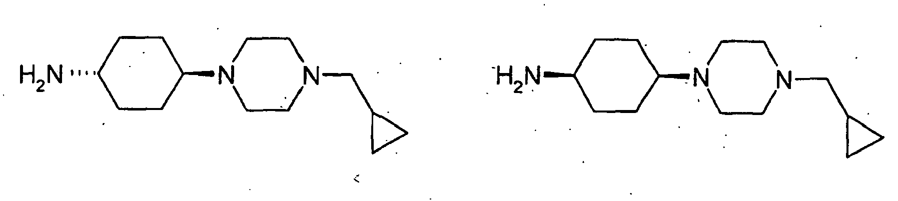

## Claims

1. Compounds of general formula (I), 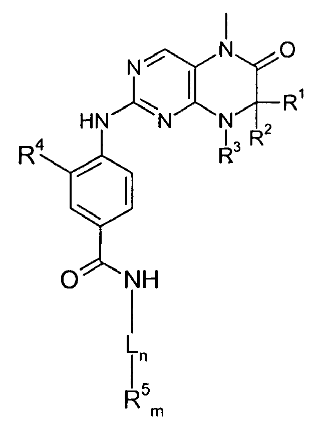 wherein
R¹, R² which may be identical or different, denote hydrogen or optionally substituted C₁-C₆-alkyl,
or
R¹ and R² together denote a 2- to 5-membered alkyl bridge which may contain 1 to 2 heteroatoms,
R³ denotes hydrogen or a group selected from among optionally substituted C₁-C₁₂-alkyl, C₂-C₁₂-alkenyl, C₂-C₁₂-alkynyl and C₆-C₁₄-aryl, or a group selected from among optionally substituted C₃-C₁₂-cycloalkyl, C₃-C₁₂-cycloalkenyl, C₇-C₁₂-polycycloalkyl, C₇-C₁₂-polycycloalkenyl, C₅-C₁₂-spirocycloalkyl, optionally substituted C₃-C₁₂-heterocycloalkyl which contains 1 to 2 heteroatoms, and C₃-C₁₂-heterocycloalkenyl which contains 1 to 2 heteroatoms, or
R¹ and R³ or R² and R³ together denote a saturated or unsaturated C₃-C₄-alkyl bridge which may contain 1 heteroatom,
R⁴ denotes a group selected from among hydrogen, -CN, hydroxy, -NR₆R₇ and halogen, or
a group selected from among optionally substituted C₁-C₆-alkyl, C₂-C₆-alkenyl, C₂-C₆-alkynyl, C₁-C₅-alkyloxy, C₂-C₅-alkenyloxy, C₂-C₅-alkynyloxy, C₁-C₆-alkylthio, C₁-C₆-alkylsulphoxo and C₁-C₆-alkylsulphonyl,
L denotes a linker selected from among optionally substituted C₂-C₁₀-alkyl, C₂-C₁₀-alkenyl, C₆-C₁₄-aryl, -C₂-C₄-alkyl-C₆-C₁₄-aryl, -C₆-C₁₄-aryl-C₁-C₄-alkyl, C₃-C₁₂-cycloalkyl and heteroaryl which contains 1 or 2 nitrogen atoms,
n denotes 0 or 1
m denotes 1 or 2
R⁵ denotes a group selected from among optionally substituted morpholinyl, piperidinyl, piperazinyl, piperazinylcarbonyl, pyrrolidinyl, tropenyl, R⁸-diketomethylpiperazinyl, sulphoxomorpholinyl, sulphonylmorpholinyl, thiomorpholinyl, -NR⁸R⁹ and azacycloheptyl,
R⁶, R⁷, which may be identical or different, denote hydrogen or C₁-C₄-alkyl, and
R⁸, R⁹ denote unsubstituted nitrogen substituents at R⁵, which may be identical or different, either hydrogen or a group selected from among C₁-C₆-alkyl, -C₁-C₄-alkyl-C₃-C₁₀-cycloalkyl, C₃-C₁₀-cycloalkyl, C₆-C₁₄-aryl, -C₁-C₄-alkyl-C₆-C₁₄-aryl, pyranyl, pyridinyl, pyrimidinyl, C₁-C₄-alkyloxycarbonyl, C₆-C₁₄-arylcarbonyl, C₁-C₄-alkylcarbonyl, C₆-C₁₄-arylmethyloxycarbonyl, C₆-C₁₄-arylsulphonyl, C₁-C₄-alkylsulphonyl and C₆-C₁₄-aryl-C₁-C₄-alkylsulphonyl,
wherein the term heterocycloalkyl, unless otherwise stated in the definitions, denotes 3- to 12-membered saturated or unsaturated heterocycles which may contain nitrogen, oxygen or sulphur as heteroatoms,
the term alkenyl, in so far as it is a component of other groups, denotes branched or unbranched alkylene groups having 2 to 10 carbon atoms, if they have at least one double bond,
the term aryl denotes an aromatic ring system having 6 to 14 carbon atoms,
the substituents of the optionally substituted alkyl groups, unless stated otherwise, may be selected from one or more fluorine atoms,
the substituents of the optionally substituted alkenyl groups, unless stated otherwise, may be selected from one or more fluorine atoms,
the substituents of the optionally substituted alkynyl groups, unless stated otherwise, may be selected from one or more fluorine atoms,
the substituents of the optionally substituted cycloalkyl groups, unless stated otherwise, may be selected from -OH, -NO₂ -CN, -OMe, -OCHF₂, -OCF₃, -NH₂ or halogen,
the substituents of the optionally substituted heterocycloalkyl groups, unless stated otherwise, may be selected from C₁-C₄-alkyl,
the substituents of the optionally substituted aryl groups, unless stated otherwise, may be selected from -OH, -NO₂, -CN, -OMe, -OCHF₂, -OCF₃, -NH₂ or halogen,
optionally in the form of the tautomers, the racemates, the enantiomers, the diastereomers and the mixtures thereof, and optionally the pharmacologically acceptable acid addition salts thereof.

2. Compounds according to claim 1, wherein
R¹ to R⁴, R⁶ and R⁷ are as hereinbefore defined, and
L denotes a linker selected from among optionally substituted C₂-C₁₀-alkyl, C₂-C₁₀-alkenyl, C₆-C₁₄-aryl, -C₂-C₄-alkyl-C₆-C₁₄-aryl, -C₆-C₁₄-aryl-C₁-C₄-alkyl, optionally bridged C₃-C₁₂-cycloalkyl and heteroaryl which contains 1 or 2 nitrogen atoms
n denotes 1
m denotes 1 or 2
R⁵ denotes a group which is bound to L via a nitrogen atom, selected from among optionally substituted morpholinyl, piperidinyl, R⁸-piperazinyl, pyrrolidinyl, tropenyl, R⁸-diketomethylpiperazinyl, sulphoxomorpholinyl, sulphonylmorpholinyl, thiomorpholinyl, -NR⁸R⁹ and azacycloheptyl,
R⁸, R⁹ denote unsubstituted nitrogen substituents at R⁵, which may be identical or different, hydrogen or a group selected from among C₁-C₆-alkyl, -C₁-C₄-alkyl-C₃-C₁₀-cycloalkyl, C₃-C₁₀-cycloalkyl, C₆-C₁₄-aryl, -C₁-C₄-alkyl-C₆-C₁₄-aryl, pyranyl, pyridinyl, pyrimidinyl, C₁-C₄-alkyloxycarbonyl, C₆-C₁₄-arylcarbonyl, C₁-C₄-alkylcarbonyl, C₆-C₁₄-arylmethyloxycarbonyl, C₆-C₁₄-arylsulphonyl, C₁-C₄-alkylsulphonyl and C₆-C₁₄-aryl-C₁-C₄-alkylsulphonyl,
optionally in the form of the tautomers, the racemates, the enantiomers, the diastereomers and the mixtures thereof, and optionally the pharmacologically acceptable acid addition salts thereof.

3. Compounds according to claim 1, wherein
R¹ to R⁴, R⁶ and R⁷ are as hereinbefore defined,
L denotes a linker selected from among optionally substituted C₂-C₁₀-alkyl, C₂-C₁₀-alkenyl, C₆-C₁₄-aryl, -C₂-C₄-alkyl-C₆-C₁₄-aryl, -C₆-C₁₄-aryl-C₁-C₄-alkyl, optionally bridged C₃-C₁₂-cycloalkyl and heteroaryl which contains 1 or 2 nitrogen atoms
n denotes 0 or 1
m denotes 1 or 2
R⁵ denotes a group which is bound to L via a carbon atom, selected from among R⁸ - piperidinyl, R⁸R⁹-piperazinyl, R⁸-pyrrolidinyl, R⁸-piperazinylcarbonyl, R⁸-tropenyl, R⁸-morpholinyl and R⁸-azacycloheptyl, and
R⁸, R⁹ denote unsubstituted nitrogen substituents at R⁵, which may be identical or different, hydrogen or a group selected from among C₁-C₆-alkyl, -C₁-C₄-alkyl-C₃-C₁₀-cycloalkyl, C₃-C₁₀-cycloalkyl, C₆-C₁₄-aryl, -C₁-C₄-alkyl-C₆-C₁₄-aryl, pyranyl, pyridinyl, pyrimidinyl, C₁-C₄-alkyloxycarbonyl, C₆-C₁₄-arylcarbonyl, C₁-C₄-alkylcarbonyl, C₆-C₁₄-arylmethyloxycarbonyl, C₆-C₁₄-arylsulphonyl, C₁-C₄-alkylsulphonyl and C₆-C₁₄-aryl-C₁-C₄-alkylsulphonyl,
optionally in the form of the tautomers, the racemates, the enantiomers, the diastereomers and the mixtures thereof, and optionally the pharmacologically acceptable acid addition salts thereof.

4. Compounds according to one of claims 1 to 3, wherein
L, m, n and R³ to R⁹ are as hereinbefore defined, and
R¹, R² which may be identical or different, denote a group selected from among hydrogen, Me, Et, Pr, or
R¹ and R² together form a C₂-C₄-alkyl bridge,
optionally in the form of the tautomers, the racemates, the enantiomers, the diastereomers and the mixtures thereof, and optionally the pharmacologically acceptable acid addition salts thereof.

5. Compounds according to one of claims 1 to 4, wherein
R¹, R², m, n and R⁵ to R⁸ are as hereinbefore defined, and
R³ denotes a group selected from among optionally substituted C₁-C₁₀-alkyl, C₃-C₇-cycloalkyl, C₃-C₆-heterocycloalkyl and C₆-C₁₄-aryl or
R¹ and R³ or R² and R³ together denote a saturated or unsaturated C₃-C₄-alkyl bridge which may contain 1 to 2 heteroatoms,
R⁴ denotes a group selected from among hydrogen, OMe, OH, Me, Et, Pr, OEt, NHMe, NH₂, F, CL, Br, O-propargyl, O-butynyl, CN, SMe, NMe₂, CONH₂, ethynyl, propynyl, butynyl and allyl, and
L denotes a linker selected from among optionally substituted phenyl, phenylmethyl, cyclohexyl and branched C₁-C₆-alkyl,
optionally in the form of the tautomers, the racemates, the enantiomers, the diastereomers and the mixtures thereof, and optionally the pharmacologically acceptable acid addition salts thereof.

6. The following compounds of general formula (I) according to one of claims 1 to 5:
| | | | | | | |
|---|---|---|---|---|---|---|
| 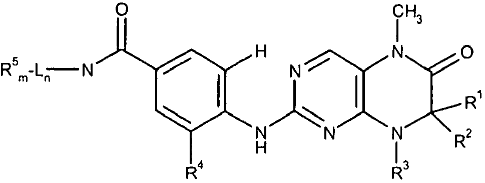 | | | | | | |
| | | | | | | |
| Example | R¹ | R² | Config R¹ or R² | R³ | R⁴ | Lₙ-R⁵m |
|---|---|---|---|---|---|---|
| 27 | H | 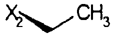 | R | 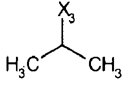 | 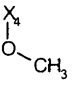 | 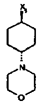 |
| 44 | H | 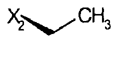 | R | 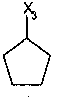 | H | 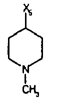 |
| 46 | H | 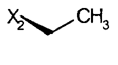 | R | 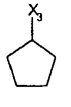 | 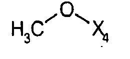 | 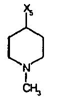 |
| 55 | H | 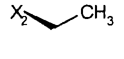 | R | 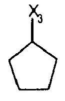 | 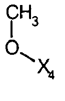 | 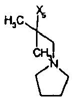 |
| 58 | H | 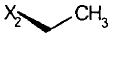 | R | 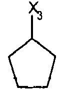 | 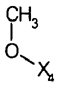 | 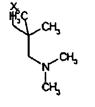 |
| 102 | H | 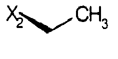 | R | 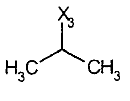 | 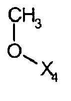 | 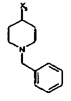 |
| 103 | H | 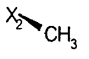 | R | 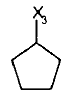 | 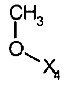 | 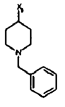 |
| 105 | H | 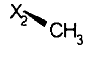 | R | 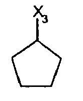 | 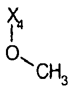 | 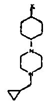 |
| 110 | H | 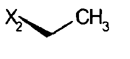 | R | 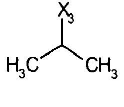 | 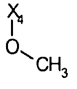 | 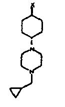 |
| 115 | H | 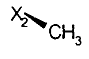 | R | 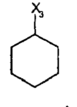 | 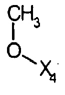 | 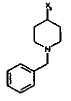 |
| 133 | H | 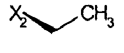 | R | 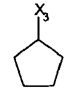 | 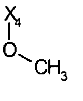 | 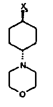 |
| 134 | H | 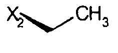 | R | 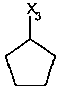 | 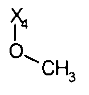 | 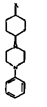 |
| 234 | H | 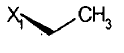 | R | 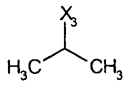 | 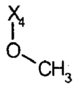 | 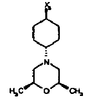 |
| 240 | H | 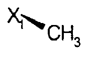 | R | 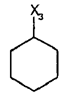 | 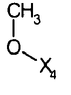 | 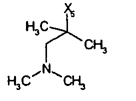 |
wherein the abbreviations X₁, X₂, X₃, X₄ and X₅ used in the Table each denote a linkage to a position in the general formula shown under the Table, instead of the corresponding groups R¹, R², R³, R⁴ and L-R⁵.

7. Compound of formula I according to one of claims 1 to 6 for use as a pharmaceutical composition.

8. Compound of formula I according to one of claims 1 to 6 for use as a pharmaceutical composition with an antiproliferative activity.

9. Use of a compound of formula I according to one of claims 1 to 6 for preparing a pharmaceutical composition for the treatment and/or prevention of cancer, infections, inflammatory and autoimmune diseases.

10. Pharmaceutical preparations, containing as active substance one or more compounds of general formula (I) according to one of claims 1 to 6 or the physiologically acceptable salts thereof, optionally combined with conventional excipients and/or carriers.

11. Process for preparing a compound of general formula (I), 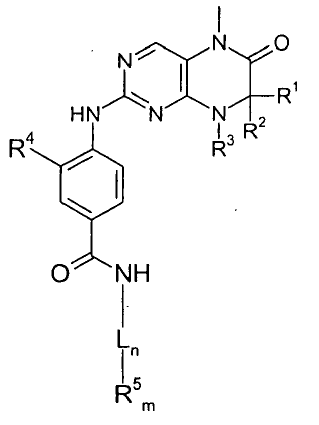 wherein
R¹-R⁵, m, n and L have the meanings given in claims 1 to 5,
**characterised in that** a compound of general formula (II) 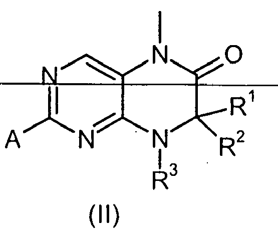 wherein
R¹-R³ have the meanings given in claims 1 to 4 and A is a leaving group, is reacted with an optionally substituted compound of general formula (III), 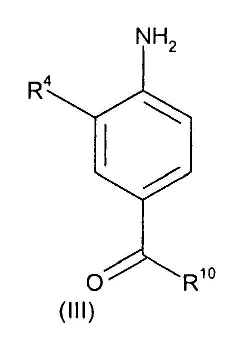 wherein
R⁴ has the meanings given in claims 1 to 5 and
R¹⁰ denotes OH, NH-L-R⁵, -O-methyl, -O-ethyl,
and then optionally the product of general formula (IV) 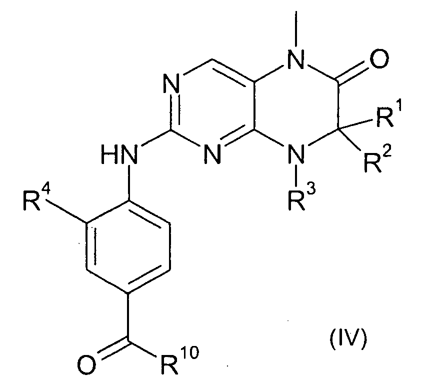 wherein
R¹ to R⁴ has the meanings given in claims 1 to 5 and
R¹⁰ denotes OH, -NH-L-R⁵, -O-methyl or -O-ethyl,
optionally after previous hydrolysis of the ester group -COR¹⁰, is reacted with an amine of general formula (V)
NH₂-L-R⁵ₘ (V)
wherein
R⁵ has the meanings given in claims 1 to 5.

12. Compound of formula (II), 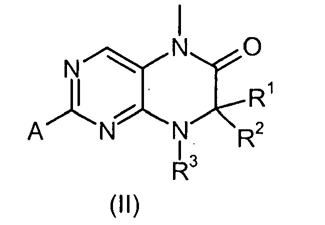 wherein
R¹-R³ have the meanings given in claims 1 to 5 and A is a leaving group.

13. Cis- or trans-4-(4-cyclopropylmethyl-piperazin-1-yl)-cyclohexylamine

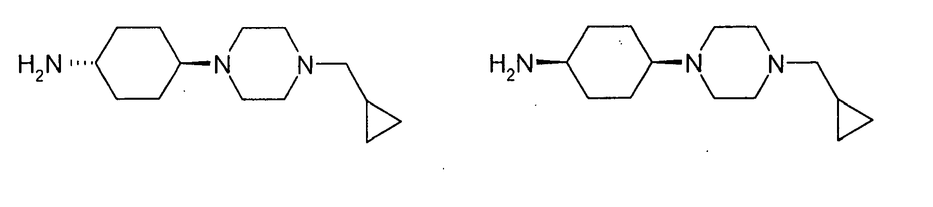

## Revendications

1. Composés de formule générale (I) 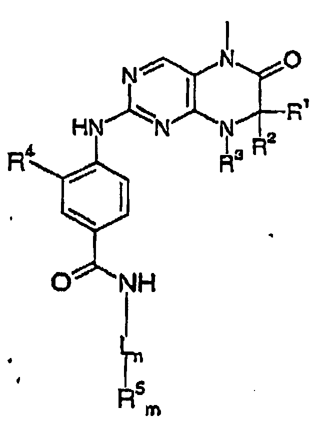 où
R¹, R² identiques ou différents, représentent l'hydrogène ou C₁-C₆-alkyle éventuellement substitué,
ou
R¹ et R² représentent ensemble un pont alkyle à 2 à 5 chaînons qui peut contenir 1 à 2 hétéroatomes,
R³ représente l'hydrogène ou un groupement choisi dans le groupe consistant en C₁-C₁₂-alkyle, C₂-C₁₂-alcényle, C₂-C₁₂-alcynyle et C₆-C₁₄-aryle éventuellement substitué, ou
un groupement choisi dans le groupe consistant en C₃-C₁₂-cycloalkyle, C₃-C₁₂-cycloalcényle, C₇-C₁₂-polycycloalkyle, C₇-C₁₂-polycycloalcényle, C₅-C₁₂-spirocycloalkyle éventuellement substitué, C₃-C₁₂-hétérocycloalkyle qui contient 1 à 2 hétéroatomes et C₃-C₁₂-hétérocycloalcényle qui contient 1 à 2 hétéroatomes, éventuellement substitué, ou
R¹ et R³ ou R² et R³ représentent ensemble un pont C₃-C₄-alkyle saturé ou insaturé qui peut contenir 1 hétéroatome,
R⁴ représente un groupement choisi dans le groupe consistant en l'hydrogène, -CN, hydroxy, -NR₆R₇ et halogène, ou
un groupement choisi dans le groupe consistant en C₁-C₆-alkyle, C₂-C₆-alcényle, C₂-C₆-alcynyle, C₁-C₅-alkyloxy, C₂-C₅-alcényloxy, C₂-C₅-alcynyloxy, C₁-C₆-alkylthio, C₁-C₆-alkylsulfoxo et C₁-C₆-alkylsulfonyle éventuellement substitué,
L représente un lieur choisi dans le groupe consistant en C₂-C₁₀-alkyle, C₂-C₁₀-alcényle, C₆-C₁₄-aryle,-C₂-C₄-alkyl-C₆-C₁₄-aryle, -C₆-C₁₄-aryl-C₂-C₄-alkyle, C₃-C₁₂-cycloalkyle et hétéroaryle qui contient 1 ou 2 hétéroatomes, éventuellement substitué,
n représente 0 ou 1,
m représente 1 ou 2
R⁵ représente un groupement choisi dans le groupe consistant en morpholinyle, pipéridinyle, pipérazinyle, pipérazinylcarbonyle, pyrrolidinyle, tropényle, R⁸-dicétométhylpipérazinyle, sulfoxomorpholinyle, sulfonylmorpholinyle, thiomorpholinyle, -NR⁸R⁹ et azacycloheptyle éventuellement substitué,
R⁶, R⁷, identiques ou différents, représentent l'hydrogène ou C₁-C₄-alkyle,
et
R⁸, R⁹, substituants de l'azote non substitués sur R⁵, identiques ou différents, représentent l'hydrogène ou un groupement choisi dans le groupe consistant en C₁-C₆-alkyle, -C₁-C₄-alkyl-C₃-C₁₀-cycloalkyle, C₃-C₁₀-cycloalkyle, C₆-C₁₄-aryle, - C₁-C₄-alkyl-C₆-C₁₄-aryle, pyranyle, pyridinyle, pyrimidinyle, C₁-C₄-alkyloxycarbonyle, C₆-C₁₄-arylcarbonyle, C₁-C₄-alkylcarbonyle, C₆-C₁₄-arylméthyloxycarbonyle, C₆-C₁₄-arylsulfonyle, C₁-C₄-alkylsulfonyle et C₆-C₁₄-aryl-C₁-C₄-alkylsulfonyle,
où le terme hétérocycloalkyle, dans la mesure où rien d'autre n'est décrit dans les définitions, représente des hétérocycles saturés ou insaturés à 3 à 12 chaînons, qui peuvent contenir comme hétéroatomes l'azote, l'oxygène ou le soufre,
où le terme alcényle, dans la mesure où il fait partie d'autres groupements, représente des groupes alkylène ramifiés ou non ramifiés avec 2 à 10 atomes de carbone, dans la mesure où ils présentent au moins une double liaison,
où le terme aryle représente un système cyclique aromatiques avec 6 à 14 atomes de carbone,
où les substituants des groupes alkyle éventuellement substitués, dans la mesure
où rien d'autre n'est décrit, peuvent être choisis parmi un ou plusieurs atomes de fluor,
où les substituants des groupes alcényle éventuellement substitués, dans la mesure
où rien d'autre n'est décrit, peuvent être choisis parmi un ou plusieurs atomes de fluor,
où les substituants des groupes alcynyle éventuellement substitués, dans la mesure
où rien d'autre n'est décrit, peuvent être choisis parmi un ou plusieurs atomes de fluor,
où les substituants des groupes cycloalkyle éventuellement substitués, dans la mesure où rien d'autre n'est décrit, peuvent être choisis parmi -OH, -NO₂ -CN, -OMe, -OCHF₂, -OCF₃, -NH₂ ou halogène,
où les substituants des groupes hétérocycloalkyle éventuellement substitués, dans la mesure où rien d'autre n'est décrit, peuvent être choisis parmi C₁-C₄-alkyle,
où les substituants des groupes aryle éventuellement substitués, dans la mesure où rien d'autre n'est décrit, peuvent être choisis parmi -OH, -NO₂, -CN, -OMe, -OCHF₂, -OCF₃, -NH₂ ou halogène,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

2. Composés selon la revendication 1 où
R¹ à R⁴, R⁶ et R⁷ présentent la signification indiquée, et
L représente un lieur choisi dans le groupe consistant en C₂-C₁₀-alkyle, C₂-C₁₀-alcényle, C₆-C₁₄-aryle, -C₂-C₁₄ -alkyl-C₆-C₁₄-aryle, -C₆-C₁₄-aryl-C₁-C₄-alkyle éventuellement substitué, C₃-C₁₂-cycloalkyle et hétéroaryle qui contient 1 ou 2 atomes d'azote, éventuellement ponté,
n représente 1,
m représente 1 ou 2
R⁵ représente un groupement, qui est lié à L par le biais d'un atome d'azote, choisi dans le groupe consistant en morpholinyle, pipéridinyle, R⁸-pipérazinyle, pyrrolidinyle, tropényle, R⁸-dicétométhylpipérazinyle, sulfoxomorpholinyle, sulfonylmorpholinyle, thiomorpholinyle, -NR⁸R⁹ et azacycloheptyle éventuellement substitué,
R⁸, R⁹, substituants de l'azote non substitués sur R⁵, identiques ou différents, qui représentent l'hydrogène ou un groupement choisi dans le groupe consistant en C₁-C₆-alkyle, -C₁-C₄-alkyl-C₃-C₁₀-cycloalkyle, C₃-C₁₀-cycloalkyle, C₆-C₁₄-aryle, - C₁-C₄-alkyl-C₆-C₁₄-aryle, pyranyle, pyridinyle, pyrimidinyle, C₁-C₄-alkyloxycarbonyle,
C₆-C₁₄-arylcarbonyle, C₁-C₄-alkylcarbonyle, C₆-C₁₄-arylméthyloxycarbonyle, C₆-C₁₄-arylsulfonyle, C₁-C₄-alkylsulfonyle et C₆-C₁₄-aryl-C₁-C₄-alkylsulfonyle,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

3. Composés selon la revendication 1 où
R¹ à R⁴, R⁶ et R⁷ présentent la signification indiquée,
L représente un lieur choisi dans le groupe consistant en C₂-C₁₀-alkyle, C₂-C₁₀-alcényle, C₆-C₁₄-aryle, -C₂-C₄-alkyl-C₆-C₁₄-aryle, -C₆-C₁₄-aryl-C₁-C₄-alkyle éventuellement substitué, C₃-C₁₂-cycloalkyle et hétéroaryle, qui contient 1 ou 2 atomes d'azote, éventuellement ponté,
n représente 0 ou 1,
m représente 1 ou 2
R⁵ représente un groupement, qui est lié à L par le biais d'un atome de carbone, choisi dans le groupe consistant en R⁸-pipéridinyle, R⁸R⁹-pipérazinyle, R⁸-pyrrolidinyle, R⁸-pipérazinylcarbonyle, R⁸-tropényle, R⁸-morpholinyle et R⁸-azacycloheptyle,
et
R⁸, R⁹, substituants de l'azote non substitués sur R⁵, identiques ou différents, qui représentent l'hydrogène ou un groupement choisi dans le groupe consistant en C₁-C₆-alkyle, -C₁-C₄-alkyl-C₃-C₁₀-cycloalkyle, C₃-C₁₀-cycloalkyle, C₆-C₁₄-aryle, - C₁-C₄-alkyl-C₆-C₁₄-aryle, pyranyle, pyridinyle, pyrimidinyle, C₁-C₄-alkyloxycarbonyle, C₆-C₁₄-arylcarbonyle, C₁-C₄-alkylcarbonyle, C₆-C₁₄-arylméthyloxycarbonyle, C₆-C₁₄-arylsulfonyle, C₁-C₄-alkylsulfonyle et C₆-C₁₄-aryl-C₁-C₄-alkylsulfonyle,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

4. Composés selon l'une des revendications 1 à 3 où
L, m, n et R³ à R⁹ présentent la signification indiquée, et
R¹, R² identiques ou différents, représentent un groupement choisi dans le groupe consistant en l'hydrogène, Me, Et, Pr, ou
R¹ et R² forment ensemble un pont C₂-C₄-alkyle,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

5. Composés selon l'une des revendications 1 à 4 où
R¹, R², m, n et R⁵ à R⁸ présentent la signification indiquée, et
R³ représente ou groupement choisi dans le groupe consistant en C₁-C₁₀-alkyle, C₃-C₇-cycloalkyle, C₃-C₆-hétérocycloalkyle et C₆-C₁₄-aryle éventuellement substitué, ou
R¹ et R³ ou R² et R³ représentent ensemble un pont C₃-C₄-alkyle saturé ou insaturé, qui peut contenir 1 à 2 hétéroatomes,
R⁴ représente un groupement choisi dans le groupe consistant en l'hydrogène, OMe, OH, Me, Et, Pr, OEt, NHMe, NH₂, F, Cl, Br, O-propargyle, O-butynyle, CN, SMe, NMe₂, CONH₂, éthynyle, propynyle, butynyle et allyle,
et
L représente un lieur choisi dans le groupe consistant en phényle, phénylméthyle, cyclohexyle et C₁-C₆-alkyle ramifié éventuellement substitué,
éventuellement sous forme de leurs tautomères, de leurs racémates, de leurs énantiomères, de leurs diastéréoisomères et de leurs mélanges, ainsi éventuellement que de leurs sels d'addition d'acide pharmacologiquement acceptables.

6. Composés suivants de formule générale (I) selon l'une des revendications 1 à 5
| | | | | | | |
|---|---|---|---|---|---|---|
| 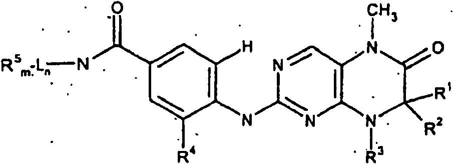 | | | | | | |
| | | | | | | |
| Ex. | R¹ | R² | config. R¹ ou R² | R³ | R⁴ | Lₙ-R⁵ₘ |
|---|---|---|---|---|---|---|
| 27 | H | 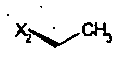 | R | 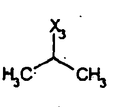 | 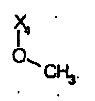 | 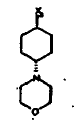 |
| 44 | H | 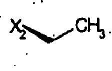 | R | 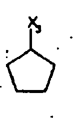 | H | 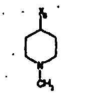 |
| 46 | H | 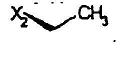 | R | 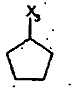 | 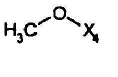 | 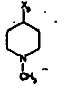 |
| 55 | H | 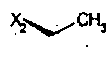 | R | 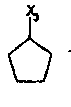 | 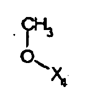 | 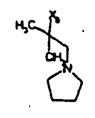 |
| 58 | H | 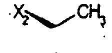 | R | 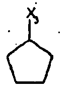 | 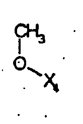 | 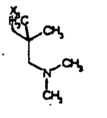 |
| 102 | H | 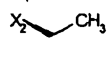 | R | 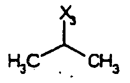 | 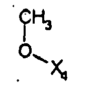 | 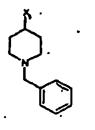 |
| 103 | H | 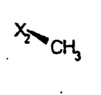 | R | 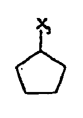 | 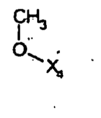 | 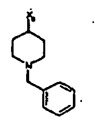 |
| 105 | H | 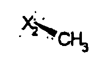 | R | 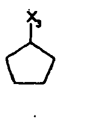 | 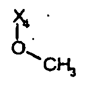 | 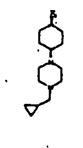 |
| 110 | H | 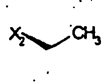 | R | 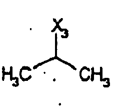 | 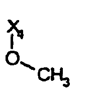 | 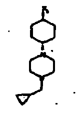 |
| 115 | H | 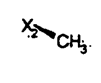 | R | 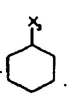 | 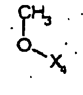 | 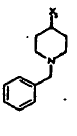 |
| 133 | H | 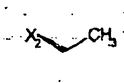 | R | 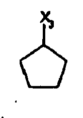 | 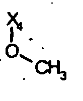 | 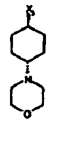 |
| 134 | H | 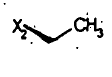 | R | 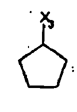 | 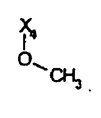 | 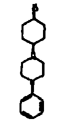 |
| 234 | H | 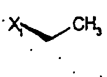 | R | 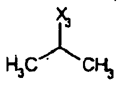 | 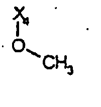 | 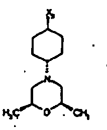 |
| 240 | H | 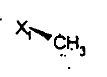 | R | 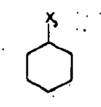 | 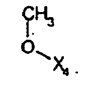 | 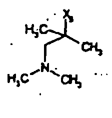 |
où les abréviations X₁, X₂, X₃, X₄ et X₅ utilisées dans le tableau représentent dans chaque cas une liaison avec une position dans la formule générale présentée dans le tableau à la place des groupements R¹, R², R³, R⁴ et L-R⁵ correspondants.

7. Composé de formule I selon l'une des revendications 1 à 6 destiné à être utilisé comme médicament.

8. Composé de formule I selon l'une des revendications 1 à 6 destiné à être utilisé comme médicament à action antiproliférative.

9. Utilisation d'un composé de formule I selon l'une des revendications 1 à 6 pour la production d'un médicament pour le traitement et/ou la prévention du cancer, d'infections, de maladies inflammatoires et auto-immunes.

10. Préparations pharmaceutiques contenant comme principe actif un ou plusieurs composés de formule générale (I) selon l'une des revendications 1 à 6 ou leurs sels physiologiquement acceptables éventuellement en combinaison avec des adjuvants et/ou supports courants.

11. Procédé pour préparer un composé de formule générale (I) 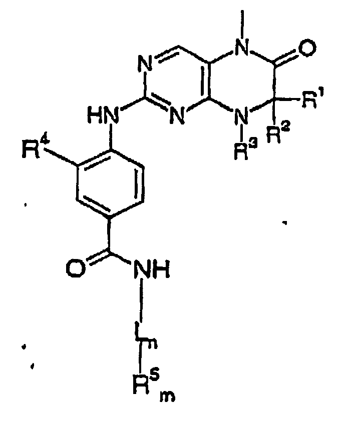 où
R¹-R⁵, m, n et L présentent la signification indiquée dans les revendications 1 à 5,
**caractérisé en ce qu'**un composé de formule générale (II) 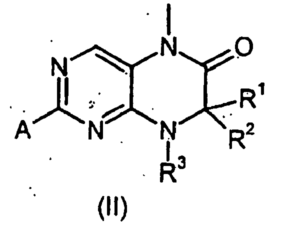 où
R¹-R³ présentent la signification indiquée dans les revendications 1 à 4 et A est un groupe partant,
est mis à réagir avec un composé éventuellement substitué de formule générale (III) 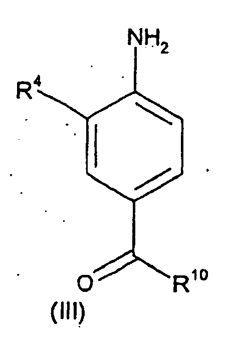 où
R⁴ présente la signification indiquée dans les revendications 1 à 5 et R¹⁰ représente OH, NH-L-R⁵, -O-méthyle, -O-éthyle,
puis, éventuellement, le produit de formule générale (IV) 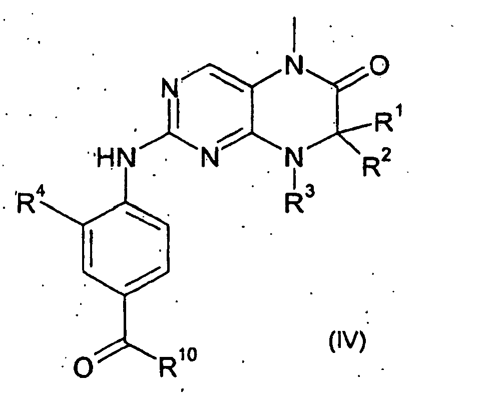 où
R¹ à R⁴ présentent la signification indiquée dans les revendications 1 à 5 et R¹⁰ représente OH, NH-L-R⁵, -O-méthyle ou -O-éthyle,
éventuellement après hydrolyse préalable du groupe ester -COR¹⁰, est mis à réagir avec une amine de formule générale (V)
NH₂-L-R⁵ₘ (V)
où
R⁵ présente la signification indiquée dans les revendications 1 à 5.

12. Composé de formule (II) 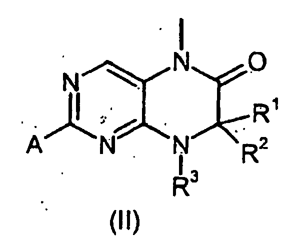 où
R¹-R³ présentent la signification indiquée dans les revendications 1 à 5 et A est un groupe partant.

13. Cis- ou trans-4-(4-cyclopropylméthyl-pipérazin-1-yl)-cyclohexylamines
